# EUROPEAN PATENT SPECIFICATION

(11) **EP 4 178 986 B1**
(45) Date of publication and mention of the grant of the patent: **01.07.2026**
(21) Application number: 21866088.4
(22) Date of filing: 13.09.2021
(51) Int. Cl.: C07K 14/705, C07K 16/28, C12N 5/10, C12N 15/62, A61K 39/395, C07K 14/725, C12N 9/10, C07K 14/715

(54) **DOWNREGULATION OF MEMBRANE-BOUND PROTEINS BY RECEPTOR TAC TECHNOLOGY**
HERUNTERREGULIERUNG VON MEMBRANGEBUNDENEN PROTEINEN DURCH REZEPTOR-TAC-TECHNOLOGIE
RÉGULATION À LA BAISSE DE PROTÉINES LIÉES À UNE MEMBRANE PAR LA TECHNOLOGIE DU RÉCEPTEUR TAC

(30) Priority: 13.09.2020 US 202063077658 P; 02.02.2021 US 202163144945 P; 11.04.2021 US 202163173476 P
(43) Date of publication of application: 17.05.2023
(73) Proprietor: Shandong Boan Biotechnology Co., Ltd., Yantai, Shandong 264670 (CN); Boan Boston LLC, Woburn, Massachusetts 01801 (US)
(72) Inventor: ZHOU, Li, Woburn, Massachusetts 01801 (US); SHI, Feng, Woburn, Massachusetts 01801 (US); HARRIS, Michael, Woburn, Massachusetts 01801 (US)
(74) Representative: karo IP
(86) International application number: PCT/CN2021/117886
(87) International publication number: WO 2022/053036

(56) References cited:
- WO-A1-2017/024318
- WO-A1-2019/007869
- WO-A1-2019/032916
- WO-A1-2019/089592
- WO-A1-2019/089592
- WO-A1-2020/146250
- WO-A1-2020/160419
- CN-A- 113 088 495
- US-A1- 2018 086 831
- TAKAHIRO KAMIYA ET AL: "A novel method to generate T-cell receptor-deficient chimeric antigen receptor T cells", BLLOD ADVANCES, vol. 2, no. 5, 13 March 2018 (2018-03-13), pages 517 - 528, XP055505570, DOI: 10.1182/bloodadvances.2017012823
- MARY ICONOMOU ET AL: "Systematic approaches to identify E3 ligase substrates", BIOCHEMICAL JOURNAL, vol. 473, no. 22, 10 November 2016 (2016-11-10), GB, pages 4083 - 4101, XP055660306, ISSN: 0264-6021, DOI: 10.1042/BCJ20160719
- KEVIN MOREAU ET AL: "Proteolysis-targeting chimeras in drug development: A safety perspective", BRITISH JOURNAL OF PHARMACOLOGY, WILEY-BLACKWELL, UK, vol. 177, no. 8, 25 February 2020 (2020-02-25), pages 1709 - 1718, XP071172105, ISSN: 0007-1188, DOI: 10.1111/BPH.15014
- YU AIXIN, MALEK THOMAS R.: "The Proteasome Regulates Receptor-mediated Endocytosis of Interleukin-2", JOURNAL OF BIOLOGICAL CHEMISTRY, vol. 276, no. 1, 5 January 2001 (2001-01-05), US , pages 381 - 385, XP055910436, ISSN: 0021-9258, DOI: 10.1074/jbc.M007991200

## Description

This application claims the benefit of U.S. Provisional Patent Application No. 63/077,658, entitled "Downregulation of Membrane-Bound Proteins by Receptor TAC Technology", filed on September 13, 2020, U.S. Provisional Patent Application No. 63/144,945, entitled "Downregulation of Membrane-Bound Proteins by Receptor TAC Technology" filed on February 2, 2021, and U.S. Provisional Patent Application No. 63/173,476, entitled "Downregulation of Membrane-Bound Proteins by Receptor TAC Technology" filed on April 11, 2021.

This invention was made subject to a joint research agreement between SHANDONG BOAN BIOTECHNOLOGY CO., LTD. and Boan Boston LLC.

### TECHNICAL FIELD

This application relates to downregulation of Membrane-Bound Proteins (MBPs), and in particular, to downregulation of Membrane-Bound Proteins by Receptor TAC Technology. The present application provides novel fusion proteins, nucleic acids encoding said proteins, vectors comprising said nucleic acids, compositions comprising said nucleic acids or vectors, host cells comprising said nucleic acids, vectors or compositions or pharmaceutical compositions. The present application also provides methods of reducing (down regulating) a target membrane-bound protein (MBP) level in a cell, methods of producing a cell having a reduced target membrane-bound protein level, or methods of treating a disease, or methods of reducing or preventing GvHD in a subject associated with the administration of one or more CAR T-cells to the subject.

### BACKGROUND

The initial success of Chimeric Antigen Receptor (CAR) T cell therapy in the treatment of hematological malignancies has heralded rapid growth in a new area of immunotherapeutic treatment strategies (Seledtsov, V. I., Goncharov et al., Human Vaccines and Immunotherapeutics 11, 851-869 (2015)). CARs are designer receptors typically with modular activation domains derived from the CD3ζ ITAM motifs and a costimulatory domain, for example from 4-1BB or CD28 (Weinkove, R., George, P., et al., Clin. Transl. Immunol. 8, e1049 (2019)). Target recognition by these receptors is usually provided through a scFv domain on the extracellular terminal of the CAR, though alternative approaches have also been designed (Kuhn, N. F. et al. Cancer Cell 35, 473-488.e6 (2019)).

Two approved CD19 CAR-T drugs (Kymriah and Yescarta) as well as Abecma (BCMA CAR-T) are all autologous products. They are made from each individual patient's own T cells. While autologous CD19 CAR-T demonstrated unprecedented efficacy in refractory and relapse cancer patients, they do represent significant challenges moving forward: they require complex manufacturing; there are usually significant variations in the quality of CAR-T cells due to patient variations, and this results in variations in the quality of the product and even production failure (with failure rates up to 14%) (Bersenev, A. Transfusion (2017). doi:10.1111/trf.14110), (Dai, X., Mei, et al., Biotechnology Journal (2019)). Moreover, the lead time required to make the product is around 3-4 weeks, so patients who cannot wait this long due to disease progression will not be able to enter clinical trials; the cost is also too high to be affordable for the majority of the patient population. The cost per patient is $475,000 for Kymriah and $373,000 for Yescarta.

Allogeneic (off-the-shelf) CAR-T on the other hand will offer many advantages. The cells can be batch-prepared in advance and can supply thousands of doses; the product is frozen, stored, and distributed for off-the-shelf use; the CAR-T cells will be prepared from healthy donors with better cell quality; the cells will be of uniform quality per batch; there will be minimal lead time for patients; and the cost of goods can be significantly reduced (Rafiq, S. et, al., Nature Reviews Clinical Oncology (2020)), (Depil, S. et al., Nature Reviews Drug Discovery (2020)).

Currently there are several ways to prepare allogeneic product that will not cause Graft versus Host Disease (GvHD) in patients: they can be prepared by gene editing technology such as CRISPR (clustered regularly interspaced short palindromic repeats)-mediated TCR knockdown, or they can be prepared through siRNA down regulation of TCR, or they can be prepared with cells that naturally do not trigger GvHD such as cord blood or PBMC-derived NK cells (Li, Y. et al., Cell Stem Cell (2018)), NK92 cell line, or gamma delta T cells etc.(Marcus, A. et al., Expert Opinion on Biological Therapy (2014)), (Depil, S. et al., Nature Reviews Drug Discovery (2020)), (Themeli, M., Rivière et al., Cell Stem Cell (2015)), (Rezvani, K. et al., Molecular Therapy (2017)).

The major challenge with gene editing technology is that there is always a risk of off-target modification. The rate of off-target modification can be as high as 50% (Zhang, X. H. et al., Molecular Therapy - Nucleic Acids (2015)), which can lead to unwanted mutations and would pose a significant risk for patients. Furthermore, the manufacturing process is complicated as it requires two steps of modifications: the first step is to transfect the CAR into T cells, the second step is to introduce CRISPR machinery to knockout TCR.

NK cells and gamma delta T cells on the other hand are associated with less persistence and proliferation *in vivo,* which will significantly affect their potency. They are also difficult to expand *in vitro* (Li, Y, Cell Stem Cell (2018)), (Rezvani, K., et al., Molecular Therapy (2017)), (Shimasaki, N. et al., Nature Reviews Drug Discovery (2020)).

How to downregulate Membrane-Bound Proteins (MBPs) in T cells or other cells while avoiding the above-mentioned defects is a problem that needs to be solved.

WO2019/007869A provides a method of controlling the level of a polypeptide sequence comprising administering a polypeptide sequence fused to a ubiquitin targeting protein which comprises a minimal degron structural motif. In particular, WO2019/007869A discloses a chimeric antigen receptor having an ScFv antigen binding domain, a CD8alpha extracellular part and CD8alpha transmembrane domain, and a C-terminal degradation motif.

TAKAHIRO KAMIYA et al discloses a mechanism that is, to remove surface TCRaf3, an antibody-derived single-chain variable fragment specific for CD3eta was combined with 21 different amino acid sequences predicted to retain it intracellularly. After transduction in T cells, several of these protein expression blockers (PEBLS) co-localized intracellularly with CD3eta, blocking surface CD3 and TCRaβ expression ("A novel method to generate T-cell receptordeficient chimeric antigen receptor T cells", BLOOD ADVANCES, vol. 2, no.5, 13 March 2018 (2018-03-13), pages 517-528, DOI:10.1182/bloodadvances.2017012823).

### SUMMARY OF THE INVENTION

The present application provides novel fusion proteins, nucleic acids encoding said proteins, vectors comprising said nucleic acids, compositions comprising said nucleic acids or vectors, cells (e.g. Host cells) comprising said nucleic acids, vectors or compositions or pharmaceutical compositions. The present application also provides methods of reducing (down regulating) a target membrane-bound protein (MBP) level in a cell, methods of producing a cell having a reduced target membrane-bound protein level, or methods of treating a disease, or methods of reducing or preventing GvHD in a subject associated with the administration of one or more CAR T-cells to the subject.

In the present application, downregulation of Membrane-Bound Proteins (MBPs) is achieved by degradation tag protein-mediated degradation, wherein the degradation tag protein comprises ubiquitin ligase and/or domains of the cell surface receptors that mediate degradation (e.g. IL2Rβ sub domain, L2Rβ jm domain). The tagging specificity or targeting specificity (or recognition specificity) of the MBPs to be degraded by the degradation tag protein (e.g. ubiquitin ligase or IL2Rβ) is mediated by an antibody (or antigen-binding fragment) of MBPs to be degraded (that is, antibody mediated substrate recognition), or mediated by subunits or structural domains of the membrane-bound protein or complex to be degraded that have a transmembrane domain (that is, transmembrane domain mediated substrate recognition). The technical solution of the present application can be applied to any targets on a cell surface, independent of cell type.

In one aspect, the fusion protein of the application comprises:
a degradation tag protein that mediates degradation of a membrane-bound protein (MBP), wherein the degradation tag protein comprises an ubiquitin ligase, or one or more subunits or structural domains of the cell surface receptor which is an Interleukin receptor that mediate degradation; and
a transmembrane-linking domain comprising:
   1) a transmembrane domain and a linking protein;
      wherein the transmembrane domain is selected from the transmembrane domain of one or more subunits or structural domains of CD8α, CD4, CD3, CD28, 4-1BB and IL2R; and the linking protein is selected from one or more of an antibody or antigen-binding fragment specific binding to the MBP to be degraded; and the transmembrane domain is linked directly or indirectly to the linking protein; or
   2) a transmembrane domain of the MBP to be degraded;
      wherein
      (a) preferably, the MBP is a membrane-bound receptor; more preferably a mammalian origin membrane-bound receptor, most preferably a human membrane-bound receptor;
      (b) preferably, the ubiquitin ligase comprises one or more subunits or structural domains of E3 ubiquitin ligase or a variant thereof;
      (c) preferably, the antigen-binding fragment specific binding to MBP to be degraded in the above 1) comprises a scFv, nano antibody specifically binding to the MBP or a variant thereof;
      (d) preferably, the fusion protein having the transmembrane-linking domain of above 1) further comprises a signal peptide;
      (e) preferably, the transmembrane domain of the MBP to be degraded in the above 2) comprises a transmembrane domain of one or more of CD3ζ, CD3γ, CD3δ, CD3ε, CD3α, CD3β or a variant thereof; or
      (f) preferably, the transmembrane-linking domain of above 2) comprises an extracellular domain and a transmembrane domain of one or more of CD3ζ, CD3γ, CD3δ, CD3ε, CD3α, CD3β.
      (g) more preferably, the transmembrane-linking domain of above 2) comprises a signal peptide, extracellular domain and a transmembrane domain of one or more of CD3ζ, CD3γ, CD3δ, CD3ε, CD3α, CD3β.

In another specific embodiment, the membrane-bound receptor is selected from one or more subunits or structural domains of CD3, TCR, CD5, CD7, PD-L1, and CD47;
the E3 ubiquitin ligase is selected from the following proteins or the truncated forms thereof: C-terminus of Hsc70-interacting protein (CHIP), CHIPdTPR, F-box WD40-containing protein 7 (FBW7), FBW7.2-293, von Hippel-Lindau (VHL), VHL.152-213, Speckle-type BTB-POZ protein (SPOP), SPOP.167-374, SOCS2, SOCS2.143-198, Ubiquitin-protein ligase E3A(UBE3A), Mouse double minute 2 homolog (MDM2), Anaphase-promoting complex (APC), UBR5, LNX, Casitas B-lineage lymphoma-transforming sequence-like protein 1 (CBLL1), HECT domain and ankyrin repeat containing E3 ubiquitin protein ligase 1 (HACE1), HECT, C2 and WW domain containing E3 ubiquitin protein ligase 1 (HECW1), HECT, C2 and WW domain containing E3 ubiquitin protein ligase 2 (HECW2), HECT And RLD Domain Containing E3 Ubiquitin Protein Ligase 1 (HERC1), HERC2, HERC3, HERC4, HERC5, HERC6, HUWE1, ITCH, neural precursor cell expressed developmentally down-regulated protein 4 (NEDD4), Neural precursor cell expressed developmentally downregulated gene 4-like (NEDD4L), Peptidylprolyl isomerase (cyclophilin)-like 2 (PPIL2), PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UBOX5, UBR5, WWP1, WWP2, Parkin, MKRN1, GRAIL.IC, RNF133.IC, RNF122.rIC or RNF152.rIC; preferably, the E3 ubiquitin ligase comprises CHIP, CHIP.dTPR, FBW7.2-293, VHL.152-213, SPOP.167-374, SOCS2.143-198, GRAIL.IC, RNF133.IC, RNF122.rIC or RNF152.rIC; and more preferably, the CHIP, CHIP.dTPR, FBW7.2-293, VHL.152-213, SPOP.167-374, SOCS2.143-198, GRAIL.IC, RNF133.IC, RNF122.rIC or RNF152.rIC comprises an amino acid sequence at least about 95%, 96%, 97%, 98%, 99% or 100% identical to the amino acid sequence shown in any one of SEQ ID NOs. 21-26,62,79,81 or 83 respectively;
the subunits or structural domains of Interleukin receptors comprise a lysosomal targeting motif of IL-2R; preferably, the lysosomal targeting motif of IL-2R comprises L2Rβ jm domain; more preferably, the L2Rβ jm domain(IL2Rβjm) comprises an amino acid sequence at least about 95%, 96%, 97%, 98%, 99% or 100% identical to the amino acid sequence shown in SEQ ID NO. 58;
the first transmembrane domain in the above 1) is selected from a transmembrane domain of CD3, CD8α, CD4, preferably, CD3ζ transmembrane domain variant, CD8α transmembrane domain, or CD4 transmembrane domain comprises an amino acid sequence at least about 95%, 96%, 97%, 98%, 99% or 100% identical to the amino acid sequence shown in SEQ ID NO. 89, 18 or 15 respectively;
the signal peptide is selected from an amino acid sequence at least about 95%, 96%, 97%, 98%, 99% or 100% identical to the amino acid sequence shown in SEQ ID NO. 19 or 20 respectively;
the antibody or antigen-binding fragment of the MBP in the above 1) is selected from antibody or antigen-binding fragment of CD3, CD5, CD7, PD-L1 or CD47; preferably, antigen-binding fragment of CD3 comprises SP34 scFv, OKT3 scFv, UCHT1 scFv, UCHT1.Y177T scFv, L2K scFv, F6A scFv, BMA031 scFv, or BMA031.H6L12 scFv; antigen-binding fragment of CD5 comprises αCD5.14 scFv; antigen-binding fragment of CD7 comprises αCD7.TH69 scFv; antigen-binding fragment of PD-L1 comprises αPD-L1 scFv; antigen-binding fragment of CD47 comprises αCD47 scFv; more preferably, the SP34 scFv, OKT3 scFv, UCHT1 scFv, UCHT1.Y177T scFv, L2K scFv, F6A scFv, BMA031 scFv, BMA031.H6L12 scFv, αCD5.14 scFv, αCD7.TH69 scFv, αPD-L1 scFv or αCD47 scFv comprises an amino acid sequence at least about 95%, 96%, 97%, 98%, 99% or 100% identical to the amino acid sequence shown in any one of SEQ ID NOs. 2-13 respectively;
the transmembrane-linking domain of above 2) comprises a transmembrane domain of CD3ζ or CD3ε, preferably, an extracellular domain and a transmembrane domain of CD3ζ or CD3ε, more preferably, a second signal peptide, extracellular domain and a transmembrane domain of CD3ζ or CD3ε; more preferably, the transmembrane domain(TM) of CD3ζ or CD3ε comprises an amino acid sequence at least about 95%, 96%, 97%, 98%, 99% or 100% identical to the amino acid sequence shown in SEQ ID NO. 67, 70 respectively; more preferably, the extracellular domain(EM) of CD3ζ or CD3ε comprises an amino acid sequence at least about 95%, 96%, 97%, 98%, 99% or 100% identical to the amino acid sequence shown in SEQ ID NO. 66, 69 respectively; more preferably, the second signal peptide of CD3ζ or CD3ε comprises an amino acid sequence at least about 95%, 96%, 97%, 98%, 99% or 100% identical to the amino acid sequence shown in SEQ ID NO. 65, 68 respectively; more preferably, the second signal peptide, extracellular domain and a transmembrane domain of CD3ζ (CD3ζ.ΔIC) and the second signal peptide, extracellular domain and a transmembrane domain of CD3ε (CD3ε.ΔIC) comprises an amino acid sequence at least about 95%, 96%, 97%, 98%, 99% or 100% identical to the amino acid sequence shown in SEQ ID NO. 61, 64 respectively.

In a specific embodiment, the fusion protein further comprises one or more of a hinge, or a P2A-GFP;
preferably, the hinge is selected from a CD8α hinge or CD4 hinge; more preferably, CD8α hinge or CD4 hinge comprises an amino acid sequence at least about 95%, 96%, 97%, 98%, 99% or 100% identical to the amino acid sequence shown in SEQ ID NO.:16 or 14, respectively; more preferably, the hinge is adjacent to the N-terminal of the first transmembrane domain,
preferably, P2A-GFP comprises an amino acid sequence at least about 95%, 96%, 97%, 98%, 99% or 100% identical to the amino acid sequence shown in SEQ ID NO.:57; more preferably, the P2A-GFP sequence is at the C-terminal of the fusion protein.

In another specific embodiment, the fusion protein comprises from N-terminal to C-terminal:
a) the linking protein, the degradation tag protein, the first transmembrane domain;
   preferably, the linking protein comprises the antibody or antigen-binding fragment of the MBP to be degraded; more preferably, the antibody or antigen-binding fragment of the MBP to be degraded comprises antibody or antigen-binding fragment of CD3, CD5, CD7, PD-L1 or CD47; more preferably, antigen-binding fragment of CD3 comprises SP34 scFv, OKT3 scFv, UCHT1 scFv, UCHT1.Y177T scFv, L2K scFv, F6A scFv, BMA031 scFv, or BMA031.H6L12 scFv; antigen-binding fragment of CD5 comprises αCD5.14 scFv; antigen-binding fragment of CD7 comprises αCD7.TH69 scFv; antigen-binding fragment of PD-L1 comprises αPD-L1 scFv; antigen-binding fragment of CD47 comprises αCD47 scFv;
   preferably, the degradation tag protein comprises E3 ubiquitin ligase; more preferably, the E3 ubiquitin ligase comprises CHIP.dTPR, (CHIP: C-terminus of Hsc70-interacting protein), FBW7.2-293 (FBW7: F-box WD40-containing protein 7), VHL.152-213 (von Hippel-Lindau), SPOP.167-374 (Speckle-type BTB-POZ protein),or SOCS2.143-198;
   preferably, the first transmembrane domain comprises CD8α transmembrane domain;
b) the linking protein, the first transmembrane domain, the degradation tag protein;
   preferably, the first linking protein comprises the antibody or antigen-binding fragment of the MBP to be degraded; more preferably, the antibody or antigen-binding fragment of the MBP to be degraded comprises the antibody or antigen-binding fragment of CD3; more preferably, antigen-binding fragment of CD3 comprises SP34 scFv;
   preferably, the degradation tag protein comprises an E3 ubiquitin ligase; more preferably, E3 ubiquitin ligase comprises GRAIL.IC;
   preferably, the transmembrane domain is selected from one or more of CD3ζ transmembrane domain variant, CD8α transmembrane domain, and CD4 transmembrane domain;
c) the transmembrane-linking domain of the above 2), the cell surface receptors that mediate degradation;
   preferably, the transmembrane-linking domain of the above 2) comprises CD3ζ.ΔIC or CD3ε.ΔIC;
   preferably, the cell surface receptors that mediate degradation comprise IL2Rβjm; or
d) the transmembrane-linking domain of the above 2), the one or more of an E3 ubiquitin ligase or a variant thereof;
   preferably, the transmembrane-linking domain of the above 2) comprises CD3ζ.ΔIC;
   preferably, the one or more of an E3 ubiquitin ligase or a variant thereof comprises GRAIL.IC, CHIP.dTPR, RNF133.IC, RNF122.rIC, RNF152.rIC.

In a further specific embodiment, the fusion protein comprises from N-terminal to C-terminal:
a):
   LG112, SP-OKT3 scFv-CHIP.dTPR-CD8α hinge/TM-P2A-eGFP;
   LG114, SP-OKT3 scFv-FBW7-CD8α hinge/TM-P2A-eGFP;
   LG115, SP-OKT3 scFv-VHL-CD8α hinge/TM-P2A-eGFP;
   LG116, SP-OKT3 scFv-SPOP-CD8α hinge/TM-P2A-eGFP;
   LG117, SP-OKT3 scFv-SOCS2-CD8α hinge/TM-P2A-eGFP;
   LG118, SP-SP34 scFv-hCHIP.dTPR-CD8α hinge/TM-P2A-eGFP;
   LG123, SP-UCHT1 scFv-hCHIP.dTPR-CD8α hinge/TM-P2A-eGFP;
   LG124, SP-UCHT1.Y177T scFv-hCHIP.dTPR-CD8α hinge/TM-P2A-eGFP;
   LG125, SP-L2K scFv-hCHIP.dTPR-CD8α hinge/TM-P2A-eGFP;
   LG126, SP-F6A scFv-hCHIP.dTPR-CD8α hinge/TM-P2A-eGFP;
   LG119, SP-BMA031.wt scFv-hCHIP.dTPR-CD8α hinge/TM-P2A-eGFP;
   LG120, SP-BMA031.H6L12 scFv-hCHIP.dTPR-CD8α hinge/TM-P2A-eGFP;
   LG137, SP-αCD5.14 scFv-hCHIP.dTPR-CD8α hinge/TM-P2A-eGFP;
   LG138, SP-αCD7.TH69 scFv-hCHIP.dTPR-CD8α hinge/TM-P2A-eGFP;
   MLB052, SP-αPD-L1 scFv-hCHIP.dTPR-CD8α hinge/TM-P2A-eGFP;
   MLB053, SP-αCD47 scFv-hCHIP.dTPR-CD8α hinge/TM-P2A-eGFP;
b):
   LG222, SP-SP34-CD3ζ.ΔIC-1 -GRAIL.IC-P2A-eGFP;
   LG222.1, SP-SP34-CD8α hinge/TM -GRAIL.IC-P2A-eGFP;
   LG222.2, SP-SP34-CD4 hinge/TM -GRAIL.IC-P2A-eGFP;
c):
   MLB014, CD3ζ.ΔIC-IL2Rβjm-P2A-eGFP;
   MLB046, CD3ε.ΔIC-IL2Rβjm-P2A-eGFP;
d):
   LG171, CD3ζ.ΔIC-GRAIL.IC-P2A-eGFP;
   LG171p1, CAG-CD3ζ.ΔIC-GRAIL.IC-P2A-eGFP;
   LG213, CD3ζ.ΔIC-CHIP. dTPR-P2A-GFP;
   LG174, CD3ζ.ΔIC-RNF133.IC-P2A-GFP;
   LG180, CD3ζ.ΔIC-RNF122.rIC-P2A-GFP;
   LG183, CD3ζ.ΔIC-RNF152.rIC-P2A-GFP;
   optionally, the fusion protein does not include P2A-eGFP(i.e. P2A-GFP) and/or SP.

In another aspect, the present application provides a nucleic acid, wherein the nucleic acid comprises a polynucleotide encoding a fusion protein of the present application.

In another aspect, the present application provides a vector, wherein the vector comprises a nucleic acid of the present application.

In a specific embodiment, preferably, an expression promoter for the fusion protein comprises CAG or EF1a;
more preferably, the CAG or EF1a comprises a nucleic acid sequence at least about 95%, 96%, 97%, 98%, 99% or 100% identical to the nucleic acid sequence shown in SEQ ID NO.: 72 or 88, respectively;
more preferably, the expression promoter for the a) fusion protein is EF1a;
more preferably, the expression promoter for the b) fusion protein is EF1a;
more preferably, the expression promoter for the c) fusion protein is EF1a;
more preferably, the expression promoter for the LG171p1 in d) fusion protein is CAG, more preferably, the expression promoter for the other fusion proteins in d) fusion protein is EF1a.

In another aspect, the present application provides a composition, the composition comprising a nucleic acid or a vector of the present application.

In another aspect, the composition comprises a first nucleic acid and a second nucleic acid; or comprising a first vector having a first nucleic acid and a second vector having a second nucleic acid; or comprising a vector having a first nucleic acid and a second nucleic acid, wherein
(1)the first nucleic acid encodes the fusion protein of the present application;
(2)the second nucleic acid encodes a chimeric antigen receptor (CAR) comprising:
   (a)an extracellular ligand-binding domain comprising a single chain variable fragment (scFv) specifically binding to a predetermined antigen;
   (b)a transmembrane domain; and
   (c)a cytoplasmic segment comprising one or more signaling domains, preferably comprising a 4-1BB signaling domain and a CD3ζ signaling domain.

In a specific embodiment, the fusion protein in the composition, which comprises a first nucleic acid and a second nucleic acid; or comprising a first vector having a first nucleic acid and a second vector having a second nucleic acid; or comprising a vector having a first nucleic acid and a second nucleic acid, is selected from one or more of the above a)-d) fusion proteins.

In a specific embodiment, the predetermined antigen is a tumor-related antigen.

In a further specific embodiment, the tumor-related antigen is selected from the following group: CEA, Claudin 18.2, GPC3, Receptor tyrosine kinase-like Orphan Receptor 1 (ROR1), CD38, CD19, CD20, CD22, BCMA, CAIX, CD446, CD133, EGFR, EGFRvIII, EpCam, GD2, EphA2, Her1, Her2, ICAM-1, IL13Ra2, Mesothelin, MUC1, MUC16, NKG2D, PSCA, NY-ESO-1, MART-1, WT1, MAGE-A10, MAGE-A3, MAGE-A4, EBV, NKG2D, PD1, PD-L1, CD25, IL-2, or CD3.

In a further specific embodiment, the tumor-related antigen is CEA.

In another aspect, the present application provides a cell (e.g. host cell), the cell comprising a nucleic acid or a vector or a composition of the present application.

In a specific embodiment, the host cell is a mammalian cell, preferably a human cell.

In a further specific embodiment, the host cell is a T cell or a primary T cell, gamma delta T cell, NK cell, NKT cell, macrophage, B cell, or non-immune cell.

In a further specific embodiment, the host cell is an allogeneic cell.

In another aspect, the present application provides a pharmaceutical composition, the pharmaceutical composition comprising the fusion protein, the nucleic acid, vector, composition or cell of the present application.

In a specific embodiment, the composition further comprises one or more pharmaceutically acceptable excipients.

In another aspect, the present application provides a method of reducing a target membrane-bound protein level in a cell, comprising introducing into a cell a nucleic acid, a vector, or a composition of the present application.

In a specific embodiment, the cell (e.g. host cell) is a mammalian cell, preferably a human cell.

In a further embodiment, the host cell is a T cell or a primary T cell, gamma delta T cell, NK cell, NKT cell, macrophage, B cell, or non-immune cell.

In a further embodiment, the host cell is an allogeneic cell.

In another aspect, the present application provides a nucleic acid, a vector, or a composition of the present application for use in a method of producing a cell having reduced target membrane-bound protein level.. Preferably, the cell is a mammalian cell, more preferably a human cell; more preferably, the cell is a T cell or a primary T cell, gamma delta T cell, NK cell, NKT cell, macrophage, B cell, or non-immune cell; most preferably the cell is an allogeneic cell.

In another aspect, the present application provides a pharmaceutical composition, the pharmaceutical composition comprising the fusion protein, the nucleic acid, vector, composition or cell of the present application for use in a method of treating a disease, comprising administering to a subject in need thereof a therapeutically effective amount of allogeneic cells having the composition of the present application or administering to a subject in need thereof a therapeutically effective amount of cells of the present application, wherein preferably, the subject has reduced Graft-versus-Host Disease (GvHD). Preferably, the cell is a mammalian cell, more preferably a human cell; more preferably, the cell is a T cell or a primary T cell, gamma delta T cell, NK cell, NKT cell, macrophage, B cell, or non-immune cell; most preferably the cell is an allogeneic cell.

In another aspect, the present application provides a method of reducing or preventing GvHD in a subject associated with the administration of one or more CAR T-cells to the subject, comprising
(1) transfecting the one or more CAR T-cells with a nucleic acid or vector or composition of the application, and
(2) administering the CAR T-cells to the subject.

In another aspect, the present application provides a method of downregulating membrane-bound protein (MBP) in a cell population, comprising adding to said cell population a host cell of the present application, wherein the host cell expresses fusion protein comprising scFv specifically binding to said MBP, and/or the host cell expresses fusion protein comprising a component of MBP or a fragment thereof that has a transmembrane domain.

In a further aspect, the host cell population is a T cell population, preferably a human primary T cell population.

In a further aspect, the MBP is CD3, preferably human CD3

The present application has the following advantages:
1) The fusion protein of the present application could reduce (down regulate) a target membrane-bound protein (MBP) level in a cell; and
2) CAR T-cells of the present application could reduce or prevent GvHD in a subject.

### BRIEF DESCRIPTION OF THE DRAWINGS

The novel features of the present disclosure are set forth with particularity in the appended claims. A better understanding of the features and advantages of the present disclosure are explained in the following detailed description in the embodiments and the examples.

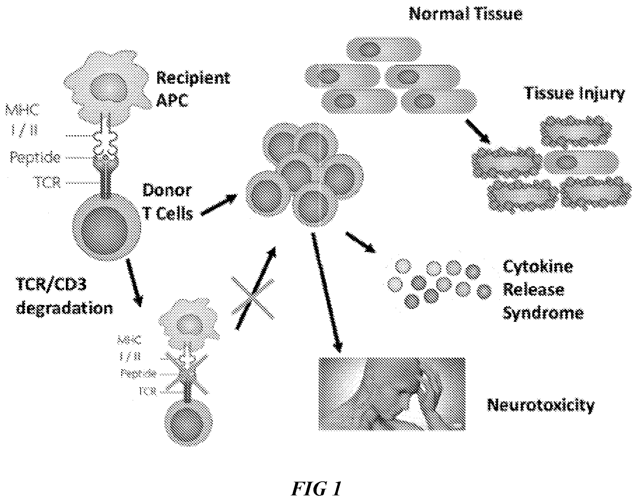

**Fig. 1** **Cartoon for allogenic CAR-T host cells generation to avoid potential GvHD responses against recipient tissues.** Due to different development/maturation experiences of T cells, donor T cells, when infused into different recipients, may potentially elicit strong immune responses, e.g. cytotoxicity, cytokine release syndrome and neurotoxicity, against foreign antigens (GvHD). By degrading TCR/CD3 complex in donor cells, such kind of unfavorable effects could be largely eliminated.

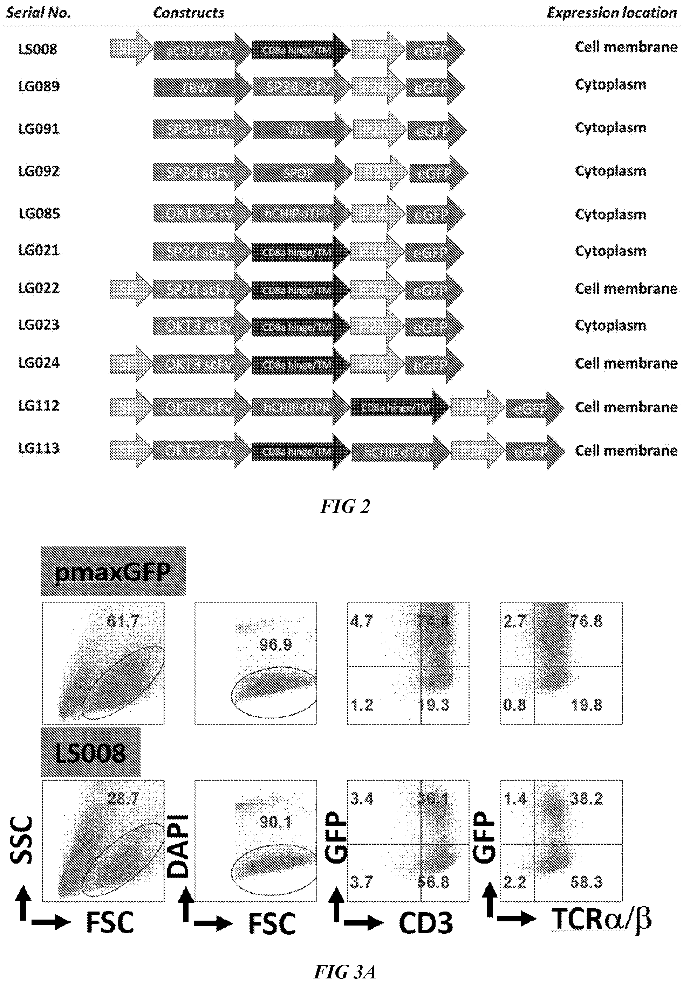

**Fig. 2** **Schematic representation of TCR/CD3 Degradation constructs and Reference constructs.** A list of E3 ligases or a variant thereof (functional domain of E3 ligases) (e.g.FBW7, VHL, SPOP and CHIP.dTPR), were tested by linking to scFv against human CD3 for TCR/CD3 complex degradation purposes. Either clone SP34 or OKT3 was adopted for scFv generation. Some of these constructs were further anchored on the cell membrane through a CD8α hinge / transmembrane domain. LS008 construct (CD19 CAR) was adopted here as a negative control. Cytoplasmic constructs (LG089, LG091, LG092, LG085 constructs) obtained by engaging antibody or antigen-binding fragment of the CD3 to E3 ligase/ubiquitin, but have no SP sequence and no hinge/TM sequence, and expressed in cytoplasm of a cell. Cytoplasmic or Membrane-anchored constructs (LG021, LG022, LG023, LG024 constructs) have no E3 ligase/ubiquitin sequence, only antibody or antigen-binding fragment of the CD3. TCR/CD3 degradation constructs (LG112, LG113) were obtained by engaging antibody or antigen-binding fragment of the CD3 to E3 ligase/ubiquitin, and have both SP sequence and hinge/TM sequence.

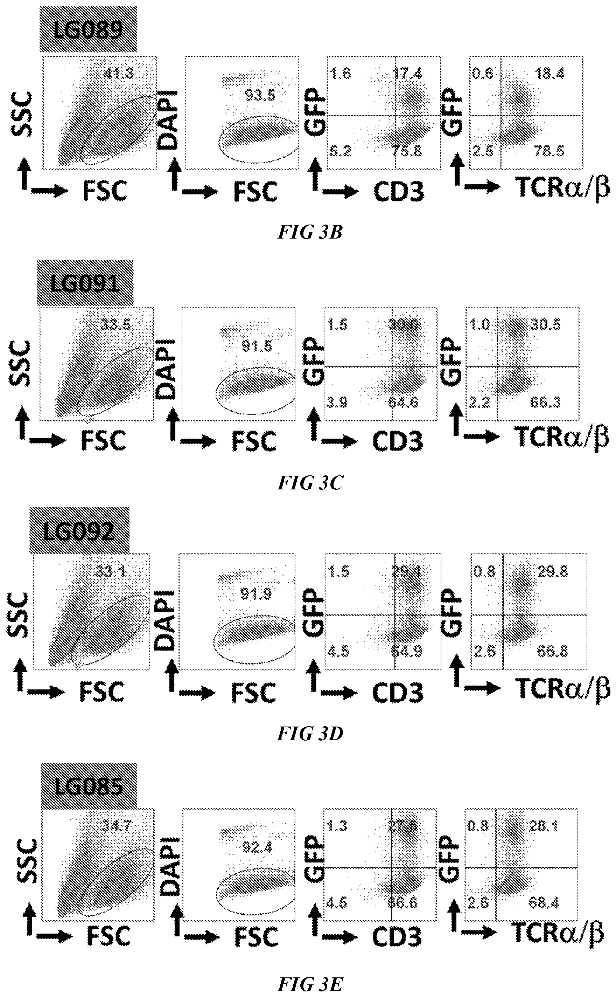

**Fig. 3A-3E** **Effect of cytoplasmic constructs (LG089, LG091, LG092, LG085) on TCR/CD3 complex in human primary T cells.** Human isolated primary T cells were activated *in vitro* using StemCell Immunocult-XF/Activator for 3 days, in the presence of human IL-2, IL-7 and IL-15. Then activated primary T cells were collected and electroporated in the presence of plasmids expressing one of the cytoplasmic constructs for TCR/CD3 degradation. Two days later, cells were harvested and stained for both CD3 (PE) and TCRα/β (APC). **Fig.3A** shows systemic controls pmaxGFP and LS008 has good transfection efficiency in primary T cells, and there was no downregulation of TCRα/β and CD3 in either of these two constructs. FACS results of Fig. 3B-3E show no obvious degradation of TCR/CD3 complex when anti-CD3 scFv (e.g. SP34 scFv or OKT3 scFv) conjugated E3 ligases, including FBW7 **(****Fig.3B****),** VHL **(****Fig.3C****),** SPOP **(****Fig.3D****)** and hCHIP.dTPR **(****Fig.3E****),** were expressed in cytoplasm.

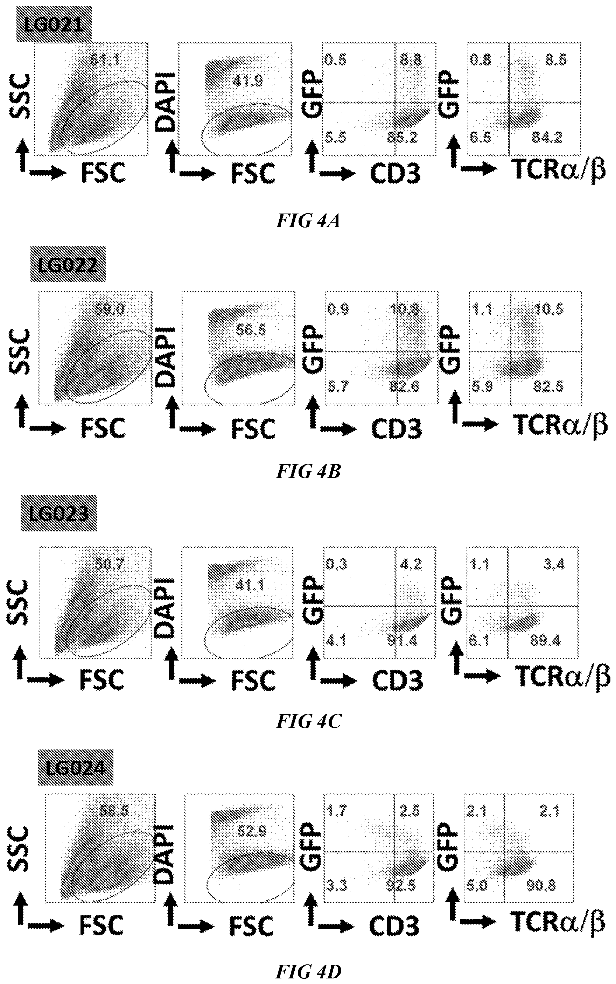

**Fig. 4A-4D** **Membrane-anchored OKT3 construct (LG021, LG022, LG023, LG024) confers downregulation of TCR/CD3 complex in human primary T cells.** Human isolated primary T cells were activated *in vitro* using StemCell Immunocult-XF/Activator for 3 days, in the presence of human IL-2, IL-7 and IL-15. Then cells were collected and electroporated with 1 µg DNA plasmid expressing one of the cytoplasmic/membrane-anchored constructs for TCR/CD3 degradation. Two days later, cells were harvested and stained for both CD3 (PE) and TCRα/β (APC). There was no significant downregulation of TCR/CD3 complex when SP34 scFv (LG021) **(****Fig.4A****)** or OKT3 scFv (LG023) **(****Fig.4C****)** dependent constructs were expressed in cytoplasm. Similarly, membrane-anchored SP34 scFv constructs (LG022) did not show any effects either **(****Fig.4B****).** However, when OKT3 scFv sequence was adopted in the membrane-anchored format, significant downregulation of TCR/CD3 complex was observed (LG024) **(****Fig.4D****).**

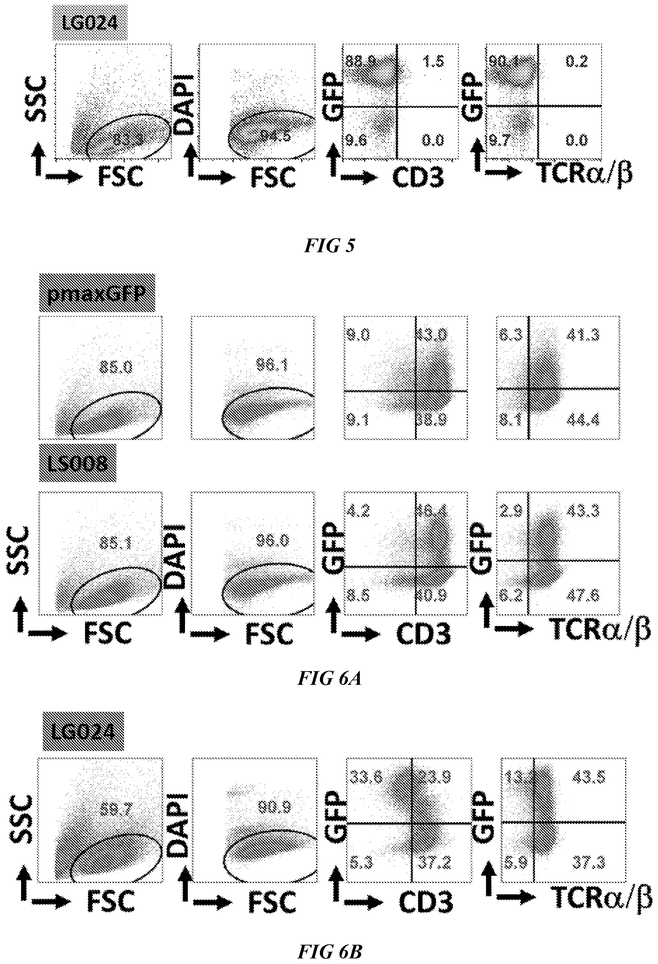

**Fig. 5** **Membrane-anchored OKT3 construct confers downregulation of TCR/CD3 complex in Jurkat cells.** Jurkat cells were electroporated with both PiggyBac transposase mRNA and transposon expressing the membrane-anchored OKT3 construct(LG024). The Jurkat Cells were maintained until day 57 and subjected to flow cytometry testing post staining with CD3 (PE) and TCRα/β (APC), showing significant degradation of TCR/CD3 complex in cells with stable integration of transposon expressing the membrane-anchored OKT3 construct(LG024) (GFP positive).

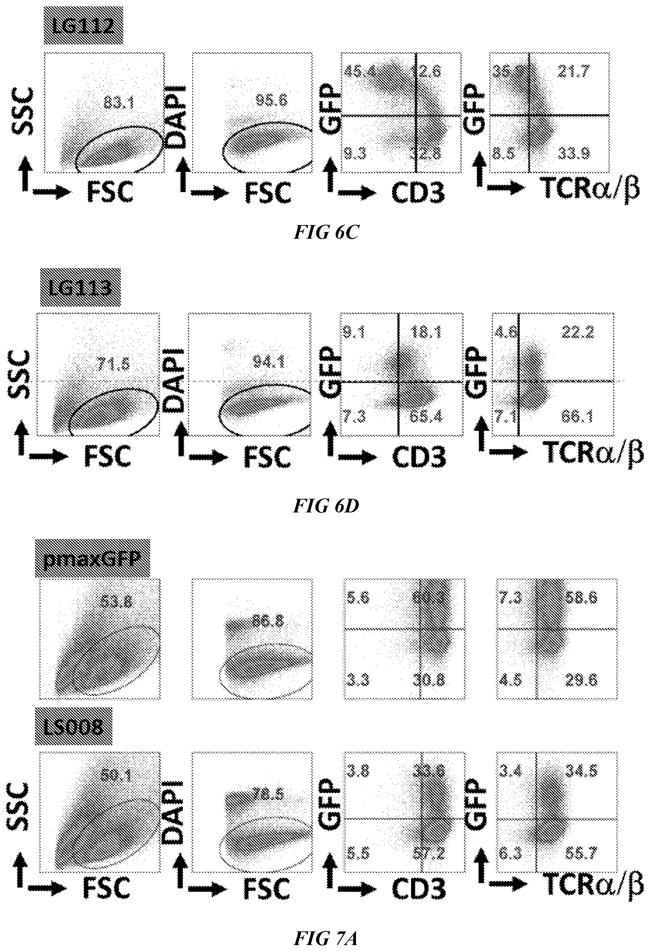

**Fig. 6A-6D** **The membrane outside linkage of E3 ligase to OKT3 scFv construct (LG112) confers better downregulation of TCR/CD3 complex in Jurkat cells line than cytoplasmic linkage of E3 ligase to OKT3 scFv construct(LG113)**. Jurkat cells were electroporated with both PiggyBac transposase mRNA and transposon expressing different membrane-anchored OKT3 constructs (LG112, LG113). The Jurkat Cells were harvested on day 3 and subjected to flow cytometry analysis post staining with CD3 (PE) and TCRα/β (APC). **Fig.6A** shows systemic controls pmaxGFP and LS008 have good transfection efficiency in Jurkat cells, and there was no downregulation of TCRα/β and CD3 in either of these two constructs. Membrane-anchored OKT3 scFv construct without E3 ubiquitin ligase (LG024) shows significant downregulation of TCR-CD3 complex in Jurkat cells **(****Fig.6B****).** When hCHIP.dTPR was linked to OKT3 scFv in an outside membrane manner (proximal linkage) (LG112) **(****Fig.6C****),** instead of cytoplasmic manner (LG113) **(****Fig.6D****),** even stronger downregulation of TCR/CD3 complex was obtained.

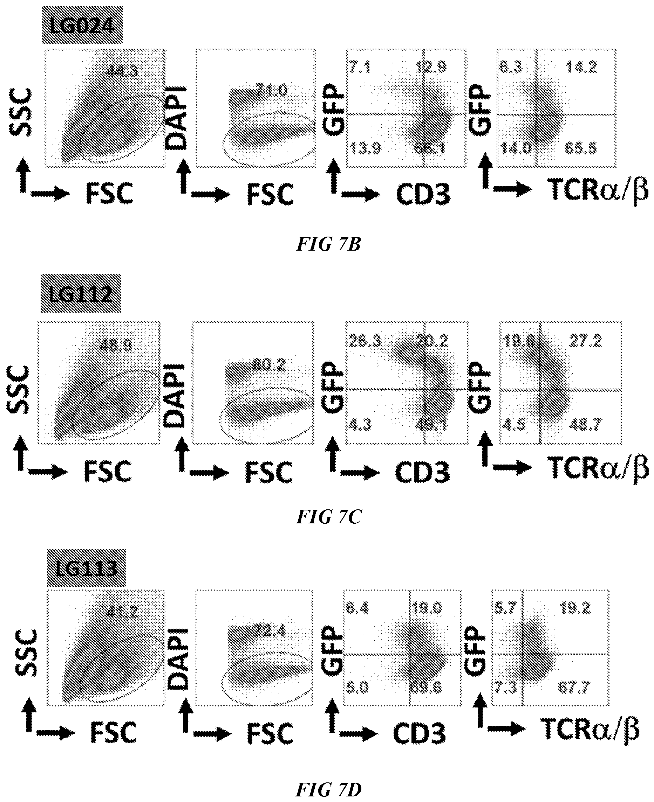

**Fig. 7A-7D** **The membrane outside linkage of E3 ligase to OKT3 construct (LG112) confers better downregulation of TCR/CD3 complex in human primary T cells than cytoplasmic linkage of E3 ligase to OKT3 scFv construct (LG113).** Human isolated primary T cells were activated *in vitro* using StemCell Immunocult-XF/Activator for 3 days, in the presence of human IL-2, IL-7 and IL-15. Then the activated primary T cells were collected and electroporated with both PiggyBac transposase mRNA and transposon expressing different membrane-anchored OKT3 constructs. Three days later, the primary T cells were harvested and stained for both CD3 (PE) and TCRα/β (APC). **Fig.7A** shows systemic controls pmaxGFP and LS008 have good transfection efficiency in primary T cells, and there was no downregulation of TCRα/β and CD3 in either of these two constructs. Similar to what we found in Jurkat cell line, the degradation could be more significant when hCHIP.dTPR was linked to OKT3 scFv in an outside membrane manner (proximal linkage) (LG112) **(****Fig.7C****),** instead of cytoplasmic manner (LG113) **(****Fig.7D****)** .No significant changes on TCR/CD3 degradation were found when E3 ubiquitin ligase (CHIP.dTPR) was located in the cytoplasmic portion of the whole construct (LG113, **Fig. 7D**) in comparison to Membrane-anchored OKT3 scFv construct without E3 ubiquitin ligase (LG024) **(****Fig. 7B****).**

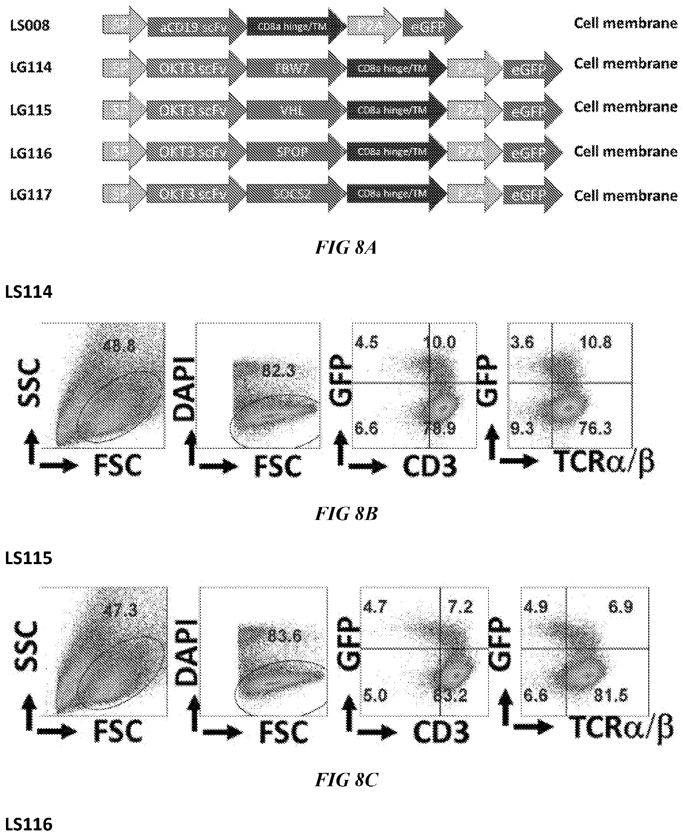

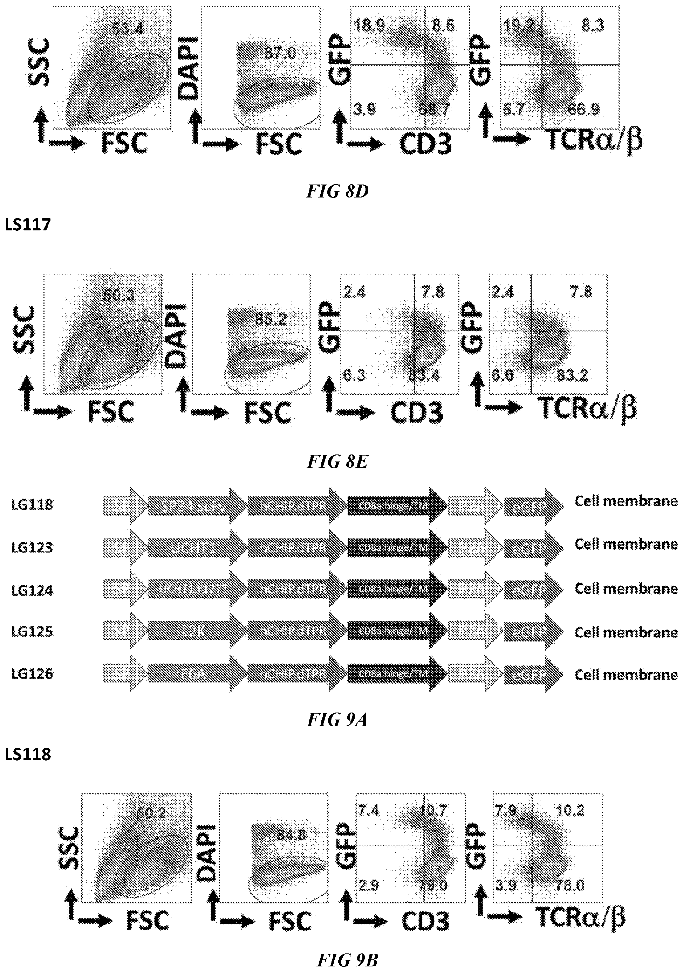

**Fig. 8A-8E** **TCR/CD3 Degradation constructs with different E3 ligases.** (Fig. 8A) A list of constructs with different E3 ligases, including FBW7, VHL, SPOP and SOCS2, linked to membrane anchored OKT3 scFv, were designed and synthesized. Human isolated primary T cells were activated *in vitro* using StemCell Immunocult-XF/Activator for 3 days, in the presence of human IL-2, IL-7 and IL-15. Then the primary T cells were collected and electroporated with both PiggyBac transposase mRNA and transposon expressing different membrane-anchored OKT3 constructs. Three days later, the primary T cells were harvested and stained for both CD3 (PE) and TCRα/β (APC). FBW7 (Fig. 8B), VHL (Fig. 8C), SPOP (Fig. 8D) and SOCS2 (Fig. 8E) were tested for TCR/CD3 downregulation effects.

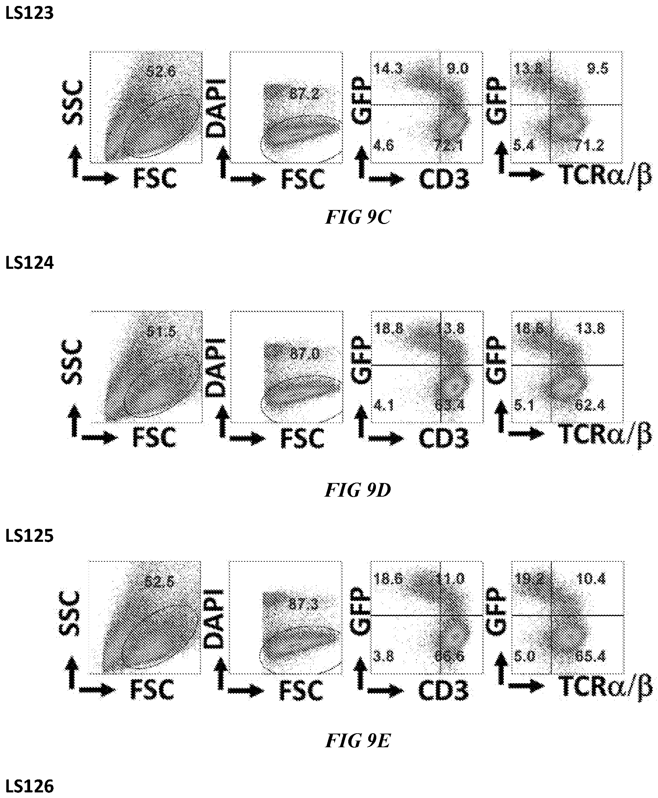

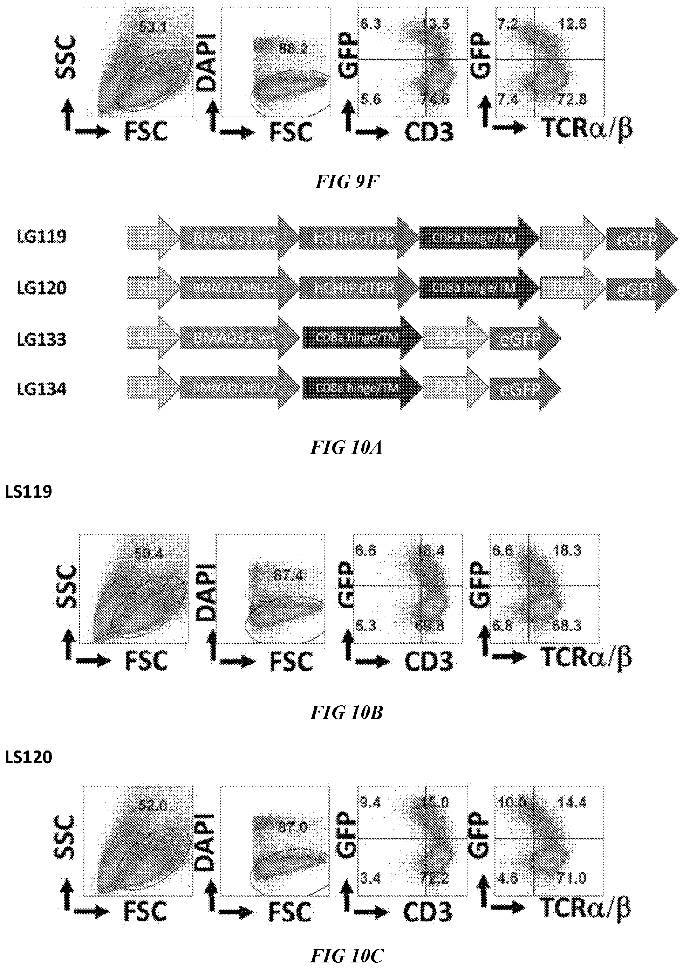

**Fig. 9A-9F** **TCR/CD3 Degradation constructs with different CD3 targeting scFv.** (Fig. 9A) A list of TCR/CD3 degradation constructs with different TCR-CD3 targeting scFv, including SP34, UCHT1, UCHT1.Y177T, L2K and F6A, were designed and synthesized. Human isolated primary T cells were activated *in vitro* using StemCell Immunocult-XF/Activator for 3 days, in the presence of human IL-2, IL-7 and IL-15. Then the primary T cells were collected and electroporated with both PiggyBac transposase mRNA and transposon DNA constructs. Three days later, the primary T cells were harvested and stained for both CD3 (PE) and TCRα/β (APC). TCR/CD3 downregulation effects by SP34 (Fig. 9B), UCHT1 (Fig. 9C), UCHT1.Y177T (Fig. 9D), L2K (Fig. 9E) and F6A (Fig. 9F) were tested.

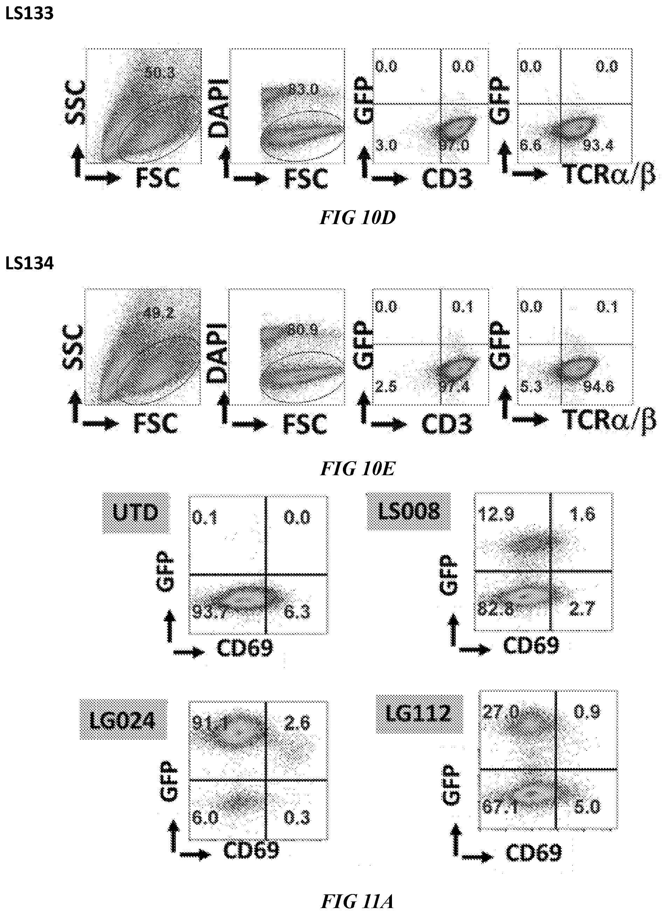

**Fig. 10A-10E** **E3 ligase (CHIP.dTPR) is necessary for optimized TCR/CD3 downregulation in human primary T cells.** (Fig. 10A) A list of TCR degradation constructs with TCR targeting scFv, BMA031 or BMA031.H6L12, were designed and synthesized in the presence or absence of CHIP.dTPR. Human isolated primary T cells were activated *in vitro* using StemCell Immunocult-XF/Activator for 3 days, in the presence of human IL-2, IL-7 and IL-15. Then cells were collected and electroporated with both PiggyBac transposase mRNA and transposon DNA constructs. Three days later, the primary T cells were harvested and stained for both CD3 (PE) and TCRα/β (APC). TCR/CD3 downregulation effects by BMA031 (LG119, Fig. 10B) (LG133, Fig. 10D) or BMA031. H6L12 (LG120, Fig. 10C) (LG134, Fig. 10E) were tested in the presence (Fig. 10B) (Fig. 10C) or absence (Fig. 10D) (Fig. 10E) of CHIP.dTPR.

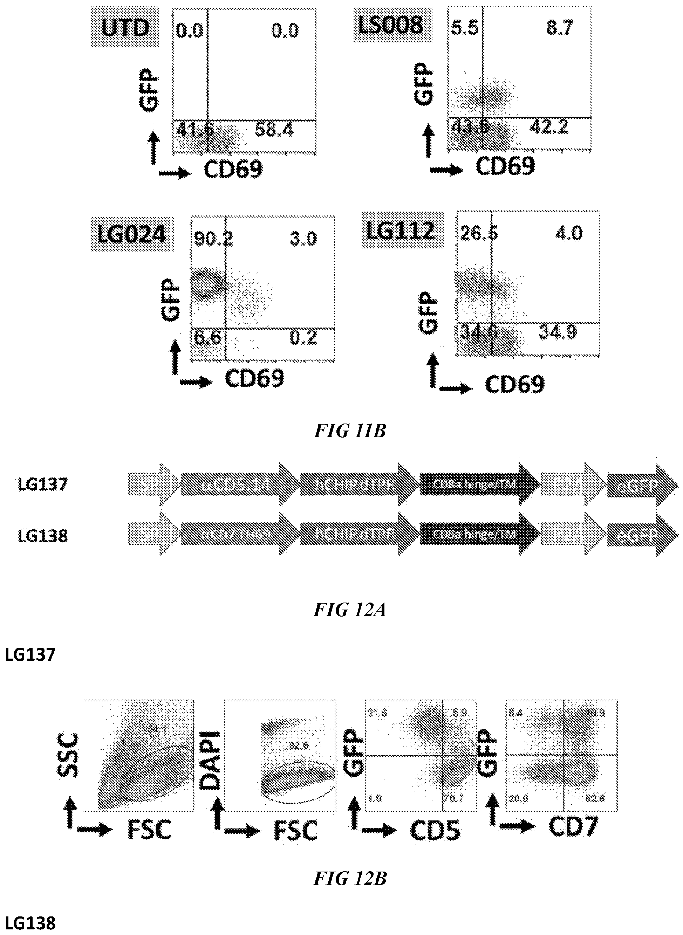

**Fig. 11A****-** **Fig.11B** **show CD3 downregulation confers lower activation of Jurkat cells through CD3/CD28/CD2 stimulation.** **Fig. 11A** shows that CD69 (a T cell activation marker) expression levels were low for all cells at the baseline; **Fig. 11B** shows the CD69 expression levels in either LG024 or LG112 transfected GFP+ cells were significantly less compared to T cells LS008 transfected cells at 4 hours post stimulation.

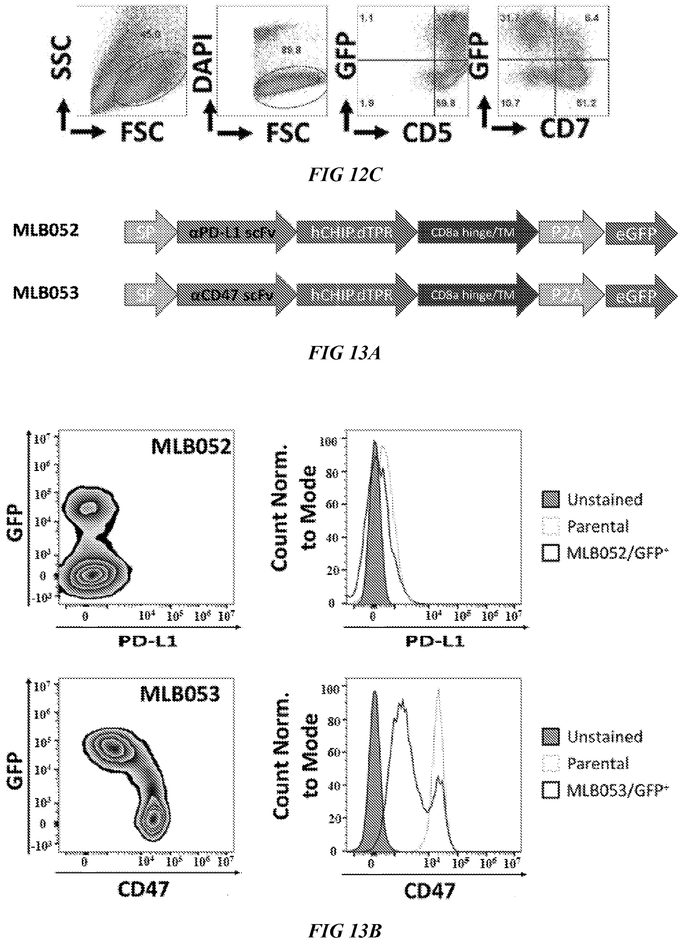

**Fig. 12A-12C** **CD5 or CD7 downregulation constructs and validation.** Fig. 12A shows a list of CD5 or CD7 degradation constructs were designed and synthesized. Fig. 12B shows αCD5.14 scFv conferred strong downregulation of CD5; Fig. 12C shows αCD7. TH69 scFv (CD7-targeting scFv) also showed significant downregulation effect in human primary T cells.
**Fig.13A-13B** **Down regulation of PD-L1 and CD47 by E3 CHIP construct.** Fig. 13A shows schematic representation of E3 Constructs MLB052 and MLB053, which are specific to PD-L1 and CD47, respectively. Each construct is comprised of an N-terminal ScFv domain specific to the target antigen and a truncated version of the E3 ligase CHIP lacking the TPR domain. The E3 degrader construct (MLB052 or MLB053) is anchored to the membrane by a flexible hinge and transmembrane anchor. For PD-L1, 1) Representative flow cytometry plot showing two distinct populations of Jurkat Cells, GFP+ and GFP-(i.e. upper panel of left column of Fig. 13B), Jurkat cells were stained with PE-conjugated anti-PD-L1 antibody; 2) Histogram plot of PD-L1 expression on E3-CHIP-expressing cells, gated on GFP+ expression(i.e. the black line marked area in the upper panel of right column of Fig. 13B), relative to untransfected Jurkat parental cells(i.e. dashed line marked area in the upper panel of right column of Fig. 13B). For CD 47, 1) Representative flow cytometry plot of Jurkat cells transfected with anti-CD47 E3 CHIP (GFP+) (i.e. the lower panel of left column of Fig. 13B) and stained with an APC-conjugated anti-CD47 primary antibody; 2) Histogram showing CD47 expression of E3 CHIP transfected Jurkat cells, gated on GFP+ expression, relative to untransfected parental control cells (the lower panel of right column of Fig. 13B).

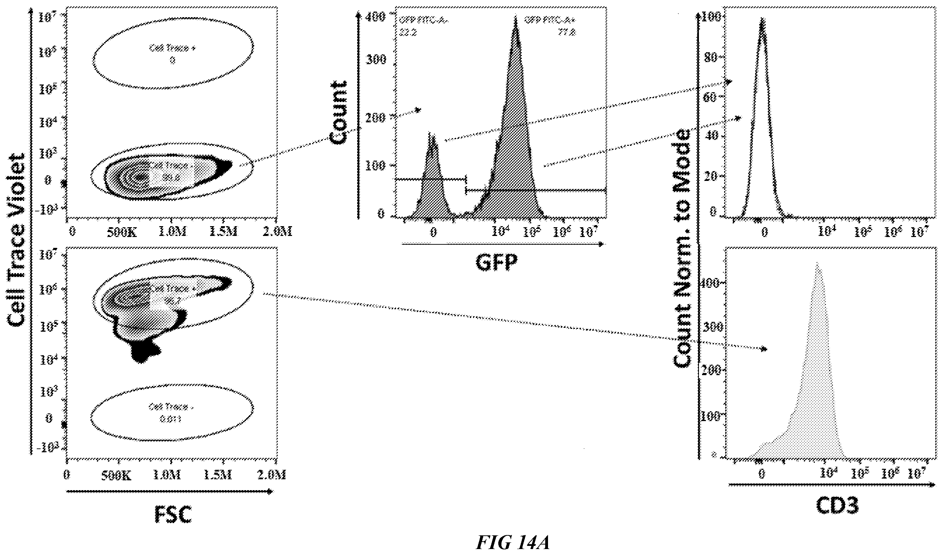

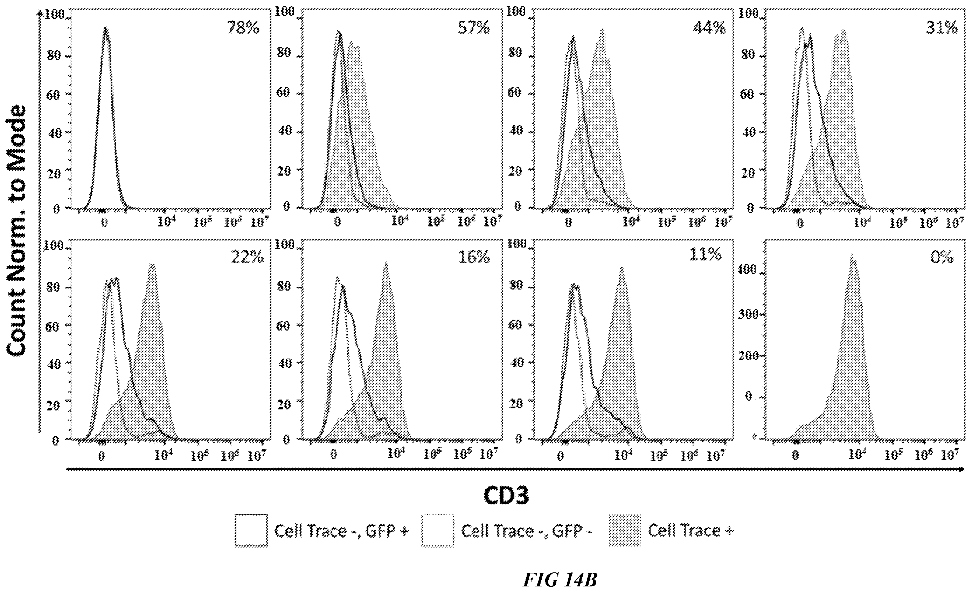

**Fig.14A****-****Fig.14B** **Paracrine downregulation of TCR expression by E3 CHIP linked CD3-targeting construct (LG024).** Fig. 14A shows Gating Strategy for co-culture assay showing paracrine downregulation of TCR expression. Gating on CellTrace Violet-positive(i.e. Cell Trace ⁺cells, without LG024-transfection) and -negative cells (i.e. Cell Trace ⁻cells, LG024-transfected) allows for the characterization of these distinct populations (the left column of Fig. 14A). LG024-transfected cells (i.e. Cell Trace ⁻ cells) were further gated into a GFP-positive population expressing the E3 ligase construct and a GFP-negative population that was not transfected(the middle column of Fig. 14A). The cells were stained with PE-conjugated αCD3 (Clone Hit3a), to evaluate TCR surface expression(the left column of Fig. 14A). Fig. 14B shows Jurkat cells electroporated with LG024(i.e. Cell Trace - cells, LG024-transfected) were serially diluted with parental stock Jurkat cells labeled with CellTrace Violet(i.e. Cell Trace ⁺ cells, without LG024-transfection). Serial dilutions to the fraction of E3 Ligase-expressing cells show antigen-specific downregulation of surface molecules on non-expressing cells. LG024-transfected Jurkat cells were serially diluted with CellTrace Violet-labeled parental cells so that the total proportion of E3 ligase positive cells were: 78%, 57%, 44%, 31%, 22%, 16%, 11%, and 0%. The parental population (i.e. the un-transfected population) is shown as the shaded, grey histogram(i.e. filled area). The transfected population was further gated into GFP-positive (solid black line marked area) and GFP-negative (dashed black line marked area). Cells were stained using a PE-conjugated CD3 antibody (Clone Hit3a) to determine TCR surface expression.

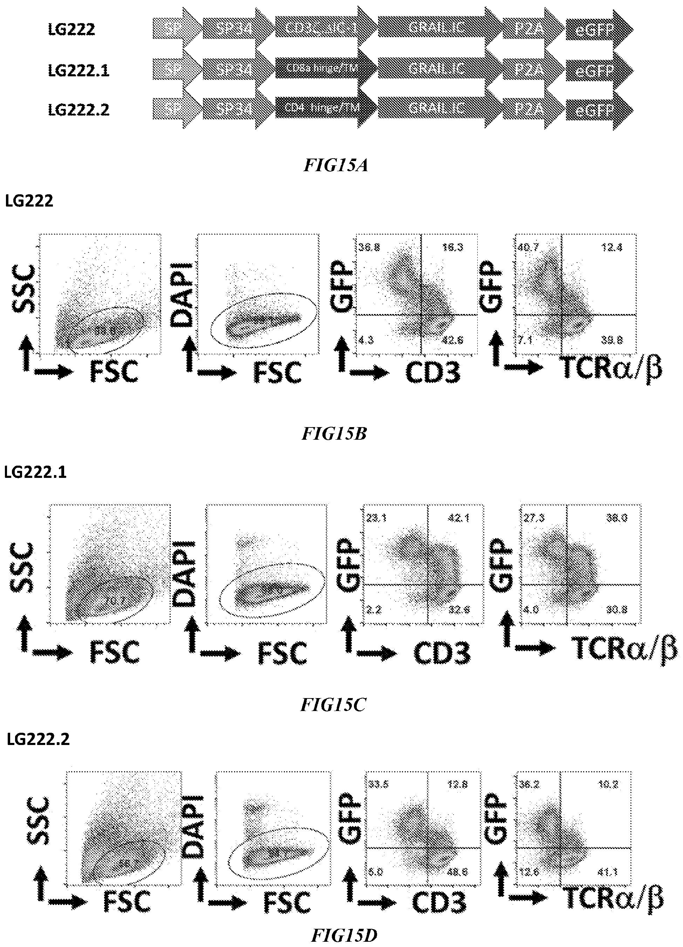

**Fig.15A****-** **Fig.15D** **Impacts of different hinge/transmembrane domains on TCR/CD3 downregulation in Jurkat mediated by both CD3-targeting scFv (i.e.SP34) and GRAIL.IC.** Fig.15A shows construct map for LG222, LG222.1 and LG222.2; Fig.15B-D shows Jurkat cells were electroporated with PiggyBac vectors expressing LG222, LG222.1 or LG222.2 in combination with PiggyBac Transposase mRNA. At day 3 post electroporation, TCR surface expression was evaluated by CD3 and TCRα/β staining.

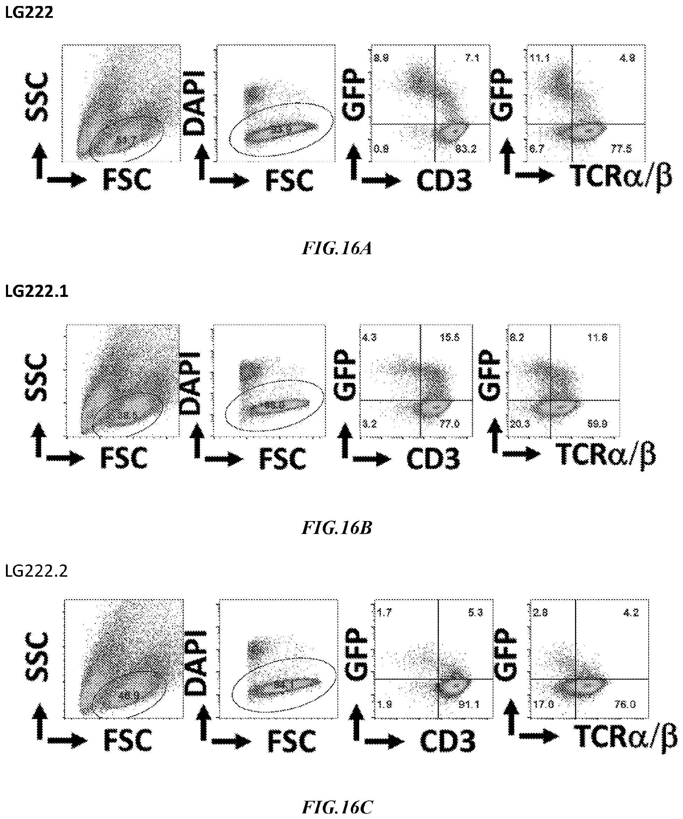

**Fig.16A****-** **Fig.16C** **The impacts of different hinge/transmembrane domains on TCR/CD3 downregulation in human primary T cells mediated by both CD3-targeting scFv (i.e.SP34) and GRAIL.IC.** Fig.16A-C shows human primary T cells were pre-activated and electroporated with PiggyBac vectors expressing LG222, LG222.1 or LG222.2 in combination with PiggyBac Transposase mRNA. At day 3 post electroporation, TCR surface expression was evaluated by CD3 and TCRα/β staining.

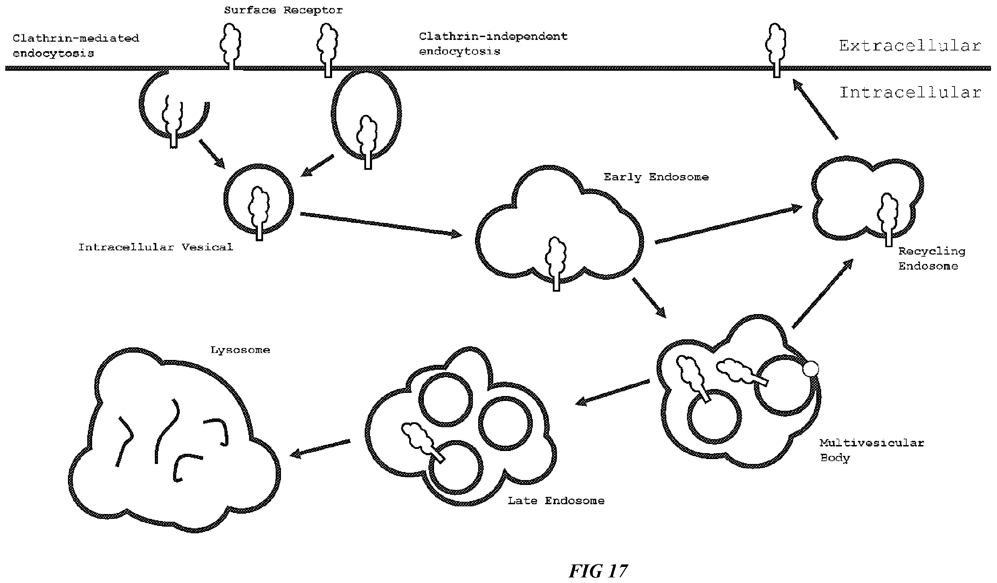

**Fig. 17** **Overview of receptor endocytosis.** Cell surface receptors are endocytosed via clathrin-mediated endocytosis or via clathrin-independent endocytosis, where they are internalized as intracellular vesicles. Vesicles containing the surface receptor ectodomain within their lumens are trafficked via adaptor proteins to the early endosomes for further sorting. From the early endosome, surface receptors can either be trafficked back to the cell surface through recycling endosomes, or aggregated within multivesicular bodies. Sorting back to the surface or to alternative intracellular pathways is dependent on motifs in the transmembrane and entodomains of these surface receptors. From the multivesicular bodies, receptors are processed further into late endosomes and eventually to lysosomes, where the low pH of the lumen, as well as lysosomal proteases, results in the degradation of the cell surface receptor and its associated cargo.

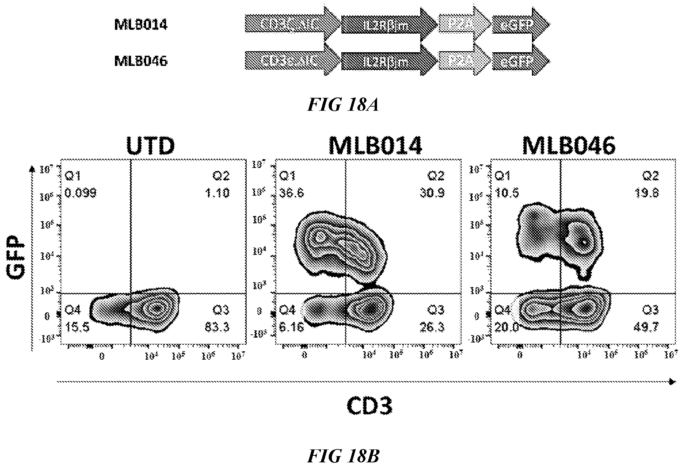

**Fig.18A** **Schematic diagram of the CD3ζ.ΔIC-IL2Rβjm(MLB014) and CD3ε.ΔIC-IL2Rβjm(MLB046) chimeric proteins (i.e. fusion proteins).** Our design is based on the on the extracellular and transmembrane sequences of CD3ζ (MLB014) and CD3ε (MLB046) directly fused to the first 27 amino acids of the IL2Rβ entodomain(i.e. IL2Rβ jm), which bears its lysosomal targeting motif. Inclusion of the CD3ζ and CD3ε transmembrane domains allows our constructs to be incorporated into the multi-subunit TCR complex.
**Fig.18B** **Expression of CD3ζ.ΔIC-IL2Rβjm and CD3ε.ΔIC-IL2Rβjm chimeric proteins (i.e. fusion proteins) causes TCR downregulation in Jurkat E6.1 NFAT-luciferase reporter cells.** Jurkat cells were electroporated with PiggyBac vectors expressing the CD3 fusion proteins and PiggyBac Transposase mRNA to induce expression of CD3ζ.ΔIC-IL2Rβjm (MLB014) or CD3ε.ΔIC-IL2Rβjm (MLB046). At day 5 post electroporation, TCR surface expression was evaluated by CD3 staining. As the TCR is an obligate heteromeric complex, downregulation of CD3 is indicative of overall TCR downregulation. These data suggest that incorporation of MLB014/(CD3ζ.ΔIC-IL2Rβjm) construct or MLB046/(CD3ε.ΔIC-IL2Rβjm) into the TCR complex promotes increased internalization of the entire complex.

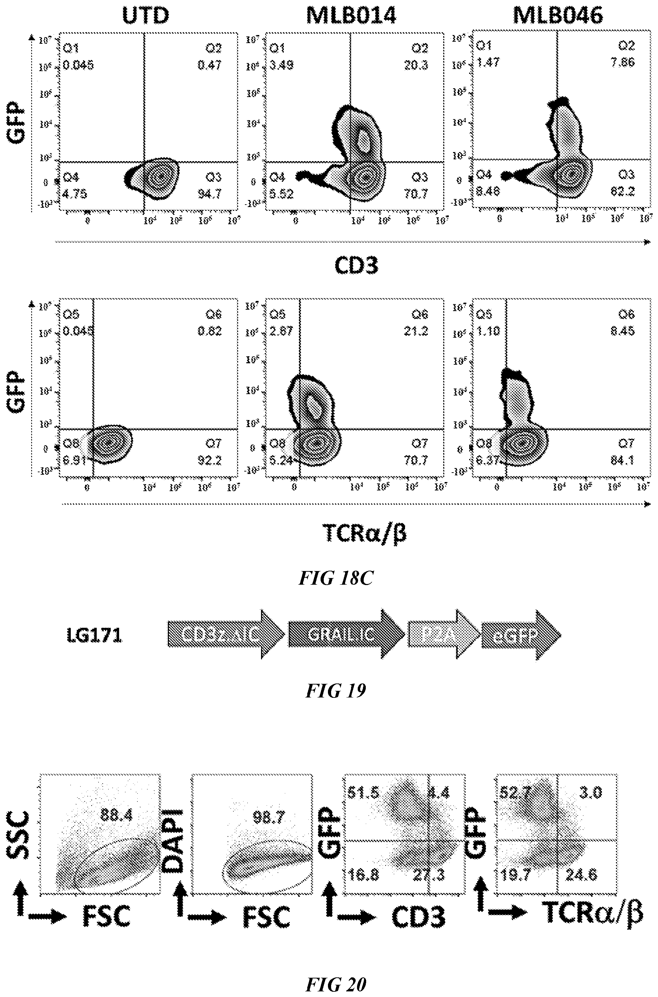

**Fig.18C** **Expression of CD3ζ.ΔIC-IL2Rβjm and CD3ε.ΔIC-IL2Rβjm chimeric proteins causes TCR downregulation in donor-derived T cells.** As with the Jurkat reporter cells, primary T cells were electroporated with PiggyBac vector and PiggyBac Transposase mRNA to induce expression of CD3ζ.ΔIC-IL2Rβjm (MLB014) or CD3ε.ΔIC-IL2Rβjm (MLB046). At day 5 post-electroporation, TCR expression was evaluated by staining for CD3 (upper panels) and TCRα/β (lower panels). These data suggest that the same underlying mechanisms are working in both primary T cells and Jurkat cells, but that the efficiency of TCR downregulation is somewhat reduced in primary T cells.
**Fig.19** **Schematic diagram of the CD3ζ.ΔIC-GRAIL.IC(LG171).** Our design is based on the extracellular and transmembrane sequences of CD3ζ (i.e.CD3ζ.ΔIC or CD3ζ truncation) and intracellular domain of membrane-anchored E3 ligase GRAIL(GRAIL.IC). Inclusion of the CD3ζ transmembrane domains may allow our constructs to be incorporated into the multi-subunit TCR complex.
**Fig.20** **Expression of LG171 chimeric proteins causes TCR/ CD3 downregulation in Jurkat cells.** Jurkat cells were electroporated with PiggyBac vectors expressing the CD3 fusion proteins(i.e. LG171) and PiggyBac Transposase mRNA to induce expression of CD3ζ.ΔIC-GRAIL.IC(LG171). At day 3 post electroporation, TCR surface expression was evaluated by CD3 and TCRα/β staining.

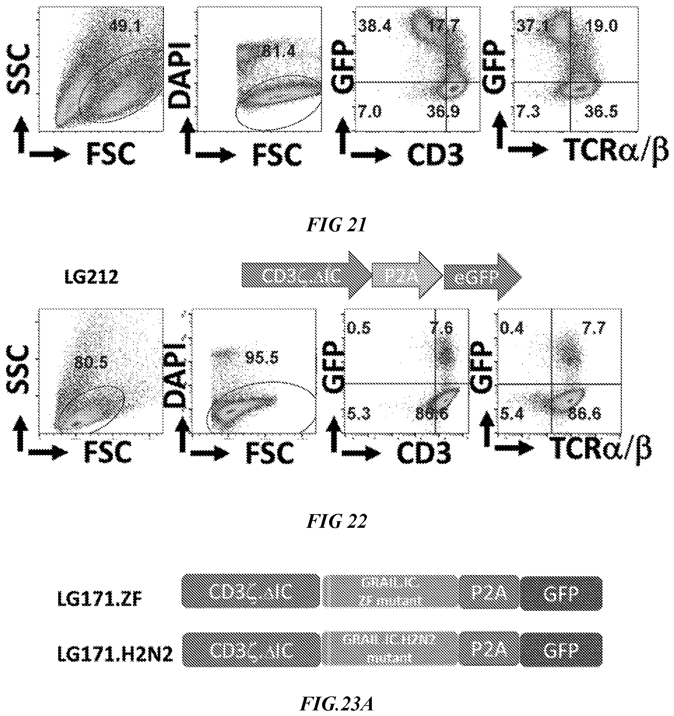

**Fig.21** **Expression of LG171 chimeric proteins causes TCR/ CD3 downregulation in human primary T cells.** As with the Jurkat reporter cells, primary T cells were electroporated with PiggyBac vector LG171 and PiggyBac Transposase to induce expression of CD3ζ.ΔIC-GRAIL.IC(LG171). At day 3 post-electroporation, TCR expression was evaluated by staining for CD3 and TCRα/β. Similarly, downregulation of TCR/CD3 expression were significant in GFP+ fraction.
**Fig.22** **LG212 (without E3 ligase) could not reduce TCR expression in human primary T cells.** Primary T cells were electroporated with PiggyBac vector LG212 and PiggyBac Transposase. At day 3 post-electroporation, TCR expression was evaluated by staining for CD3 and TCRα/β. No TCR/CD3 downregulation can be seen when only CD3ζ (i.e.CD3ζ. ΔIC) was adopted in human primary T cells.

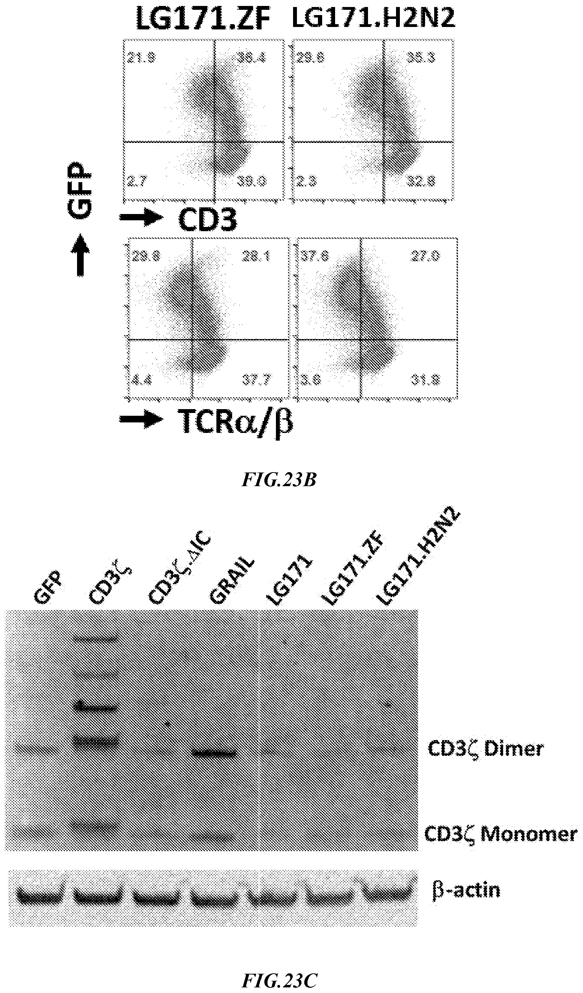

**Fig.23A-23C** **Mutation or domain switch in GRAIL intracellular domain confer similar downregulation of TCR in LG171 as LG212 (CD3ζ. ΔIC).** LG171.ZF(contains domain switch), LG171.H2N2(contains point mutation) constructs are shown in Fig.23A. Both FACS (Fig.23B) and western blot (Fig.23C) tests indicated that functional zinc-finger domain of GRAIL is necessary for a better TCR/CD3 downregulation in LG171 (Fig. 21).

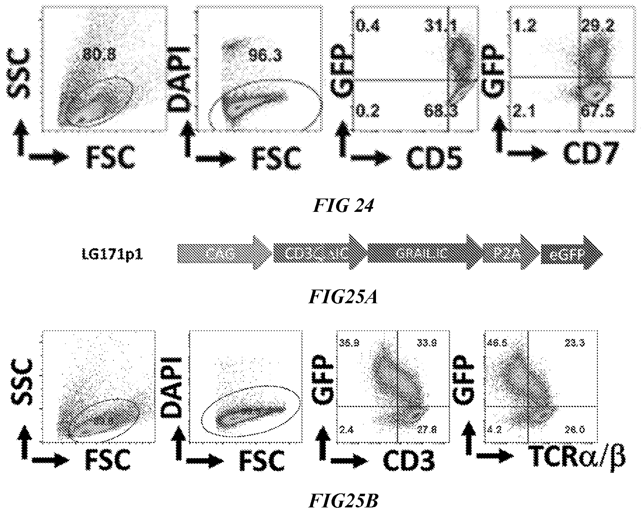

**Fig.24** **Expression of LG171 chimeric proteins couldnot confer non-specific downregulation of CD5 or CD7.** The downregulation effect in primary T cells by LG171 was TCR/CD3 specific, as neither CD5 nor CD7 was affected at all.

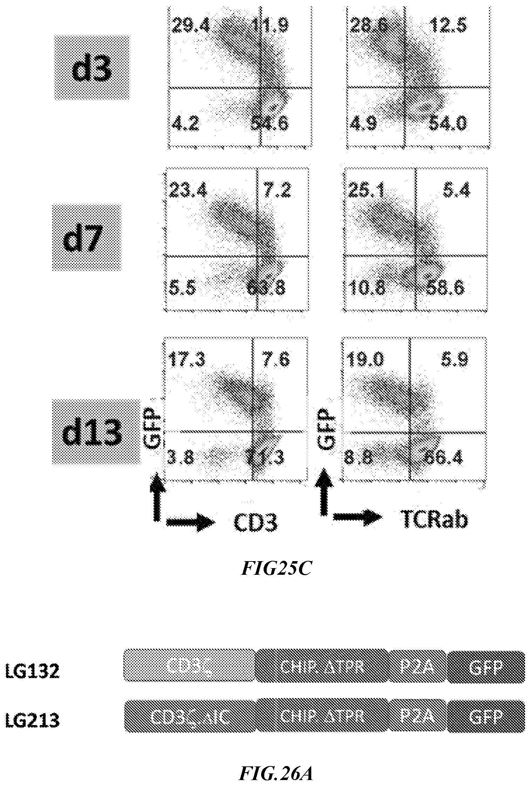

**Fig.25A-25C** **LG171p1 with CAG promoter confers downregulation of TCR/CD3 on T cells.** LG171p1 fusion protein construct is shown in Fig. 25A. Fig.25A shows construct LG171p1 was generated by replacing EF1a promoter in LG171 with CAG promoter; Fig.25B shows CAG promoter confers downregulation of TCR/CD3 on Jurkat cells. Jurkat cells were electroporated with PiggyBac vectors expressing LG171p1 fusion proteins and PiggyBac Transposase mRNA. At day 3 post electroporation, TCR surface expression was evaluated by CD3 and TCRα/β staining. Fig.25C shows CAG promoter confers sustainable downregulation of TCR/CD3 on human primary T cells. As with the Jurkat reporter cells, primary T cells were pre-activated and electroporated with PiggyBac vector LG171p1 and PiggyBac Transposase. At day 3, 7 and 13 post-electroporation, TCR surface expression was evaluated by CD3 and TCRα/β staining.

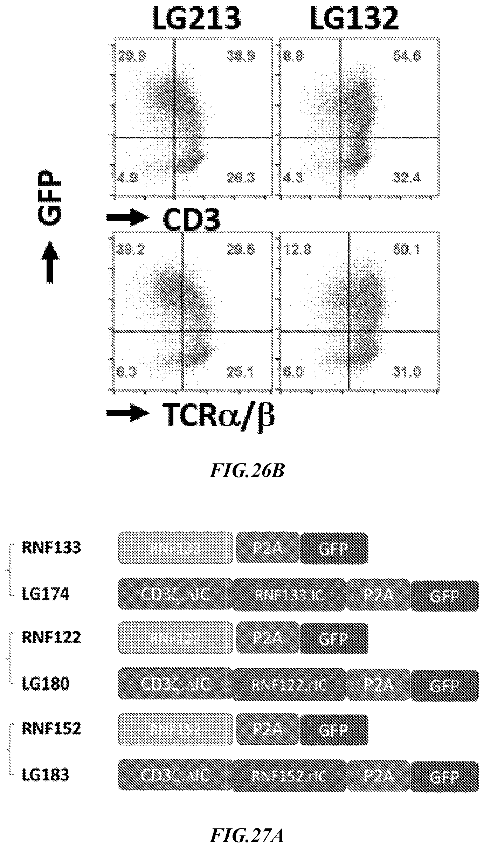

**Fig.26A-26B** **The downregulation in LG213 was mainly contributed by CD3ζ truncation.** CHIP.ΔTPR was linked to full length CD3ζ or CD3ζ.ΔIC to generate new chimeras( LG132, LG213) for TCR targeting (Fig.26A). FACS data for TCR levels in primary T cells transfected with LG132 or LG213. Compared to no effects in LG132 with full length of CD3ζ, the downregulation in LG213 were mainly contributed by CD3ζ truncation(Fig.26B).

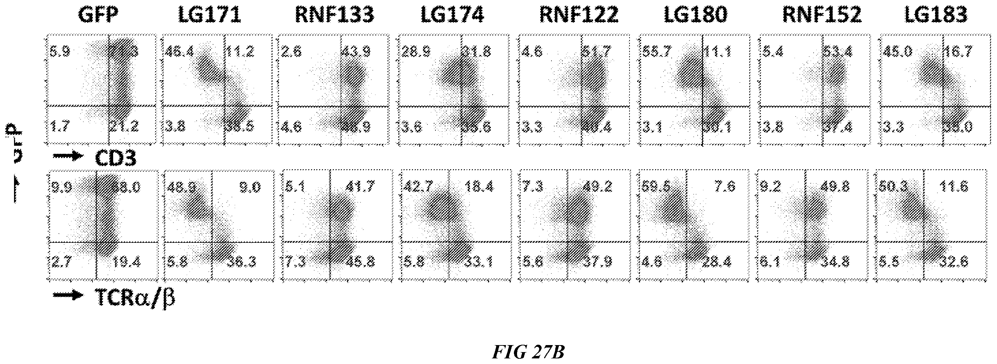

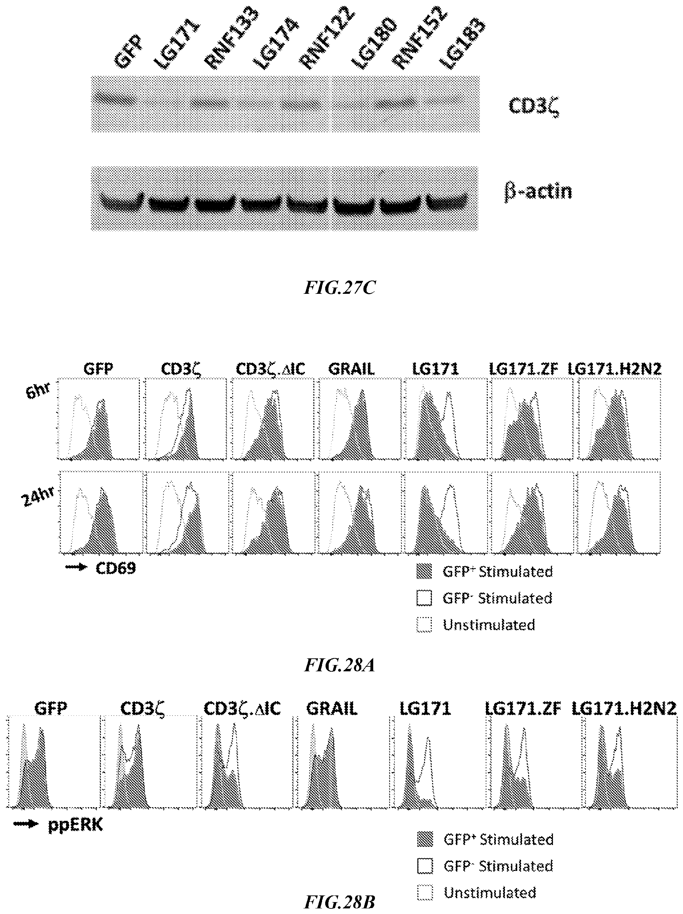

**Fig.27A-27C****. Additional E3 chimeras based on CD3ζ truncation and their efficacy in TCR downregulation.** Fig.27A shows construct design for additional E3 ligase and their chimeras constructs based on CD3 ζ truncation. Fig.27B FACS data for additional E3 ligase and their chimeras constructs in TCR downregulation. Fig.27C Western blot data showing a significant decrease of TCR in additional E3 chimera constructs based on CD3ζ, using β-action as the marker.
**Fig.28A****-** **Fig.28B** **Decreased activation in Jurkat cells transfected with LG171.** Fig.28A shows decreased activation in Jurkat cells transfected with LG171 (diminished CD69 upregulation), Fig.28B shows decreased activation in Jurkat cells transfected with LG171 (diminished ppERK upregulation). Jurkat cells transfected with the indicated constructs were briefly stimulated with αCD3/CD28. CD69 (Fig.28A) and ppERK (Fig.28B) were checked by FACS.

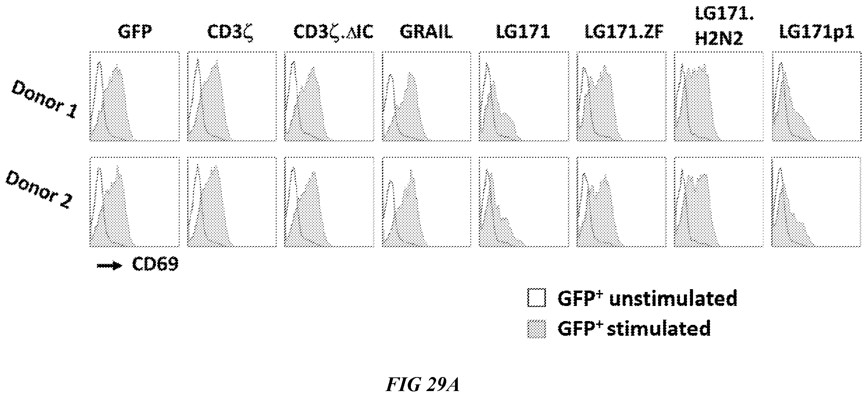

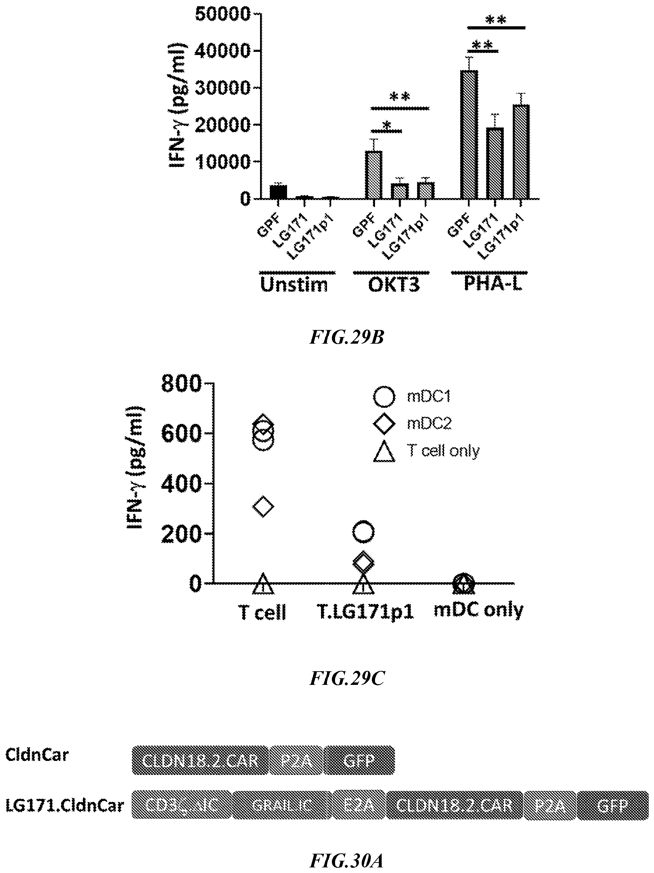

**Fig.29A-29C** **Decreased activation in primary T cells transfected with LG171 and LG171p1**. Human primary T cells transfected with the indicated constructs were stimulated with OKT3(anti CD3 antibody) for CD69 upregulation and IFN-γ cytokine secretion. CD69 upregulation was checked by FACS(Fig.29A). 24hrs later, supernatant was collected and tested for IFN-γ levels (Fig.29B). Mock T cells (untreated T cells) or LG171p1 transfected T cells were cocultured with allogeneic mDC cells for 5 days before testing IFN-γ levels using ELISA (Fig.29C). Fig.29A shows decreased activation in primary T cells transfected with LG171(CD69). Fig.29B shows decreased activation in primary T cells transfected withLG171 and LG171p1(IFN-γ). Fig.29C shows decreased activation in primary T cells transfected with LG171p1(IFN-γ).

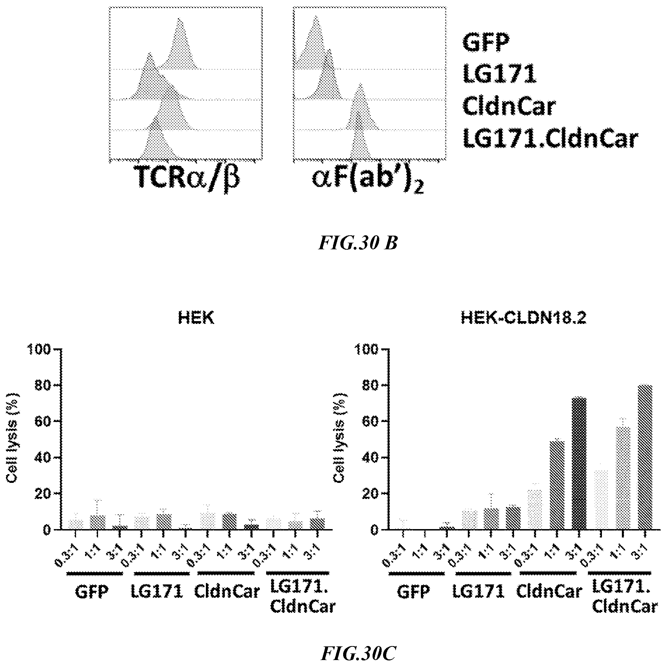

**Fig.30A****-****Fig.30C** **Expression and killing efficiency of LG171.CldnCar.** LG171.CldnCar and CldnCar fusion protein constructs are shown in Fig.30A. Fig.30B shows TCR/CAR expression in T cells transfected with the indicated constructs. Fig.30C shows cytotoxicity assays by coculturing engineered T cells with either parental HEK cells(i.e. HEK) or CLDN18.2 overexpressing HEK cells (i.e. HEK-CLDN 18.2) for 24 hrs.

### DETAILED DESCRIPTION OF THE INVENTION

Unless defined otherwise, technical and scientific terms used herein have the same meaning as generally used in the art to which this application belongs. For purposes of interpreting this specification, the following definitions will apply and whenever appropriate, terms used in the singular will also include the plural and vice versa. As used herein and in the appended claims, the singular forms "a", "an", and "the" also refer to the plural forms unless the context clearly dictates otherwise, e.g., reference to "a host cell" includes a plurality of such host cells.

As used herein, a "T cell receptor (TCR) complex", otherwise known as the TCR/CD3 complex, is a multimeric complex on the T-cell surface whose activation leads to the activation of the T-cell. The complex comprises (i) TCR, and (ii) CD3 T-cell co-receptor. As set forth below, the TCR comprises alpha (α) and beta (β) chains. The CD3 T-cell co-receptor comprises a CD3-gamma (CD3γ) chain, a CD3-delta (CD3δ) chain, two CD3-epsilon (CD3ε) chains and two zeta-(ζ) chains as accessory molecules.

TCRs allow for the antigen-specific activation of T-cells. Every T-cell expresses clonal TCRs which recognize a specific peptide/MHC complex during physical contact between T-cell and antigen-presenting cell-APC (via MHC class II) or any other cell type (via MHC class I). The TCR is a disulfide-linked membrane-anchored heterodimeric protein normally consisting of the highly variable alpha (α) and beta (β) chains. Each chain of the TCR comprises two extracellular domains: a variable (V) region and a constant (C) region, both of immunoglobulin superfamily (IgSF) domain forming antiparallel beta-sheets. The constant region is proximal to the cell membrane, followed by a transmembrane region and a short cytoplasmic tail, while the variable region binds to the peptide/MHC complex. The variable domain of the TCR alpha-chain and the TCR beta-chain each have three hypervariable or complementarity determining regions (CDRs) that contribute to the TCR's specificity for a particular peptide/MHC complex. The variable region of the beta-chain also has an additional area of hypervariability (HV4) that does not normally contact antigen.

A "single-chain variable fragment (scFv)" is a fusion protein of the variable regions of the heavy (VH) and light chains (VL) of an antibody, connected with a short linker peptide of ten to about 25 amino acids. The linker is usually rich in glycine for flexibility, as well as serine or threonine for solubility, and can either connect the N-terminus of the VH with the C-terminus of the VL, or vice versa. This protein retains the specificity of the original antibody, despite removal of the constant regions and the introduction of the linker. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain into a functional antigen binding site and thereby provide the antigen binding property of full length antibodies.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the molecules of the application are used to delay development of a disease or to slow the progression of a disease.

The term "cancer" or "tumor" as used herein refers to proliferative diseases, such as ovarian cancer, pancreatic cancer, colon cancer, colorectal cancer, lymphomas, lymphocytic leukemias, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

Before the various embodiments are described, it is to be understood that the teachings of this disclosure are not limited to the particular embodiments described, and as such can, of course, vary. It is also to be understood that the terminology used herein is for the purpose of describing particular embodiments only, and is not intended to be limiting, since the scope of the present teachings will be limited only by the appended claims.

The present application provides novel fusion proteins with particularly advantageous properties such as reducing the membrane-bound protein (MBP) level when expressed in a host cell.

In the present application, downregulation of Membrane-Bound Proteins (MBPs) is achieved by degradation tag protein-mediated degradation, wherein the degradation tag protein comprises ubiquitin ligase and/or domains of the cell surface receptors that mediate degradation (e.g. IL2Rβ sub domain). The tagging specificity or targeting specificity (or recognition specificity) of the MBPs to be degraded by the degradation tag protein (e.g. ubiquitin ligase or IL2Rβ) is mediated by an antibody (or its antigen-binding fragment) of MBPs to be degraded (that is, antibody mediated substrate recognition), or mediated by subunits or structural domains of the membrane-bound protein or complex to be degraded that have a transmembrane domain (that is, transmembrane domain mediated substrate recognition). The technical solution of the present application can be applied to any targets on a cell surface, independent of cell type. Preferably, the MBPs to be degraded can be membrane-bound receptors or receptor complex; more preferably the membrane-bound receptors can be T-cell receptors (TCRs). Thus, preferably, this application relates to the generation of allogeneic chimeric antigen receptor T cells (CAR-T) through TCR down regulation by targeted ubiquitin-mediated degradation by Receptor TAC (receptor targeting chimera), or to the generation of a fusion protein (e.g. a fusion protein comprising a component of the membrane-bound protein to be degraded or a fragment thereof that has a transmembrane domain fused to the degradation tag protein (e.g. E3 ubiquitin ligase and/or domains of cell surface receptors that mediate degradation)), the degradation tag protein may be incorporated into the multi-subunit TCR complex during the formation of the TCR complex so as to cause downregulation of the TCR complex. Specifically, an antibody (such as an anti-CD3 antibody) or antigen-binding fragment thereof was fused extracellularly with E3 ubiquitin ligase (such as the C-terminus of Hsc70-interacting protein (CHIP)) followed by the transmembrane domain, or an E3 ubiquitin ligase or a cell surface receptor that mediates degradation was fused intracellularly to a component of the membrane-bound protein or complex to be degraded or a fragment thereof that has a transmembrane domain. When expressed in host cells (such as primary T cells), MBP (such as TCR complex) is significantly down regulated. This is a novel MBP downregulation technology that is based on protein degradation. This technology can be applied to any MBP, especially surface receptors, for therapeutic use or for research purposes.

### (1) Fusion proteins of the present application

### 1. Fusion protein a):

The present application discloses a fusion protein a) comprising: the first linking protein, the degradation tag protein, the first transmembrane domain.

In some embodiments, the fusion protein comprises from N-terminal to C-terminal: the first linking protein, the degradation tag protein, the first transmembrane domain.

In some embodiments, the first linking protein comprises the antibody or antigen-binding fragment of the MBP to be degraded; more preferably, the antibody or antigen-binding fragment of the MBP to be degraded comprises antibody or antigen-binding fragment of CD3, CD5, CD7, PD-L1 or CD47; more preferably, antigen-binding fragment of CD3 comprises SP34 scFv, OKT3 scFv, UCHT1 scFv, UCHT1.Y177T scFv, L2K scFv, F6A scFv, BMA031 scFv, or BMA031.H6L12 scFv; antigen-binding fragment of CD5 comprises αCD5.14 scFv; antigen-binding fragment of CD7 comprises αCD7.TH69 scFv; antigen-binding fragment of PD-L1 comprises αPD-L1 scFv; antigen-binding fragment of CD47 comprises αCD47 scFv.

In some embodiments, the degradation tag protein comprises the E3 ubiquitin ligase; more preferably, the E3 ubiquitin ligase comprises CHIP.dTPR, FBW7.2-293, VHL.152-213, SPOP.167-374, SOCS2.143-198;

In some embodiments, the first transmembrane domain comprises CD8α transmembrane domain.

In some embodiments, the fusion protein a) further comprises a first signal peptide.

Optionally, there also may be a Linker (e.g. SEQ ID No. 91) between scFv and E3 ligase.

Optionally, there also may be a Linker (e.g. SEQ ID No. 91) between CD8α transmembrane domain (CD8 TM) and E3 ligase.

### 2. Fusion protein b):

The present application discloses a fusion protein b) comprising: the first linking protein, the first transmembrane domain, the degradation tag protein.

In some embodiments, the fusion protein comprises from N-terminal to C-terminal: the first linking protein, the first transmembrane domain, the degradation tag protein.

In some embodiments, the first linking protein comprises the antibody or antigen-binding fragment of the MBP to be degraded; more preferably, the antibody or antigen-binding fragment of the MBP to be degraded comprises antibody or antigen-binding fragment of CD3; more preferably, antigen-binding fragment of CD3 comprises SP34 scFv;

In some embodiments, the degradation tag protein comprises the E3 ubiquitin ligase; more preferably, E3 ubiquitin ligase comprises GRAIL.IC;

In some embodiments, the first transmembrane domain is selected from one or more of CD3ζ transmembrane domain variant, CD8α transmembrane domain, and CD4 transmembrane domain.

In some embodiments, the fusion protein b) further comprises a first signal peptide.

Optionally, there also may be an intracellular domain (e.g. SEQ ID No. 90) attached to the C-terminal of TM domain of Fusion protein b)(e.g.LG222, LG222.1, LG222.2).

### 3. Fusion protein c):

The present application discloses a fusion protein c) comprising: the second transmembrane-linking domain of the above 2), the cell surface receptors that mediate degradation.

In some embodiments, the fusion protein comprises from N-terminal to C-terminal: the second transmembrane-linking domain of the above 2), the cell surface receptors that mediate degradation.

In some embodiments, the second transmembrane-linking domain of the above 2) comprises CD3ζ.ΔIC or CD3ε.ΔIC;

In some embodiments, the cell surface receptors that mediate degradation comprises IL2Rβjm.

### 4. Fusion protein d):

The present application discloses a fusion protein d) comprising: the second transmembrane-linking domain of the above 2), the one or more of an E3 ubiquitin ligase or a variant thereof.

In some embodiments, the fusion protein comprises from N-terminal to C-terminal: the second transmembrane-linking domain of the above 2), the one or more of an E3 ubiquitin ligase or a variant thereof.

In some embodiments, the second transmembrane-linking domain of the above 2) comprises CD3ζ.ΔIC.

In some embodiments, the one or more of an E3 ubiquitin ligase or a variant thereof comprises GRAIL.IC, CHIP.dTPR, RNF133.IC, RNF122.rIC, RNF152.rIC.

### 5. The sequences used in Fusion proteins

In some embodiments, the SP34 scFv, OKT3 scFv, UCHT1 scFv, UCHT1.Y177T scFv, L2K scFv, F6A scFv, BMA031 scFv, BMA031.H6L12 scFv, αCD5.14 scFv, αCD7.TH69 scFv, αPD-L1 scFv or αCD47 scFv comprises an amino acid sequence at least about 95%, 96%, 97%, 98%, 99% or 100% identical to the amino acid sequence shown in any one of SEQ ID NOs. 2-13 respectively.

In some embodiments, the CHIP, CHIP.dTPR, FBW7.2-293, VHL.152-213, SPOP.167-374, SOCS2.143-198, GRAIL.IC, RNF133.IC, RNF122.rIC or RNF152.rIC comprises an amino acid sequence at least about 95%, 96%, 97%, 98%, 99% or 100% identical to the amino acid sequence shown in any one of SEQ ID NOs. 21-26,62,79,81 or 83 respectively.

In some embodiments, CD3ζ transmembrane domain variant, CD8α transmembrane domain, or CD4 transmembrane domain comprises an amino acid sequence at least about 95%, 96%, 97%, 98%, 99% or 100% identical to the amino acid sequence shown in SEQ ID NO. 89, 18 or 15 respectively.

In some embodiments, the fusion protein a) further comprises a first signal peptide; preferably, the first signal peptide is selected from an amino acid sequence at least about 95%, 96%, 97%, 98%, 99% or 100% identical to the amino acid sequence shown in SEQ ID NO. 19 or 20 respectively.

In some embodiments, the fusion protein b) further comprises a first signal peptide; preferably, the first signal peptide is selected from an amino acid sequence at least about 95%, 96%, 97%, 98%, 99% or 100% identical to the amino acid sequence shown in SEQ ID NO. 19.

In some embodiments, the CD3ζ.ΔIC and CD3ε.ΔIC comprises an amino acid sequence at least about 95%, 96%, 97%, 98%, 99% or 100% identical to the amino acid sequence shown in SEQ ID NO. 61, 64 respectively. More preferably, the transmembrane domain(TM) of CD3ζ or CD3ε comprises an amino acid sequence at least about 95%, 96%, 97%, 98%, 99% or 100% identical to the amino acid sequence shown in SEQ ID NO. 67, 70 respectively; more preferably, the extracellular domain(EM) of CD3ζ or CD3ε comprises an amino acid sequence at least about 95%, 96%, 97%, 98%, 99% or 100% identical to the amino acid sequence shown in SEQ ID NO. 66, 69 respectively; more preferably, the second signal peptide of CD3ζ or CD3ε comprises an amino acid sequence at least about 95%, 96%, 97%, 98%, 99% or 100% identical to the amino acid sequence shown in SEQ ID NO. 65, 68 respectively.

In some embodiments, the L2Rβ jm domain(IL2Rβjm) comprises an amino acid sequence at least about 95%, 96%, 97%, 98%, 99% or 100% identical to the amino acid sequence shown in SEQ ID NO. 58.

In a non-limiting example, the fusion protein of the present application further comprises one or more of a hinge or a P2A-GFP.

In some embodiments, the hinge is selected from a CD8α hinge, or CD4 hinge; more preferably, CD8α hinge or CD4 hinge comprises an amino acid sequence at least about 95%, 96%, 97%, 98%, 99% or 100% identical to the amino acid sequence shown in SEQ ID NO.:16 or 14, respectively; more preferably, the hinge is adjacent to the N-terminal of the first transmembrane domain.

In some embodiments, the P2A-GFP comprises an amino acid sequence at least about 95%, 96%, 97%, 98%, 99% or 100% identical to the amino acid sequence shown in SEQ ID NO.:57; more preferably, the P2A-GFP sequence is at C-terminal of the fusion protein.

### 6. The first transmembrane-linking domain comprises a first transmembrane domain and a first linking protein

### The first linking protein: An antibody of the membrane-bound protein or antigen-binding fragment thereof (e.g. scFv)

An antibody of the membrane-bound protein or antigen-binding fragment thereof (e.g. scFv) can be used as a linking protein that mediates binding (or specificity binding, specificity recognition) of the degradation tag protein to the membrane-bound protein (that is, antibody mediated substrate recognition).

### Single-chain variable fragment

The scFvs of the present application are those specifically binding to membrane-bound proteins (including but not limited to CD3, CD5, CD7, PD-1, PD-L1 or CD47), preferably binding to the extracellular part of those membrane-bound proteins. Preferably, the scFvs are those specifically binding to a component (or subunit) of the TCR/CD3 complex.

Thus, in some embodiments, the scFv specifically binds to CD3; for example, the CD3 is a mammalian origin CD3, preferably a human CD3.

### The first transmembrane domain:

The first transmembrane domain is selected from transmembrane domain of one or more subunits or structural domains of CD8α, CD4, CD3, CD28, 4-1BB and IL2R or a variant thereof.

### 7. The second transmembrane-linking domain comprises a transmembrane domain of the MBP to be degraded or a variant thereof

### A transmembrane domain of the MBP to be degraded or a variant thereof (Linking protein 2)

In some embodiments, one or more subunits or structural domains (or fragments) of the membrane-bound protein to be degraded or a variant thereof that has a transmembrane domain may be used as a linking protein to mediate incorporation (or specific recognition through incorporation) of the degradation tag protein to the membrane-bound protein, for example, incorporation of the degradation tag protein into the membrane-bound protein during the formation of the membrane-bound protein (that is, transmembrane domain mediated substrate recognition).

In some embodiments, the membrane-bound protein to be degraded is a membrane-bound receptor.

In some embodiments, the membrane-bound receptor is CD3, preferably a mammalian origin CD3, more preferably a human CD3.

In some embodiments, the Linking protein 2 comprises a transmembrane domain of one or more of CD3ζ, CD3γ, CD3δ, CD3ε, CD3α, CD3β or a variant thereof.

In some embodiments, the linking protein 2 comprises an extracellular domain and a transmembrane domain of one or more of CD3ζ, CD3γ, CD3δ, CD3ε, CD3α, CD3β or a variant thereof.

In some embodiments, the linking protein 2 comprises a second signal peptide, extracellular domain and a transmembrane domain of one or more of CD3ζ, CD3γ, CD3δ, CD3ε, CD3α, CD3β or a variant thereof.

### 8. Degradation tag protein 1: E3 Ubiquitin ligase

"E3 ubiquitin ligase" or "E3 ligase" or "Ubiquitin Ligase" (UL) is used herein to describe a target enzyme(s) binding site of ubiquitin ligase moieties in the bifunctional compounds according to the present application. E3 UL is a protein that in combination with an E2 ubiquitin-conjugating enzyme causes the attachment of ubiquitin to a lysine on a target protein; the E3 ubiquitin ligase targets specific protein substrates for degradation by the proteasome. Thus, E3 ubiquitin ligase alone or in complex with an E2 ubiquitin conjugating enzyme is responsible for the transfer of ubiquitin to targeted proteins. In general, the ubiquitin ligase is involved in polyubiquitination such that a second ubiquitin is attached to the first, a third is attached to the second, and so forth. Polyubiquitination marks proteins for degradation by the proteasome. However, there are some ubiquitination events that are limited to monoubiquitination, in which only a single ubiquitin is added by the ubiquitin ligase to a substrate molecule. Mono-ubiquitinated proteins are not targeted to the proteasome for degradation, but may instead be altered in their cellular location or function, for example, via binding other proteins that have domains capable of binding ubiquitin. Further complicating matters, different lysines on ubiquitin can be targeted by an E3 to make chains. The most common lysine is Lys48 on the ubiquitin chain. This is the lysine used to make polyubiquitin, which is recognized by the proteasome.

In some embodiments, the E3 ubiquitin ligase is selected from the following proteins or the truncated forms thereof: GRAIL, C-terminus of Hsc70-interacting protein (CHIP), CHIP.dTPR, F-box WD40-containing protein 7 (FBW7), FBW7.2-293, von Hippel-Lindau (VHL), VHL.152-213, Speckle-type BTB-POZ protein (SPOP), SPOP.167-374, Ubiquitin-protein ligase E3A (UBE3A), Mouse double minute 2 homolog (MDM2), Anaphase-promoting complex (APC), UBR5, LNX, Casitas B-lineage lymphoma-transforming sequence-like protein 1 (CBLL1), HECT domain and ankyrin repeat containing E3 ubiquitin protein ligase 1 (HACE1), HECT, C2 and WW domain containing E3 ubiquitin protein ligase 1 (HECW1), HECT, C2 and WW domain containing E3 ubiquitin protein ligase 2 (HECW2), HECT And RLD Domain Containing E3 Ubiquitin Protein Ligase 1 (HERC1), HERC2, HERC3, HERC4, HERC5, HERC6, HUWE1, ITCH, neural precursor cell expressed developmentally down-regulated protein 4 (NEDD4), Neural precursor cell expressed developmentally downregulated gene 4-like (NEDD4L), Peptidylprolyl isomerase (cyclophilin)-like 2 (PPIL2), PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UBOX5, UBR5, WWP1, WWP2, Parkin, MKRN1, GRAIL.IC, RNF133.IC, RNF122.rIC or RNF152.rIC.

In the present application, the E3 Ubiquitin ligase is used as a degradation tag protein, and the degradation of MBP mediated by E3 Ubiquitin ligase also can be achieved extracellularly and/or intracellularly.

### Degradation tag protein 2: one or more subunits or structural domains of cell surface receptors that mediate degradation

The one or more subunits or structural domains of cell surface receptors that mediate degradation (that is, a degradation tag protein) may be a protein that mediates degradation (or endocytosis and degradation, degradation after endocytosis) of the MBP or a component thereof, and for example, the degradation of the MBP or a component thereof mediated by one or more subunits or structural domains of the cell surface receptors may be accomplished intracellularly by an E3 ubiquitin ligase, a lysosome or other degradation pathways, and possibly after endocytosis of the MBP or a component thereof.

The one or more subunits or structural domains of the cell surface receptors that mediate degradation is specifically incorporated into MBP or a component thereof by fusing to a component of the membrane-bound protein (MBP) that is used to form the MBP during the formation of the MBP. Further, the component of the MBP that is used to form the MBP can be one or more domains of the MBP, optionally, one or more domains of CD3ε or CD3ζ.

The cell surface receptors that mediate degradation are usually different from the MBP to be degraded, although the cell surface receptors may also belong to a MBP.

In some embodiments, the cell surface receptors that mediate degradation and are used as the degradation tag protein of the present application comprise Interleukin receptors, or a variant derived therefrom.

In some embodiments, the subunits or structural domains of Interleukin receptors comprises a lysosomal targeting motif of IL-2R; preferably, the lysosomal targeting motif of IL-2R comprises L2Rβ jm domain.

### 9. Transmembrane Domain

"Transmembrane domain" includes an amino acid sequence of about 15 amino acid residues in length which spans the plasma membrane. More preferably, a transmembrane domain includes about at least 20, 25, 30, 35, 40, or 45 amino acid residues and spans the plasma membrane. Transmembrane domains are rich in hydrophobic residues, and typically have an alpha-helical structure. In a preferred embodiment, at least 50%, 60%, 70%, 80%, 90%, 95% or more of the amino acids of a transmembrane domain are hydrophobic, e.g., leucines, isoleucines, tyrosines, or tryptophans. Transmembrane domains are described in, for example, Zagotta, W. N. et al. (1996) Annu. Rev. Neurosci. 19:23 5-263.

The function of transmembrane domain in the fusion protein is to anchor said fusion protein to the cell surface, and thus any transmembrane domain fulfilling that function can be used in the present application.

A transmembrane domain suitable for the present application can be selected from the following: CD8α, CD28, 4-1BB or IL2R transmembrane domain, that is, the first transmembrane domain in above 6.

A transmembrane domain suitable for the present application also can be, a transmembrane domain that is a structural domain of the linking protein itself, that is, the transmembrane domain in above 7.

### (2) Nucleic Acid Encoding Fusion proteins, CARs, Vector Comprising Said Nucleic Acid, and Composition Comprising said Nucleic Acid or Vector

The present application provides a nucleic acid encoding the fusion proteins described herein. The nucleic acid encoding the fusion proteins can be easily prepared from an amino acid sequence of the specified fusion proteins by a conventional method. A nucleotide sequence encoding an amino acid sequence can be obtained from the aforementioned NCBI RefSeq IDs or accession numbers of GenBank for an amino acid sequence of each domain, and the nucleic acid of the present disclosure can be prepared using a standard molecular biological and/or chemical procedure. For example, based on the nucleotide sequence, a nucleic acid can be synthesized, and the nucleic acid of the present disclosure can be prepared by combining DNA fragments which are obtained from a cDNA library using a polymerase chain reaction (PCR).

The nucleic acid of the present disclosure can be linked to another nucleic acid so as to be expressed under control of a suitable promoter. Examples of the promoter include a promoter that constitutively promotes the expression of a gene or operatively linked construct, a promoter that induces the expression of a gene or operatively linked construct by the action of a drug or the like (e.g. tetracycline or doxorubicin). The nucleic acid of the present disclosure can be also linked to, in order to attain efficient transcription of the nucleic acid, other regulatory elements that cooperate with a promoter or a transcription initiation site, for example, a nucleic acid comprising an enhancer sequence or a terminator sequence. In addition to the nucleic acid of the present disclosure, a gene that can be a marker for confirming expression of the nucleic acid (e.g. a drug resistance gene, a gene encoding a reporter enzyme, or a gene encoding a fluorescent protein) may be incorporated.

In an embodiment, the nucleic acid is codon-optimized nucleic acid for expression in a particular host.

The present application further provides a vector comprising nucleic acid encoding the fusion proteins described herein. The term "vector", "expression vector", and "expression construct" or "construct" are used interchangeably, and are both defined to be a plasmid, virus, or other nucleic acid designed for protein expression in a cell. The vector or construct is used to introduce a gene into a host cell whereby the vector will interact with polymerases in the cell to express the protein encoded in the vector/construct. The expression vector and/or expression construct may exist in a cell extrachromosomally or integrated into the chromosome. When integrated into the chromosome the nucleic acids comprising the expression vector or expression construct will be an expression vector or expression construct.

The present application further provides a composition comprising at least one nucleic acid or at least one vector described herein.

The present application further provides a composition comprising a first nucleic acid and a second nucleic acid, comprising a first vector having a first nucleic acid and a second vector having a second nucleic acid, or comprising a vector having a first nucleic acid and a second nucleic acid, wherein
(1)the first nucleic acid encodes a fusion protein described herein,
(2)the second nucleic acid encodes a chimeric antigen receptor (CAR) comprising:
   (a)an extracellular ligand-binding domain comprising single chain variable fragment (scFv) specifically binding to a predetermined antigen;
   (b)a transmembrane domain, and
   (c)a cytoplasmic segment comprising one or more signaling domains, preferably comprising a 4-1BB signaling domain and a CD3ζ signaling domain.

In some embodiments, the predetermined antigen is a tumor-related antigen.

In some embodiments, the tumor-related antigen is selected from the following group: CEA, Claudin 18.2, GPC3, Receptor tyrosine kinase-like Orphan Receptor 1 (ROR1), CD38, CD19, CD20, CD22, BCMA, CAIX, CD446, CD133, EGFR, EGFRvIII, EpCam, GD2, EphA2, Her1, Her2, ICAM-1, IL13Ra2, Mesothelin, MUC1, MUC16, NKG2D, PSCA, NY-ESO-1, MART-1, WT1, MAGE-A10, MAGE-A3, MAGE-A4, EBV, NKG2D, PD1, PD-L1, CD25, IL-2, and CD3.

In some embodiments, the tumor-related antigen is CEA.

### (3) Host cells

The present application provides a host cell comprising the nucleic acid or vector or composition described herein. Thus, when expressed, the fusion proteins described herein will be expressed on the surface of the host cells for cell therapy.

In some embodiments, the host cell is an allogeneic cell.

In some embodiments, the host cell is a mammalian cell, preferably a primate cell, more preferably a human cell.

In some embodiments, the host cell is selected from a T cell, or a primary T cell gamma delta T cell, NK cell, NKT cell, macrophage, B cell, or non-immune cell.

### (4) Pharmaceutical composition

Pharmaceutical compositions of the present application comprise a fusion protein-expressing cell, preferably a fusion protein and a CAR-expressing cell, as described herein, in combination with one or more pharmaceutically or physiologically acceptable carriers, diluents or excipients. Such compositions may comprise buffers such as neutral-buffered saline, phosphate-buffered saline and the like; carbohydrates such as glucose, mannose, sucrose, dextrans, or mannitol; proteins, polypeptides or amino acids such as glycine; antioxidants; chelating agents such as EDTA or glutathione; adjuvants (e.g., aluminum hydroxide); and preservatives. Compositions of the present application are in one aspect formulated for intravenous administration.

Pharmaceutical compositions of the present application may be administered in a manner appropriate to the disease to be treated (or prevented). The quantity and frequency of administration will be determined by such factors as the condition of the patient, and the type and severity of the patient's disease, although appropriate dosages may be determined through clinical trials.

Suitable pharmaceutically acceptable excipients are well known to a person skilled in the art. Examples of pharmaceutically acceptable excipients include phosphate-buffered saline (e.g. 0.01 M phosphate, 0.138 M NaCl, 0.0027 M KCI, pH 7.4), an aqueous solution containing a mineral acid salt such as a hydrochloride, a hydrobromide, a phosphate, or a sulfate, saline, a solution of glycol or ethanol, and a salt of an organic acid such as an acetate, a propionate, a malonate or a benzoate. In some embodiments, an adjuvant such as a wetting agent or an emulsifier, and a pH buffering agent can also be used. In some embodiments, the pharmaceutically acceptable excipients described in Remington's Pharmaceutical Sciences (Mack Pub. Co., N.J. 1991) can be appropriately used. The composition of the present application can be formulated into a known form suitable for parenteral administration, for example, injection or infusion. In some embodiments, the composition of the present application may comprise formulation additives such as a suspending agent, a preservative, a stabilizer and/or a dispersant, and a preservation agent for extending a validity term during storage.

### (5) Treatment method

The references to methods of treatment in the subsequent paragraphs of this description are to be interpreted as references to the compounds, pharmaceutical compositions and medicaments of the present invention for use in a method for treatment of the human (or animal) body by therapy (or for in vivo diagnosis). The present application discloses a method of treating disease in a subject in need thereof, comprising administering to the subject an effective amount of the pharmaceutical composition described herein.

In some embodiments, the disease is cancer.

In some embodiments, the cancer is hematological malignancy or solid tumor.

In some embodiments, the cancer is ovarian cancer, pancreatic cancer, colon cancer, colorectal cancer, lymphoma, esophageal cancer, lung cancer, ovarian cancer, hepatic cancer, head-neck cancer, or cancer of the gallbladder.

### (6) Method of producing a cell having reduced target membrane-bound protein level

The present application provides a method of producing a cell having reduced target membrane-bound protein level, comprising introducing into a cell a nucleic acid, a vector, or a composition described herein.

### (7) Method of reducing or preventing GvHD

The present application discloses a method of reducing or preventing GvHD in a subject associated with the administration of one or more CAR T-cells to the subject, comprising:
(1) transfecting the one or more CAR T-cells with a nucleic acid or a vector described herein, and
(2) administering the CAR T-cells to the subject.

### (8) Method of downregulating membrane-bound protein (MBP) in a cell population

The present application provides a method of downregulating membrane-bound protein (MBP) in a cell population, comprising adding to said cell population a host cell of the application, wherein the host cell expresses fusion protein comprising scFv specifically binding to said MBP, and/or the host cell expresses a fusion protein comprising a component of the MBP or a fragment thereof that has a transmembrane domain

Preferably, the host cell population is a T cell population, preferably a human primary T cell population.

Preferably, the MBP is CD3, preferably human CD3.

### ILLUSTRATIVE SEQUENCES IN THIS DISCLOSURE

| **DESCRIPTION** | **SEQUENCE** | **SEQ ID NO:** |
|---|---|---|
| CD19 CAR scFv | DIQMTQTTSSLSASLGDRVTISCRASQDISKYLNWYQQK PDGTVKLLIYHTSRLHSGVPSRFSGSGSGTDYSLTISNLE QEDIATYFCQQGNTLPYTFGGGTKLEITGGGGSGGGGSG GGGSEVKLQESGPGLVAPSQSLSVTCTVSGVSLPDYGVS WIRQPPRKGLEWLGVIWGSETTYYNSALKSRLTIIKDNS KSQVFLKMNSLQTDDTAIYYCAKHYYYGGSYAMDYW GQGTSVTVSS | 1 |
| SP34 scFv | EVQLVESGGGLVQPGGSLKLSCAASGFTFNKYAMNWVR QAPGKGLEWVARIRSKYNNYATYYADSVKDRFTISRDDS KNTAYLQMNNLKTEDTAVYYCVRHGNFGNSYISYWAY WGQGTLVTVSSGGGGSGGGGSGGGGSGGGGSQTVVTQ EPSLTVSPGGTVTLTCGSSTGAVTSGYYPNWVQQKPGQ APRGLIGGTKFLAPGTPARFSGSLLGGKAALTLSGVQPE DEAEYYCALWYSNRWVFGGGTKLTVL | 2 |
| OKT3 scFv | QVQLVQSGGGVVQPGRSLRLSCKASGYTFTRYTMHWV RQAPGKGLEWIGYINPSRGYTNYNQKFKDRFTISRDNSK NTAFLQMDSLRPEDTGVYFCARYYDDHYSLDYWGQGT PVTVSSGGGGSGGGGSGGGGSDIQMTQSPSSLSASVGD RVTITCSASSSVSYMNWYQQTPGKAPKRWIYDTSKLAS GVPSRFSGSGSGTDYTFTISSLQPEDIATYYCQQWSSNPF TFGQGTKLQITR | 3 |
| UCHT1 scFv | MDIQMTQTTSSLSASLGDRVTISCRASQDIRNYLNWYQQ KPDGTVKLLIYYTSRLHSGVPSKFSGSGSGTDYSLTISNL EQEDIATYFCQQGNTLPWTFAGGTKLEIKGGGGSGGGG SGGGGSGGGGSEVQLQQSGPELVKPGASMKISCKASGY SFTGYTMNWVKQSHGKNLEWMGLINPYKGVSTYNQKF KDKATLTVDKSSSTAYMELLSLTSEDSAVYYCARSGYYG DSDWYFDVWGQGTTLTVFS | 4 |
| UCHT1.Y177T scFv | MDIQMTQSPSSLSASVGDRVTITCRASQDIRNYLNWYQ QKPGKAPKLLIYYTSRLESGVPSRFSGSGSGTDYTLTISS | 5 |
| | LQPEDFATYYCQQGNTLPWTFGQGTKVEIKGGGGSGGG GSGGGGSEVQLVESGGGLVQPGGSLRLSCAASGYSFTG YTMNWVRQAPGKGLEWVALINPTKGVSTYNQKFKDRF TISVDKSKNTAYLQMNSLRAEDTAVYYCARSGYYGDSD WYFDVWGQGTLVTVSS | |
| L2K scFv | DIQLTQSPAIMSASPGEKVTMTCRASSSVSYMNWYQQK SGTSPKRWIYDTSKVASGVPYRFSGSGSGTSYSLTISSME AEDAATYYCQQWSSNPLTFGAGTKLELKGGGGSGGGG SGGGGSDIKLQQSGAELARPGASVKMSCKTSGYTFTRY TMHWVKQRPGQGLEWIGYINPSRGYTNYNQKFKDKAT LTTDKSSSTAYMQLSSLTSEDSAVYYCARYYDDHYCLDY WGQGTTLTVSS | 6 |
| F6A scFv | QTVVTQEPSLTVSPGGTVTLTCGSSTGAVTSGYYPNWVQ QKPGQAPRGLIGGTKFLAPGTPARFSGSLLGGKAALTLS GVQPEDEAEYYCALWYSNRWVFGGGTKLTVLGGGGSG GGGSGGGGSEVQLLESGGGLVQPGGSLKLSCAASGFTF NIYAMNWVRQAPGKGLEWVARIRSKYNNYATYYADSV KSRFTISRDDSKNTAYLQMNNLKTEDTAVYYCVRHGNF GNSYVSFFAYWGQGTLVTVSS | 7 |
| BMA031 scFv(i.e. BMA031.wt scFv) | EVQLQQSGPELVKPGASVKMSCKASGYKFTSYVMHWV KQKPGQGLEWIGYINPYNDVTKYNEKFKGKATLTSDKS SSTAYMELSSLTSEDSAVHYCARGSYYDYDGFVYWGQG TLVTVSAGGGGSGGGGSGGGGSQIVLTQSPAIMSASPGE KVTMTCSATSSVSYMHWYQQKSGTSPKRWIYDTSKLAS GVPARFSGSGSGTSYSLTISSMEAEDAATYYCQQWSSNP LTFGAGTKLELK | 8 |
| BMA031.H6L12 scFv | QVQLVQSGAEVKKPGASVKVSCKASGYKFTSYVMHWV RQAPGQGLEWIGYINPYNDVTKYNEKFKGRVTITADKST STAYMELSSLRSEDTAVHYCARGSYYDYDGFVYWGQGT LVTVSSGGGGSGGGGSGGGGSEIVLTQSPATLSLSPGERA TLSCSATSSVSYMHWYQQKPGKAPKRWIYDTSKLASGV PSRFSGSGSGTEFTLTISSLQPEDFATYYCQQWSSNPLTFG QGTKLEIK | 9 |
| αCD5.14 scFv | IDAMGNIQLVQSGPELKKPGETVKISCKASGYTFTNYGM NWVKQAPGKGLRWMGWINTHTGEPTYADDFKGRFAFS LETSASTAYLQINNLKNEDTATYFCTRRGYDWYFDVWG | 10 |
| | AGTTVTVSSGGGGSGGGGSGGGGSDIKMTQSPSSMYAS LGERVTITCKASQDINSYLSWFHHKPGKSPKTLIYRANR LVDGVPSRFSGSGSGQDYSLTISSLDYEDMGIYYCQQYD ESPWTFGGGTKLEMKGSGDPA | |
| αCD7.TH69 scFv | EVQLVESGGGLVKPGGSLKLSCAASGLTFSSYAMSWVR QTPEKRLEWVASISSGGFTYYPDSVKGRFTISRDNARNIL YLQMSSLRSEDTAMYYCARDEVRGYLDVWGAGTTVTV SSGGGGSGGGGSGGGGSAAYKDIQMTQTTSSLSASLGD RVTISCSASQGISNYLNWYQQKPDGTVKLLIYYTSSLHS GVPSRFSGSGSGTDYSLTISNLEPEDIATYYCQQYSKLPY TFGGGTKLEIKR | 11 |
| αPD-L1 scFv | EVQLVQSGGGVVQPGRSLRLSCAASGFTFSNYAMHWV RQAPGKGLEWVAIITYAGSNEYYADSVKGRFTISRDNSK NTLYLQMNSLRPEDTAVYYCARDRIWVDYWGQGTLVT VSSGGGGSGGGGSGGGGSDIQMTQSPDSLAVSLGERATI NCKSSQSVLYSSNNKNYLAWYQQKPGQPPKLLIYWAST RESGVPDRFSGSGSGTDFTLTISSLQAEDVAVYYCQQYY STPLTFGGGTKVEIK | 12 |
| αCD47 ScFv | QVQLVQSGAEVKKPGASVKVSCKASGYTFTNYNMHW VRQAPGQRLEWMGTIYPGNDDTSYNQKFKDRVTITADT SASTAYMELSSLRSEDTAVYYCARGGYRAMDYWGQGT LVTVSSGGGGSGGGGSGGGGSDIVMTQSPLSLPVTPGEP ASISCRSSQSIVYSNGNTYLGWYLQKPGQSPQLLIYKVS NRFSGVPDRFSGSGSGTDFTLKISRVEAEDVGVYYCFQG SHVPYTFGQGTKLEIK | 13 |
| CD4 hinge | DSGQVLLESNIKVLPTWSTPVQP | 14 |
| CD4 Transmembrane domain | MALIVLGGVAGLLLFIGLGIFF | 15 |
| CD8α hinge | GTTTPAPRPPTPAPTIASQPLSLRPEACRPAAGGAVHTRG LDFACD | 16 |
| CD8α transmembrane domain(used in LS008*) | IYIWAPLAGTCGVLLLSLVITLYC | 17 |
| CD8α transmembrane domain | IYIWAPLAGTCGVLLLSLVIT | 18 |
| Signal Peptide (CD8α) | MALPVTALLLPLALLLHAARP | 19 |
| Signal Peptide(IGHV3-23) | MEFGLSWLFLVAILKGVQC | 20 |
| CHIP | | 21 |
| CHIP.dTPR(i.e.h CHIP.dTPR or hCHIP. ΔTPR) | RLNFGDDIPSALRIAKKKRWNSIEERRIHQESELHSYLSR LIAAERERELEECQRNHEGDEDDSHVRAQQACIEAKHD KYMADMDELFSQVDEKRKKRDIPDYLCGKISFELMREP CITPSGITYDRKDIEEHLQRVGHFDPVTRSPLTQEQLIPNL AMKEVIDAFISENGWVEDY | 22 |
| FBW7.2-293(i.e. FBW7) | CVPRSGLILSCICLYCGVLLPVLLPNLPFLTCLSMSTLESV TYLPEKGLYCQRLPSSRTHGGTESLKGKNTENMGFYGT LKMIFYKMKRKLDHGSEVRSFSLGKKPCKVSEYTSTTG LVPCSATPTTFGDLRAANGQGQQRRRITSVQPPTGLQEW LKMFQSWSGPEKLLALDELIDSCEPTQVKHMMQVIEPQ FQRDFISLLPKELALYVLSFLEPKDLLQAAQTCRYWRILA EDNLLWREKCKEEGIDEPLHIKRRKVIKPGFIHSPWKSAY IRQHRIDTNWRRGELKSP | 23 |
| VHL.152-213(i.e.VHL) | TLPVYTLKERCLQVVRSLVKPENYRRLDIVRSLYEDLED HPNVQKDLERLTQERIAHQRMGD | 24 |
| SPOP.167-374(i.e.SPOP) | SVNISGQNTMNMVKVPECRLADELGGLWENSRFTDCCL CVAGQEFQAHKAILAARSPVFSAMFEHEMEESKKNRVEI NDVEPEVFKEMMCFIYTGKAPNLDKMADDLLAAADKY ALERLKVMCEDALCSNLSVENAAEILILADLHSADQLKT QAVDFINYHASDVLETSGWKSMVVSHPHLVAEAYRSLA SAQCPFLGPPRKRLKQS | 25 |
| SOCS2.143-198(i.e. SOCS2) | PRNGTVHLYLTKPLYTSAPSLQHLCRLTINKCTGAIWGLP LPTRLKDYLEEYKFQV | 26 |
| LS008* (αCD19 CAR) | MALPVTALLLPLALLLHAARPDIQMTQTTSSLSASLGDR VTISCRASQDISKYLNWYQQKPDGTVKLLIYHTSRLHSG VPSRFSGSGSGTDYSLTISNLEQEDIATYFCQQGNTLPYT FGGGTKLEITGGGGSGGGGSGGGGSEVKLQESGPGLVAP SQSLSVTCTVSGVSLPDYGVSWIRQPPRKGLEWLGVIW GSETTYYNSALKSRLTIIKDNSKSQVFLKMNSLQTDDTAI YYCAKHYYYGGSYAMDYWGQGTSVTVSSTTTPAPRPP TPAPTIASQPLSLRPEACRPAAGGAVHTRGLDFACDIYIW APLAGTCGVLLLSLVITLYCKRGRKKLLYIFKQPFMRPV QTTQEEDGCSCRFPEEEEGGCELRVKFSRSADAPAYKQG QNQLYNELNLGRREEYDVLDKRRGRDPEMGGKPRRKN PQEGLYNELQKDKMAEAYSEIGMKGERRRGKGHDGLY QGLSTATKDTYDALHMQALPPR | 27 |
| LG024* | MALPVTALLLPLALLLHAARPQVQLVQSGGGVVQPGRS LRLSCKASGYTFTRYTMHWVRQAPGKGLEWIGYINPSR GYTNYNQKFKDRFTISRDNSKNTAFLQMDSLRPEDTGV YFCARYYDDHYSLDYWGQGTPVTVSSGGGGSGGGGSG GGGSDIQMTQSPSSLSASVGDRVTITCSASSSVSYMNWY QQTPGKAPKRWIYDTSKLASGVPSRFSGSGSGTDYTFTIS SLQPEDIATYYCQQWSSNPFTFGQGTKLQITRGTTTPAPR PPTPAPTIASQPLSLRPEACRPAAGGAVHTRGLDFACDIYI WAPLAGTCGVLLLSLVIT | 28 |
| LG085* | QVQLVQSGGGVVQPGRSLRLSCKASGYTFTRYTMHWV RQAPGKGLEWIGYINPSRGYTNYNQKFKDRFTISRDNSK NTAFLQMDSLRPEDTGVYFCARYYDDHYSLDYWGQGT PVTVSSGGGGSGGGGSGGGGSDIQMTQSPSSLSASVGD RVTITCSASSSVSYMNWYQQTPGKAPKRWIYDTSKLAS GVPSRFSGSGSGTDYTFTISSLQPEDIATYYCQQWSSNPF TFGQGTKLQITRGSGSGSGRLNFGDDIPSALRIAKKKRW NSIEERRIHQESELHSYLSRLIAAERERELEECQRNHEGD EDDSHVRAQQACIEAKHDKYMADMDELFSQVDEKRKK RDIPDYLCGKISFELMREPCITPSGITYDRKDIEEHLQRVG HFDPVTRSPLTQEQLIPNLAMKEVIDAFISENGWVEDY | 29 |
| LG089* | CVPRSGLILSCICLYCGVLLPVLLPNLPFLTCLSMSTLESV TYLPEKGLYCQRLPSSRTHGGTESLKGKNTENMGFYGT LKMIFYKMKRKLDHGSEVRSFSLGKKPCKVSEYTSTTG | 30 |
| | LVPCSATPTTFGDLRAANGQGQQRRRITSVQPPTGLQEW LKMFQSWSGPEKLLALDELIDSCEPTQVKHMMQVIEPQ FQRDFISLLPKELALYVLSFLEPKDLLQAAQTCRYWRILA EDNLLWREKCKEEGIDEPLHIKRRKVIKPGFIHSPWKSAY IRQHRIDTNWRRGELKSPGSGSGSGEVQLVESGGGLVQP GGSLKLSCAASGFTFNKYAMNWVRQAPGKGLEWVARI RSKYNNYATYYADSVKDRFTISRDDSKNTAYLQMNNLK TEDTAVYYCVRHGNFGNSYISYWAYWGQGTLVTVSSGG GGSGGGGSGGGGSGGGGSQTVVTQEPSLTVSPGGTVTL TCGSSTGAVTSGYYPNWVQQKPGQAPRGLIGGTKFLAP GTPARFSGSLLGGKAALTLSGVQPEDEAEYYCALWYSN RWVFGGGTKLTVL | |
| LG091* | EVQLVESGGGLVQPGGSLKLSCAASGFTFNKYAMNWVR QAPGKGLEWVARIRSKYNNYATYYADSVKDRFTISRDDS KNTAYLQMNNLKTEDTAVYYCVRHGNFGNSYISYWAY WGQGTLVTVSSGGGGSGGGGSGGGGSGGGGSQTVVTQ EPSLTVSPGGTVTLTCGSSTGAVTSGYYPNWVQQKPGQ APRGLIGGTKFLAPGTPARFSGSLLGGKAALTLSGVQPE DEAEYYCALWYSNRWVFGGGTKLTVLGSGSGSGTLPV YTLKERCLQVVRSLVKPENYRRLDIVRSLYEDLEDHPNV QKDLERLTQERIAHQRMGD | 31 |
| LG092* | EVQLVESGGGLVQPGGSLKLSCAASGFTFNKYAMNWVR QAPGKGLEWVARIRSKYNNYATYYADSVKDRFTISRDDS KNTAYLQMNNLKTEDTAVYYCVRHGNFGNSYISYWAY WGQGTLVTVSSGGGGSGGGGSGGGGSGGGGSQTVVTQ EPSLTVSPGGTVTLTCGSSTGAVTSGYYPNWVQQKPGQ APRGLIGGTKFLAPGTPARFSGSLLGGKAALTLSGVQPE DEAEYYCALWYSNRWVFGGGTKLTVLGSGSGSGSVNIS GQNTMNMVKVPECRLADELGGLWENSRFTDCCLCVAG QEFQAHKAILAARSPVFSAMFEHEMEESKKNRVEINDVE PEVFKEMMCFIYTGKAPNLDKMADDLLAAADKYALER LKVMCEDALCSNLSVENAAEILILADLHSADQLKTQAV DFINYHASDVLETSGWKSMVVSHPHLVAEAYRSLASAQ CPFLGPPRKRLKQS | 32 |
| LG112* | MALPVTALLLPLALLLHAARPQVQLVQSGGGVVQPGRS LRLSCKASGYTFTRYTMHWVRQAPGKGLEWIGYINPSR | 33 |
| | GYTNYNQKFKDRFTISRDNSKNTAFLQMDSLRPEDTGV YFCARYYDDHYSLDYWGQGTPVTVSSGGGGSGGGGSG GGGSDIQMTQSPSSLSASVGDRVTITCSASSSVSYMNWY QQTPGKAPKRWIYDTSKLASGVPSRFSGSGSGTDYTFTIS SLQPEDIATYYCQQWSSNPFTFGQGTKLQITRGSGSGSG RLNFGDDIPSALRIAKKKRWNSIEERRIHQESELHSYLSR LIAAERERELEECQRNHEGDEDDSHVRAQQACIEAKHD KYMADMDELFSQVDEKRKKRDIPDYLCGKISFELMREP CITPSGITYDRKDIEEHLQRVGHFDPVTRSPLTQEQLIPNL AMKEVIDAFISENGWVEDYGTTTPAPRPPTPAPTIASQPL SLRPEACRPAAGGAVHTRGLDFACDIYIWAPLAGTCGVL LLSLVIT | |
| LG112*ΔSP | QVQLVQSGGGVVQPGRSLRLSCKASGYTFTRYTMHWV RQAPGKGLEWIGYINPSRGYTNYNQKFKDRFTISRDNSK NTAFLQMDSLRPEDTGVYFCARYYDDHYSLDYWGQGT PVTVSSGGGGSGGGGSGGGGSDIQMTQSPSSLSASVGD RVTITCSASSSVSYMNWYQQTPGKAPKRWIYDTSKLAS GVPSRFSGSGSGTDYTFTISSLQPEDIATYYCQQWSSNPF TFGQGTKLQITRGSGSGSGRLNFGDDIPSALRIAKKKRW NSIEERRIHQESELHSYLSRLIAAERERELEECQRNHEGD EDDSHVRAQQACIEAKHDKYMADMDELFSQVDEKRKK RDIPDYLCGKISFELMREPCITPSGITYDRKDIEEHLQRVG HFDPVTRSPLTQEQLIPNLAMKEVIDAFISENGWVEDYG TTTPAPRPPTPAPTIASQPLSLRPEACRPAAGGAVHTRGL DFACDIYIWAPLAGTCGVLLLSLVIT | 34 |
| LG113* | MALPVTALLLPLALLLHAARPQVQLVQSGGGVVQPGRS LRLSCKASGYTFTRYTMHWVRQAPGKGLEWIGYINPSR GYTNYNQKFKDRFTISRDNSKNTAFLQMDSLRPEDTGV YFCARYYDDHYSLDYWGQGTPVTVSSGGGGSGGGGSG GGGSDIQMTQSPSSLSASVGDRVTITCSASSSVSYMNWY QQTPGKAPKRWIYDTSKLASGVPSRFSGSGSGTDYTFTIS SLQPEDIATYYCQQWSSNPFTFGQGTKLQITRGTTTPAPR PPTPAPTIASQPLSLRPEACRPAAGGAVHTRGLDFACDIYI WAPLAGTCGVLLLSLVITGSGSGSGRLNFGDDIPSALRIA KKKRWNSIEERRIHQESELHSYLSRLIAAERERELEECQR NHEGDEDDSHVRAQQACIEAKHDKYMADMDELFSQVD | 35 |
| | EKRKKRDIPDYLCGKISFELMREPCITPSGITYDRKDIEEH LQRVGHFDPVTRSPLTQEQLIPNLAMKEVIDAFISENGW VEDY | |
| LG113*ΔSP | QVQLVQSGGGVVQPGRSLRLSCKASGYTFTRYTMHWV RQAPGKGLEWIGYINPSRGYTNYNQKFKDRFTISRDNSK NTAFLQMDSLRPEDTGVYFCARYYDDHYSLDYWGQGT PVTVSSGGGGSGGGGSGGGGSDIQMTQSPSSLSASVGD RVTITCSASSSVSYMNWYQQTPGKAPKRWIYDTSKLAS GVPSRFSGSGSGTDYTFTISSLQPEDIATYYCQQWSSNPF TFGQGTKLQITRGTTTPAPRPPTPAPTIASQPLSLRPEACR PAAGGAVHTRGLDFACDIYIWAPLAGTCGVLLLSLVITGS GSGSGRLNFGDDIPSALRIAKKKRWNSIEERRIHQESELH SYLSRLIAAERERELEECQRNHEGDEDDSHVRAQQACIE AKHDKYMADMDELFSQVDEKRKKRDIPDYLCGKISFEL MREPCITPSGITYDRKDIEEHLQRVGHFDPVTRSPLTQEQ LIPNLAMKEVIDAFISENGWVEDY | 36 |
| LG123* | MALPVTALLLPLALLLHAARPMDIQMTQTTSSLSASLGD RVTISCRASQDIRNYLNWYQQKPDGTVKLLIYYTSRLHS GVPSKFSGSGSGTDYSLTISNLEQEDIATYFCQQGNTLPW TFAGGTKLEIKGGGGSGGGGSGGGGSGGGGSEVQLQQS GPELVKPGASMKISCKASGYSFTGYTMNWVKQSHGKN LEWMGLINPYKGVSTYNQKFKDKATLTVDKSSSTAYME LLSLTSEDSAVYYCARSGYYGDSDWYFDVWGQGTTLTV FSGSGSGSGRLNFGDDIPSALRIAKKKRWNSIEERRIHQE SELHSYLSRLIAAERERELEECQRNHEGDEDDSHVRAQQ ACIEAKHDKYMADMDELFSQVDEKRKKRDIPDYLCGKI SFELMREPCITPSGITYDRKDIEEHLQRVGHFDPVTRSPLT QEQLIPNLAMKEVIDAFISENGWVEDYGTTTPAPRPPTPA PTIASQPLSLRPEACRPAAGGAVHTRGLDFACDIYIWAPL AGTCGVLLLSLVIT | 37 |
| LG123*ΔSP | MDIQMTQTTSSLSASLGDRVTISCRASQDIRNYLNWYQQ KPDGTVKLLIYYTSRLHSGVPSKFSGSGSGTDYSLTISNL EQEDIATYFCQQGNTLPWTFAGGTKLEIKGGGGSGGGG SGGGGSGGGGSEVQLQQSGPELVKPGASMKISCKASGY SFTGYTMNWVKQSHGKNLEWMGLINPYKGVSTYNQKF KDKATLTVDKSSSTAYMELLSLTSEDSAVYYCARSGYYG | 38 |
| | DSDWYFDVWGQGTTLTVFSGSGSGSGRLNFGDDIPSAL RIAKKKRWNSIEERRIHQESELHSYLSRLIAAERERELEE CQRNHEGDEDDSHVRAQQACIEAKHDKYMADMDELFS QVDEKRKKRDIPDYLCGKISFELMREPCITPSGITYDRKD IEEHLQRVGHFDPVTRSPLTQEQLIPNLAMKEVIDAFISE NGWVEDYGTTTPAPRPPTPAPTIASQPLSLRPEACRPAAG GAVHTRGLDFACDIYIWAPLAGTCGVLLLSLVIT | |
| LG124* | MALPVTALLLPLALLLHAARPMDIQMTQSPSSLSASVGD RVTITCRASQDIRNYLNWYQQKPGKAPKLLIYYTSRLES GVPSRFSGSGSGTDYTLTISSLQPEDFATYYCQQGNTLPW TFGQGTKVEIKGGGGSGGGGSGGGGSEVQLVESGGGLV QPGGSLRLSCAASGYSFTGYTMNWVRQAPGKGLEWVA LINPTKGVSTYNQKFKDRFTISVDKSKNTAYLQMNSLRA EDTAVYYCARSGYYGDSDWYFDVWGQGTLVTVSSGSG SGSGRLNFGDDIPSALRIAKKKRWNSIEERRIHQESELHS YLSRLIAAERERELEECQRNHEGDEDDSHVRAQQACIEA KHDKYMADMDELFSQVDEKRKKRDIPDYLCGKISFELM REPCITPSGITYDRKDIEEHLQRVGHFDPVTRSPLTQEQLI PNLAMKEVIDAFISENGWVEDYGTTTPAPRPPTPAPTIAS QPLSLRPEACRPAAGGAVHTRGLDFACDIYIWAPLAGTC GVLLLSLVIT | 39 |
| LG124*ΔSP | MDIQMTQSPSSLSASVGDRVTITCRASQDIRNYLNWYQ QKPGKAPKLLIYYTSRLESGVPSRFSGSGSGTDYTLTISS LQPEDFATYYCQQGNTLPWTFGQGTKVEIKGGGGSGGG GSGGGGSEVQLVESGGGLVQPGGSLRLSCAASGYSFTG YTMNWVRQAPGKGLEWVALINPTKGVSTYNQKFKDRF TISVDKSKNTAYLQMNSLRAEDTAVYYCARSGYYGDSD WYFDVWGQGTLVTVSSGSGSGSGRLNFGDDIPSALRIA KKKRWNSIEERRIHQESELHSYLSRLIAAERERELEECQR NHEGDEDDSHVRAQQACIEAKHDKYMADMDELFSQVD EKRKKRDIPDYLCGKISFELMREPCITPSGITYDRKDIEEH LQRVGHFDPVTRSPLTQEQLIPNLAMKEVIDAFISENGW VEDYGTTTPAPRPPTPAPTIASQPLSLRPEACRPAAGGAV HTRGLDFACDIYIWAPLAGTCGVLLLSLVIT | 40 |
| LG125* | MALPVTALLLPLALLLHAARPDIQLTQSPAIMSASPGEKV TMTCRASSSVSYMNWYQQKSGTSPKRWIYDTSKVASG | 41 |
| | VPYRFSGSGSGTSYSLTISSMEAEDAATYYCQQWSSNPL TFGAGTKLELKGGGGSGGGGSGGGGSDIKLQQSGAELA RPGASVKMSCKTSGYTFTRYTMHWVKQRPGQGLEWIG YINPSRGYTNYNQKFKDKATLTTDKSSSTAYMQLSSLTS EDSAVYYCARYYDDHYCLDYWGQGTTLTVSSGSGSGS GRLNFGDDIPSALRIAKKKRWNSIEERRIHQESELHSYLS RLIAAERERELEECQRNHEGDEDDSHVRAQQACIEAKH DKYMADMDELFSQVDEKRKKRDIPDYLCGKISFELMRE PCITPSGITYDRKDIEEHLQRVGHFDPVTRSPLTQEQLIPN LAMKEVIDAFISENGWVEDYGTTTPAPRPPTPAPTIASQP LSLRPEACRPAAGGAVHTRGLDFACDIYIWAPLAGTCGV LLLSLVIT | |
| LG125*ΔSP | DIQLTQSPAIMSASPGEKVTMTCRASSSVSYMNWYQQK SGTSPKRWIYDTSKVASGVPYRFSGSGSGTSYSLTISSME AEDAATYYCQQWSSNPLTFGAGTKLELKGGGGSGGGG SGGGGSDIKLQQSGAELARPGASVKMSCKTSGYTFTRY TMHWVKQRPGQGLEWIGYINPSRGYTNYNQKFKDKAT LTTDKSSSTAYMQLSSLTSEDSAVYYCARYYDDHYCLDY WGQGTTLTVSSGSGSGSGRLNFGDDIPSALRIAKKKRW NSIEERRIHQESELHSYLSRLIAAERERELEECQRNHEGD EDDSHVRAQQACIEAKHDKYMADMDELFSQVDEKRKK RDIPDYLCGKISFELMREPCITPSGITYDRKDIEEHLQRVG HFDPVTRSPLTQEQLIPNLAMKEVIDAFISENGWVEDYG TTTPAPRPPTPAPTIASQPLSLRPEACRPAAGGAVHTRGL DFACDIYIWAPLAGTCGVLLLSLVIT | 42 |
| LG126* | MALPVTALLLPLALLLHAARPQTVVTQEPSLTVSPGGTV TLTCGSSTGAVTSGYYPNWVQQKPGQAPRGLIGGTKFL APGTPARFSGSLLGGKAALTLSGVQPEDEAEYYCALWY SNRWVFGGGTKLTVLGGGGSGGGGSGGGGSEVQLLES GGGLVQPGGSLKLSCAASGFTFNIYAMNWVRQAPGKGL EWVARIRSKYNNYATYYADSVKSRFTISRDDSKNTAYLQ MNNLKTEDTAVYYCVRHGNFGNSYVSFFAYWGQGTLV TVSSGSGSGSGRLNFGDDIPSALRIAKKKRWNSIEERRIH QESELHSYLSRLIAAERERELEECQRNHEGDEDDSHVRA QQACIEAKHDKYMADMDELFSQVDEKRKKRDIPDYLC GKISFELMREPCITPSGITYDRKDIEEHLQRVGHFDPVTR | 43 |
| | SPLTQEQLIPNLAMKEVIDAFISENGWVEDYGTTTPAPRP PTPAPTIASQPLSLRPEACRPAAGGAVHTRGLDFACDIYI WAPLAGTCGVLLLSLVIT | |
| LG126*ΔSP | QTVVTQEPSLTVSPGGTVTLTCGSSTGAVTSGYYPNWVQ QKPGQAPRGLIGGTKFLAPGTPARFSGSLLGGKAALTLS GVQPEDEAEYYCALWYSNRWVFGGGTKLTVLGGGGSG GGGSGGGGSEVQLLESGGGLVQPGGSLKLSCAASGFTF NIYAMNWVRQAPGKGLEWVARIRSKYNNYATYYADSV KSRFTISRDDSKNTAYLQMNNLKTEDTAVYYCVRHGNF GNSYVSFFAYWGQGTLVTVSSGSGSGSGRLNFGDDIPSA LRIAKKKRWNSIEERRIHQESELHSYLSRLIAAERERELE ECQRNHEGDEDDSHVRAQQACIEAKHDKYMADMDELF SQVDEKRKKRDIPDYLCGKISFELMREPCITPSGITYDRK DIEEHLQRVGHFDPVTRSPLTQEQLIPNLAMKEVIDAFIS ENGWVEDYGTTTPAPRPPTPAPTIASQPLSLRPEACRPAA GGAVHTRGLDFACDIYIWAPLAGTCGVLLLSLVIT | 44 |
| LG119* | MALPVTALLLPLALLLHAARPEVQLQQSGPELVKPGASV KMSCKASGYKFTSYVMHWVKQKPGQGLEWIGYINPYN DVTKYNEKFKGKATLTSDKSSSTAYMELSSLTSEDSAVH YCARGSYYDYDGFVYWGQGTLVTVSAGGGGSGGGGS GGGGSQIVLTQSPAIMSASPGEKVTMTCSATSSVSYMHW YQQKSGTSPKRWIYDTSKLASGVPARFSGSGSGTSYSLTI SSMEAEDAATYYCQQWSSNPLTFGAGTKLELKGSGSGS GRLNFGDDIPSALRIAKKKRWNSIEERRIHQESELHSYLS RLIAAERERELEECQRNHEGDEDDSHVRAQQACIEAKH DKYMADMDELFSQVDEKRKKRDIPDYLCGKISFELMRE PCITPSGITYDRKDIEEHLQRVGHFDPVTRSPLTQEQLIPN LAMKEVIDAFISENGWVEDYGTTTPAPRPPTPAPTIASQP LSLRPEACRPAAGGAVHTRGLDFACDIYIWAPLAGTCGV LLLSLVIT | 45 |
| LG119*ΔSP | EVQLQQSGPELVKPGASVKMSCKASGYKFTSYVMHWV KQKPGQGLEWIGYINPYNDVTKYNEKFKGKATLTSDKS SSTAYMELSSLTSEDSAVHYCARGSYYDYDGFVYWGQG TLVTVSAGGGGSGGGGSGGGGSQIVLTQSPAIMSASPGE KVTMTCSATSSVSYMHWYQQKSGTSPKRWIYDTSKLAS GVPARFSGSGSGTSYSLTISSMEAEDAATYYCQQWSSNP | 46 |
| | LTFGAGTKLELKGSGSGSGRLNFGDDIPSALRIAKKKRW NSIEERRIHQESELHSYLSRLIAAERERELEECQRNHEGD EDDSHVRAQQACIEAKHDKYMADMDELFSQVDEKRKK RDIPDYLCGKISFELMREPCITPSGITYDRKDIEEHLQRVG HFDPVTRSPLTQEQLIPNLAMKEVIDAFISENGWVEDYG TTTPAPRPPTPAPTIASQPLSLRPEACRPAAGGAVHTRGL DFACDIYIWAPLAGTCGVLLLSLVIT | |
| LG120* | MALPVTALLLPLALLLHAARPQVQLVQSGAEVKKPGAS VKVSCKASGYKFTSYVMHWVRQAPGQGLEWIGYINPY NDVTKYNEKFKGRVTITADKSTSTAYMELSSLRSEDTAV HYCARGSYYDYDGFVYWGQGTLVTVSSGGGGSGGGGS GGGGSEIVLTQSPATLSLSPGERATLSCSATSSVSYMHWY QQKPGKAPKRWIYDTSKLASGVPSRFSGSGSGTEFTLTIS SLQPEDFATYYCQQWSSNPLTFGQGTKLEIKGSGSGSGR LNFGDDIPSALRIAKKKRWNSIEERRIHQESELHSYLSRLI AAERERELEECQRNHEGDEDDSHVRAQQACIEAKHDK YMADMDELFSQVDEKRKKRDIPDYLCGKISFELMREPCI TPSGITYDRKDIEEHLQRVGHFDPVTRSPLTQEQLIPNLA MKEVIDAFISENGWVEDYGTTTPAPRPPTPAPTIASQPLS LRPEACRPAAGGAVHTRGLDFACDIYIWAPLAGTCGVLL LSLVIT | 47 |
| LG120*ΔSP | QVQLVQSGAEVKKPGASVKVSCKASGYKFTSYVMHWV RQAPGQGLEWIGYINPYNDVTKYNEKFKGRVTITADKST STAYMELSSLRSEDTAVHYCARGSYYDYDGFVYWGQGT LVTVSSGGGGSGGGGSGGGGSEIVLTQSPATLSLSPGERA TLSCSATSSVSYMHWYQQKPGKAPKRWIYDTSKLASGV PSRFSGSGSGTEFTLTISSLQPEDFATYYCQQWSSNPLTFG QGTKLEIKGSGSGSGRLNFGDDIPSALRIAKKKRWNSIEE RRIHQESELHSYLSRLIAAERERELEECQRNHEGDEDDS HVRAQQACIEAKHDKYMADMDELFSQVDEKRKKRDIP DYLCGKISFELMREPCITPSGITYDRKDIEEHLQRVGHFD PVTRSPLTQEQLIPNLAMKEVIDAFISENGWVEDYGTTTP APRPPTPAPTIASQPLSLRPEACRPAAGGAVHTRGLDFAC DIYIWAPLAGTCGVLLLSLVIT | 48 |
| LG137* | MEFGLSWLFLVAILKGVQCIDAMGNIQLVQSGPELKKPG ETVKISCKASGYTFTNYGMNWVKQAPGKGLRWMGWI | 49 |
| | NTHTGEPTYADDFKGRFAFSLETSASTAYLQINNLKNED TATYFCTRRGYDWYFDVWGAGTTVTVSSGGGGSGGGG SGGGGSDIKMTQSPSSMYASLGERVTITCKASQDINSYLS WFHHKPGKSPKTLIYRANRLVDGVPSRFSGSGSGQDYSL TISSLDYEDMGIYYCQQYDESPWTFGGGTKLEMKGSGD PAGSGSGSGRLNFGDDIPSALRIAKKKRWNSIEERRIHQE SELHSYLSRLIAAERERELEECQRNHEGDEDDSHVRAQQ ACIEAKHDKYMADMDELFSQVDEKRKKRDIPDYLCGKI SFELMREPCITPSGITYDRKDIEEHLQRVGHFDPVTRSPLT QEQLIPNLAMKEVIDAFISENGWVEDYGTTTPAPRPPTPA PTIASQPLSLRPEACRPAAGGAVHTRGLDFACDIYIWAPL AGTCGVLLLSLVIT | |
| LG137*ΔSP | IDAMGNIQLVQSGPELKKPGETVKISCKASGYTFTNYGM NWVKQAPGKGLRWMGWINTHTGEPTYADDFKGRFAFS LETSASTAYLQINNLKNEDTATYFCTRRGYDWYFDVWG AGTTVTVSSGGGGSGGGGSGGGGSDIKMTQSPSSMYAS LGERVTITCKASQDINSYLSWFHHKPGKSPKTLIYRANR LVDGVPSRFSGSGSGQDYSLTISSLDYEDMGIYYCQQYD ESPWTFGGGTKLEMKGSGDPAGSGSGSGRLNFGDDIPS ALRIAKKKRWNSIEERRIHQESELHSYLSRLIAAERERE L EECQRNHEGDEDDSHVRAQQACIEAKHDKYMADMDEL FSQVDEKRKKRDIPDYLCGKISFELMREPCITPSGITYDR KDIEEHLQRVGHFDPVTRSPLTQEQLIPNLAMKEVIDAFI SENGWVEDYGTTTPAPRPPTPAPTIASQPLSLRPEACRPA AGGAVHTRGLDFACDIYIWAPLAGTCGVLLLSLVIT | 50 |
| LG138* | MALPVTALLLPLALLLHAARPEVQLVESGGGLVKPGGSL KLSCAASGLTFSSYAMSWVRQTPEKRLEWVASISSGGFT YYPDSVKGRFTISRDNARNILYLQMSSLRSEDTAMYYCA RDEVRGYLDVWGAGTTVTVSSGGGGSGGGGSGGGGSA AYKDIQMTQTTSSLSASLGDRVTISCSASQGISNYLNWY QQKPDGTVKLLIYYTSSLHSGVPSRFSGSGSGTDYSLTIS NLEPEDIATYYCQQYSKLPYTFGGGTKLEIKRGSGSGSG RLNFGDDIPSALRIAKKKRWNSIEERRIHQESELHSYLSR LIAAERERELEECQRNHEGDEDDSHVRAQQACIEAKHD KYMADMDELFSQVDEKRKKRDIPDYLCGKISFELMREP CITPSGITYDRKDIEEHLQRVGHFDPVTRSPLTQEQLIPNL | 51 |
| | AMKEVIDAFISENGWVEDYGTTTPAPRPPTPAPTIASQPL SLRPEACRPAAGGAVHTRGLDFACDIYIWAPLAGTCGVL LLSLVIT | |
| LG138*ΔSP | EVQLVESGGGLVKPGGSLKLSCAASGLTFSSYAMSWVR QTPEKRLEWVASISSGGFTYYPDSVKGRFTISRDNARNIL YLQMSSLRSEDTAMYYCARDEVRGYLDVWGAGTTVTV SSGGGGSGGGGSGGGGSAAYKDIQMTQTTSSLSASLGD RVTISCSASQGISNYLNWYQQKPDGTVKLLIYYTSSLHS GVPSRFSGSGSGTDYSLTISNLEPEDIATYYCQQYSKLPY TFGGGTKLEIKRGSGSGSGRLNFGDDIPSALRIAKKKRW NSIEERRIHQESELHSYLSRLIAAERERELEECQRNHEGD EDDSHVRAQQACIEAKHDKYMADMDELFSQVDEKRKK RDIPDYLCGKISFELMREPCITPSGITYDRKDIEEHLQRVG HFDPVTRSPLTQEQLIPNLAMKEVIDAFISENGWVEDYG TTTPAPRPPTPAPTIASQPLSLRPEACRPAAGGAVHTRGL DFACDIYIWAPLAGTCGVLLLSLVIT | 52 |
| MLB052* | MALPVTALLLPLALLLHAARPEVQLVQSGGGVVQPGRS LRLSCAASGFTFSNYAMHWVRQAPGKGLEWVAIITYAG SNEYYADSVKGRFTISRDNSKNTLYLQMNSLRPEDTAVY YCARDRIWVDYWGQGTLVTVSSGGGGSGGGGSGGGGS DIQMTQSPDSLAVSLGERATINCKSSQSVLYSSNNKNYL AWYQQKPGQPPKLLIYWASTRESGVPDRFSGSGSGTDFT LTISSLQAEDVAVYYCQQYYSTPLTFGGGTKVEIKGSGSG SGRLNFGDDIPSALRIAKKKRWNSIEERRIHQESELHSYL SRLIAAERERELEECQRNHEGDEDDSHVRAQQACIEAK HDKYMADMDELFSQVDEKRKKRDIPDYLCGKISFELMR EPCITPSGITYDRKDIEEHLQRVGHFDPVTRSPLTQEQLIP NLAMKEVIDAFISENGWVEDYGTTTPAPRPPTPAPTIASQ PLSLRPEACRPAAGGAVHTRGLDFACDIYIWAPLAGTCG VLLLSLVIT | 53 |
| MLB052*ΔSP | EVQLVQSGGGVVQPGRSLRLSCAASGFTFSNYAMHWV RQAPGKGLEWVAIITYAGSNEYYADSVKGRFTISRDNSK NTLYLQMNSLRPEDTAVYYCARDRIWVDYWGQGTLVT VSSGGGGSGGGGSGGGGSDIQMTQSPDSLAVSLGERATI NCKSSQSVLYSSNNKNYLAWYQQKPGQPPKLLIYWAST RESGVPDRFSGSGSGTDFTLTISSLQAEDVAVYYCQQYY | 54 |
| | STPLTFGGGTKVEIKGSGSGSGRLNFGDDIPSALRIAKKK RWNSIEERRIHQESELHSYLSRLIAAERERELEECQRNHE GDEDDSHVRAQQACIEAKHDKYMADMDELFSQVDEKR KKRDIPDYLCGKISFELMREPCITPSGITYDRKDIEEHLQR VGHFDPVTRSPLTQEQLIPNLAMKEVIDAFISENGWVED YGTTTPAPRPPTPAPTIASQPLSLRPEACRPAAGGAVHTR GLDFACDIYIWAPLAGTCGVLLLSLVIT | |
| MLB053* | MALPVTALLLPLALLLHAARPQVQLVQSGAEVKKPGAS VKVSCKASGYTFTNYNMHWVRQAPGQRLEWMGTIYP GNDDTSYNQKFKDRVTITADTSASTAYMELSSLRSEDTA VYYCARGGYRAMDYWGQGTLVTVSSGGGGSGGGGSG GGGSDIVMTQSPLSLPVTPGEPASISCRSSQSIVYSNGNT YLGWYLQKPGQSPQLLIYKVSNRFSGVPDRFSGSGSGTD FTLKISRVEAEDVGVYYCFQGSHVPYTFGQGTKLEIKGS GSGSGRLNFGDDIPSALRIAKKKRWNSIEERRIHQESELH SYLSRLIAAERERELEECQRNHEGDEDDSHVRAQQACIE AKHDKYMADMDELFSQVDEKRKKRDIPDYLCGKISFEL MREPCITPSGITYDRKDIEEHLQRVGHFDPVTRSPLTQEQ LIPNLAMKEVIDAFISENGWVEDYGTTTPAPRPPTPAPTI ASQPLSLRPEACRPAAGGAVHTRGLDFACDIYIWAPLAG TCGVLLLSLVIT | 55 |
| MLB053*ΔSP | QVQLVQSGAEVKKPGASVKVSCKASGYTFTNYNMHW VRQAPGQRLEWMGTIYPGNDDTSYNQKFKDRVTITADT SASTAYMELSSLRSEDTAVYYCARGGYRAMDYWGQGT LVTVSSGGGGSGGGGSGGGGSDIVMTQSPLSLPVTPGEP ASISCRSSQSIVYSNGNTYLGWYLQKPGQSPQLLIYKVS NRFSGVPDRFSGSGSGTDFTLKISRVEAEDVGVYYCFQG SHVPYTFGQGTKLEIKGSGSGSGRLNFGDDIPSALRIAKK KRWNSIEERRIHQESELHSYLSRLIAAERERELEECQRNH EGDEDDSHVRAQQACIEAKHDKYMADMDELFSQVDEK RKKRDIPDYLCGKISFELMREPCITPSGITYDRKDIEEHLQ RVGHFDPVTRSPLTQEQLIPNLAMKEVIDAFISENGWVE DYGTTTPAPRPPTPAPTIASQPLSLRPEACRPAAGGAVHT RGLDFACDIYIWAPLAGTCGVLLLSLVIT | 56 |
| P2A-GFP**(i.e. P2A-eGFP) | ATNFSLLKQAGDVEENPGPMVSKGEELFTGVVPILVELDG DVNGHKFSVSGEGEGDATYGKLTLKFICTTGKLPVPWPT | 57 |
| | LVTTLTYGVQCFSRYPDHMKQHDFFKSAMPEGYVQERT IFFKDDGNYKTRAEVKFEGDTLVNRIELKGIDFKEDGNIL GHKLEYNYNSHNVYIMADKQKNGIKVNFKIRHNIEDGS VQLADHYQQNTPIGDGPVLLPDNHYLSTQSALSKDPNE KRDHMVLLEFVTAAGITLGMDELYK | |
| IL2Rβjm(i.e. IL2Rβ jm DT or L2Rβ jm domain) | NCRNTGPWLKKVLKCNTPDPSKFFSQL | 58 |
| MLB014* (CD3ζ.ΔIC-IL2Rβjm) | MKWKALFTAAILQAQLPITEAQSFGLLDPKLCYLLDGIL FIYGVILTALFLNCRNTGPWLKKVLKCNTPDPSKFFSQL | 59 |
| MLB046 * (CD3ε.ΔIC -IL2Rβjm) | MQSGTHWRVLGLCLLSVGVWGQDGNEEMGGITQTPYK VSISGTTVILTCPQYPGSEILWQHNDKNIGGDEDDKNIGS DEDHLSLKEFSELEQSGYYVCYPRGSKPEDANFYLYLRA RVCENCMEMDVMSVATIVIVDICITGGLLLLVYYWSNCR NTGPWLKKVLKCNTPDPSKFFSQL | 60 |
| CD3ζ.ΔIC | MKWKALFTAAILQAQLPITEAQSFGLLDPKLCYLLDGIL FIYGVILTALFL | 61 |
| GRAIL.IC | YSARRLRNARAQSRKQRQLKADAKKAIGRLQLRTLKQ GDKEIGPDGDSCAVCIELYKPNDLVRILTCNHIFHKTCVD PWLLEHRTCPMCKCDILKALGIEVDVEDGSVSLQVPVSN EISNSASSHEEDNRSETASSGYASVQGTDEPPLEEHVQST NESLQLVNHEANSVAVDVIPHVDNPTFEEDETPNQETAV REIKS | 62 |
| LG171 * (CD3ζ.ΔIC-GRAIL.IC) | MKWKALFTAAILQAQLPITEAQSFGLLDPKLCYLLDGIL FIYGVILTALFLYSARRLRNARAQSRKQRQLKADAKKAI GRLQLRTLKQGDKEIGPDGDSCAVCIELYKPNDLVRILTC NHIFHKTCVDPWLLEHRTCPMCKCDILKALGIEVDVED GSVSLQVPVSNEISNSASSHEEDNRSETASSGYASVQGTD EPPLEEHVQSTNESLQLVNHEANSVAVDVIPHVDNPTFEE DETPNQETAVREIKS | 63 |
| CD3ε.ΔIC | MQSGTHWRVLGLCLLSVGVWGQDGNEEMGGITQTPYK VSISGTTVILTCPQYPGSEILWQHNDKNIGGDEDDKNIGS DEDHLSLKEFSELEQSGYYVCYPRGSKPEDANFYLYLRA RVCENCMEMDVMSVATIVIVDICITGGLLLLVYYWS | 64 |
| MLB014.SP (i.e. the signal peptide of CD3ζ) | MKWKALFTAAILQAQLPITEA | 65 |
| MLB014.EM (i.e. the extracellular domain of CD3ζ) | QSFGLLDPK | 66 |
| MLB014.TM (i.e. the transmembrane domain of CD3ζ) | LCYLLDGILFIYGVILTALFL | 67 |
| MLB046.SP (i.e. the signal peptide of CD3ε) | MQSGTHWRVLGLCLLSVGVWGQ | 68 |
| MLB046.EM (i.e. the extracellular domain of CD3ε) | DGNEEMGGITQTPYKVSISGTTVILTCPQYPGSEILWQHN DKNIGGDEDDKNIGSDEDHLSLKEFSELEQSGYYVCYPR GSKPEDANFYLYLRARVCENCMEMD | 69 |
| MLB046.TM (i.e. the transmembrane domain of CD3ε) | VMSVATIVIVDICITGGLLLLVYYWS | 70 |
| LG212 * (CD3ε.ΔIC) | MQSGTHWRVLGLCLLSVGVWGQDGNEEMGGITQTPYK VSISGTTVILTCPQYPGSEILWQHNDKNIGGDEDDKNIGS DEDHLSLKEFSELEQSGYYVCYPRGSKPEDANFYLYLRA RVCENCMEMDVMSVATIVIVDICITGGLLLLVYYWS | 71 |
| CAG promoter | ctcgacattgattattgactagttattaatagtaatcaattacggggtcattagttcatagccc atatatggagttccgcgttacataacttacggtaaatggcccgcctggctgaccgcccaa cgacccccgcccattgacgtcaataatgacgtatgttcccatagtaacgccaatagggac tttccattgacgtcaatgggtggagtatttacggtaaactgcccacttggcagtacatcaag tgtatcatatgccaagtacgccccctattgacgtcaatgacggtaaatggcccgcctggc attatgcccagtacatgaccttatgggactttcctacttggcagtacatctacgtattagtcat cgctattaccatggtcgaggtgagccccacgttctgcttcactctccccatctcccccccct ccccacccccaattttgtatttatttattttttaattattttgtgcagcgatgggggcgggggg ggggggggggcgcgcgccaggcggggcggggcggggcgaggggcggggcggg gcgaggcggagaggtgcggcggcagccaatcagagcggcgcgctccgaaagtttcct tttatggcgaggcggcggcggcggcggccctataaaaagcgaagcgcgcggcgggc gggagtcgctgcgcgctgccttcgccccgtgccccgctccgccgccgcctcgcgccgc ccgccccggctctgactgaccgcgttactcccacaggtgagcgggcgggacggccctt ctcctccgggctgtaattagcgcttggtttaatgacggcttgtttcttttctgtggctgcgtga aagccttgaggggctccgggagggccctttgtgcggggggagcggctcggggggtgc | 72 |
| | gtgcgtgtgtgtgtgcgtggggagcgccgcgtgcggctccgcgctgcccggcggctgt gagcgctgcgggcgcggcgcggggctttgtgcgctccgcagtgtgcgcgaggggag cgcggccgggggcggtgccccgcggtgcggggggggctgcgaggggaacaaagg ctgcgtgcggggtgtgtgcgtgggggggtgagcagggggtgtgggcgcgtcggtcgg gctgcaaccccccctgcacccccctccccgagttgctgagcacggcccggcttcgggt gcggggctccgtacggggcgtggcgcggggctcgccgtgccgggcggggggtggc ggcaggtgggggtgccgggcggggcggggccgcctcgggccggggagggctcgg gggaggggcgcggcggcccccggagcgccggcggctgtcgaggcgcggcgagcc gcagccattgccttttatggtaatcgtgcgagagggcgcagggacttcctttgtcccaaat ctgtgcggagccgaaatctgggaggcgccgccgcaccccctctagcgggcgcgggg cgaagcggtgcggcgccggcaggaaggaaatgggcggggagggccttcgtgcgtcg ccgcgccgccgtccccttctccctctccagcctcggggctgtccgcggggggacggct gccttcgggggggacggggcagggcggggttcggcttctggcgtgtgaccggcggct ctagagcctctgctaaccatgttcatgccttcttctttttcctacagctcctgggcaacgtgct ggttattgtgctgtctcatcattttggcaaagaattg | |
| LG222* | MALPVTALLLPLALLLHAARPEVQLVESGGGLVQPGGSL KLSCAASGFTFNKYAMNWVRQAPGKGLEWVARIRSKY NNYATYYADSVKDRFTISRDDSKNTAYLQMNNLKTEDT AVYYCVRHGNFGNSYISYWAYWGQGTLVTVSSGGGGS GGGGSGGGGSGGGGSQTVVTQEPSLTVSPGGTVTLTCG SSTGAVTSGYYPNWVQQKPGQAPRGLIGGTKFLAPGTP ARFSGSLLGGKAALTLSGVQPEDEAEYYCALWYSNRWV FGGGTKLTVLQSFGLLDPKLCYLLDGILFIYGVILTALFL RVKFSRSADYSARRLRNARAQSRKQRQLKADAKKAIGR LQLRTLKQGDKEIGPDGDSCAVCIELYKPNDLVRILTCNH IFHKTCVDPWLLEHRTCPMCKCDILKALGIEVDVEDGSV SLQVPVSNEISNSASSHEEDNRSETASSGYASVQGTDEPP LEEHVQSTNESLQLVNHEANSVAVDVIPHVDNPTFEEDE TPNQETAVREIKS | 73 |
| LG222.1* | MALPVTALLLPLALLLHAARPEVQLVESGGGLVQPGGSL KLSCAASGFTFNKYAMNWVRQAPGKGLEWVARIRSKY NNYATYYADSVKDRFTISRDDSKNTAYLQMNNLKTEDT AVYYCVRHGNFGNSYISYWAYWGQGTLVTVSSGGGGS GGGGSGGGGSGGGGSQTVVTQEPSLTVSPGGTVTLTCG SSTGAVTSGYYPNWVQQKPGQAPRGLIGGTKFLAPGTP ARFSGSLLGGKAALTLSGVQPEDEAEYYCALWYSNRWV | 74 |
| | FGGGTKLTVLGTTTPAPRPPTPAPTIASQPLSLRPEACRPA AGGAVHTRGLDFACDIYIWAPLAGTCGVLLLSLVITRVK FSRSADYSARRLRNARAQSRKQRQLKADAKKAIGRLQL RTLKQGDKEIGPDGDSCAVCIELYKPNDLVRILTCNHIFH KTCVDPWLLEHRTCPMCKCDILKALGIEVDVEDGSVSL QVPVSNEISNSASSHEEDNRSETASSGYASVQGTDEPPLE EHVQSTNESLQLVNHEANSVAVDVIPHVDNPTFEEDETP NQETAVREIKS | |
| LG222.2* | MALPVTALLLPLALLLHAARPEVQLVESGGGLVQPGGSL KLSCAASGFTFNKYAMNWVRQAPGKGLEWVARIRSKY NNYATYYADSVKDRFTISRDDSKNTAYLQMNNLKTEDT AVYYCVRHGNFGNSYISYWAYWGQGTLVTVSSGGGGS GGGGSGGGGSGGGGSQTVVTQEPSLTVSPGGTVTLTCG SSTGAVTSGYYPNWVQQKPGQAPRGLIGGTKFLAPGTP ARFSGSLLGGKAALTLSGVQPEDEAEYYCALWYSNRWV FGGGTKLTVLDSGQVLLESNIKVLPTWSTPVQPMALIVL GGVAGLLLFIGLGIFFRVKFSRSADYSARRLRNARAQSR KQRQLKADAKKAIGRLQLRTLKQGDKEIGPDGDSCAVC IELYKPNDLVRILTCNHIFHKTCVDPWLLEHRTCPMCKC DILKALGIEVDVEDGSVSLQVPVSNEISNSASSHEEDNRS ETASSGYASVQGTDEPPLEEHVQSTNESLQLVNHEANSV AVDVIPHVDNPTFEEDETPNQETAVREIKS | 75 |
| GRAIL.IC.ZF mutant (Zinc-finger → CD8a hinge) | YSARRLRNARAQSRKQRQLKADAKKAIGRLQLRTLKQ GDKEIGPDGDSGTTTPAPRPPTPAPTIASQPLSLRPEACRP AAGGAVHTRGLDFACDCDILKALGIEVDVEDGSVSLQV PVSNEISNSASSHEEDNRSETASSGYASVQGTDEPPLEEH VQSTNESLQLVNHEANSVAVDVIPHVDNPTFEEDETPNQ ETAVREIKS | 76 |
| GRAIL.IC.H2N2 mutant | YSARRLRNARAQSRKQRQLKADAKKAIGRLQLRTLKQ GDKEIGPDGDSCAVCIELYKPNDLVRILTCNRIFRKTCVD PWLLEHRTCPMCKCDILKALGIEVDVEDGSVSLQVPVSN EISNSASSHEEDNRSETASSGYASVQGTDEPPLEEHVQST NESLQLVNHEANSVAVDVIPHVDNPTFEEDETPNQETAV REIKS | 77 |
| RNF133 | MHLLKVGTWRNNTASSWLMKFSVLWLVSQNCCRASVV WMAYMNISFHVGNHVLSELGETGVFGRSSTLKRVAGVI | 78 |
| | VPPEGKIQNACNPNTIFSRSKYSETWLALIERGGCTFTQK IKVATEKGASGVIIYNVPGTGNQVFPMFHQAFEDVVVV MIGNLKGTEIFHLIKKGVLITAVVEVGRKHIIWMNHYLV SFVIVTTATLAYFIFYHIHRLCLARIQNRRWQRLTTDLQN TFGQLQLRVVKEGDEEINPNGDSCVICFERYKPNDIVRIL TCKHFFHKNCIDPWILPHGTCPICKCDILKVLGIQVVVEN GTEPLQVLMSNELPETLSPSEEETNNEVSPAGTSDKVIHV EENPTSQNNDIQPHSVVEDVHPSP | |
| RNF133.IC | HRLCLARIQNRRWQRLTTDLQNTFGQLQLRVVKEGDEEI NPNGDSCVICFERYKPNDIVRILTCKHFFHKNCIDPWILP HGTCPICKCDILKVLGIQVVVENGTEPLQVLMSNELPET LSPSEEETNNEVSPAGTSDKVIHVEENPTSQNNDIQPHSV VEDVHPSP | 79 |
| RNF122 | MHPFQWCNGCFCGLGLVSTNKSCSMPPISFQDLPLNIYM VIFGTGIFVFMLSLIFCCYFISKLRNQAQSERYGYKEVVL KGDAKKLQLYGQTCAVCLEDFKGKDELGVLPCQHAFH RKCLVKWLEVRCVCPMCNKPIASPSEATQNIGILLDELV | 80 |
| RNF122.rIC | SKLRNQAQSERYGYKEVVLKGDAKKLQLYGQTCAVCL EDFKGKDELGVLPCQHAFHRKCLVKWLEVRCVCPMCN KPIASPSEATQNIGILLDELV | 81 |
| RNF 152 | METLSQDSLLECQICFNYYSPRRRPKLLDCKHTCCSVCL QQMRTSQKDVRCPWCRGVTKLPPGFSVSQLPDDPEVLA VIAIPHTSEHTPVFIKLPSNGCYMLPLPISKERALLPGDM GCRLLPGSQQKSVTVVTIPAEQQPLQGGAPQEAVEEEQD RRGVVKSSTWSGVCTVILVACVLVFLLGIVLHNMSCISK RFTVISCG | 82 |
| RNF152.rIC | WTSSKVVGRRDQEEEVAEQPAGGQLPQQEAPITVVTVS KQQSGPLLRCGMDGPLLAREKSIPLPLMYCGNSPLKIFV PTHESTHPIAIVALVEPDDPLQSVSFGPPLKTVGRCWPCR VDKQSTRMQQLCVSCCTHKCDLLKPRRRPSYYNFCIQC ELLSDQSLTE | 83 |
| GRAIL- zinc-finger domain | CAVCIELYKPNDLVRILTCNHIFHKTCVDPWLLEHRTCPM CK | 84 |
| CD8α hinge | GTTTPAPRPPTPAPTIASQPLSLRPEACRPAAGGAVHTRG LDFACD | 85 |
| LG116* | MALPVTALLLPLALLLHAARPQVQLVQSGGGVVQPGRS LRLSCKASGYTFTRYTMHWVRQAPGKGLEWIGYINPSR GYTNYNQKFKDRFTISRDNSKNTAFLQMDSLRPEDTGV YFCARYYDDHYSLDYWGQGTPVTVSSGGGGSGGGGSG GGGSDIQMTQSPSSLSASVGDRVTITCSASSSVSYMNWY QQTPGKAPKRWIYDTSKLASGVPSRFSGSGSGTDYTFTIS SLQPEDIATYYCQQWSSNPFTFGQGTKLQITRGSGSGSGS VNISGQNTMNMVKVPECRLADELGGLWENSRFTDCCL CVAGQEFQAHKAILAARSPVFSAMFEHEMEESKKNRVEI NDVEPEVFKEMMCFIYTGKAPNLDKMADDLLAAADKY ALERLKVMCEDALCSNLSVENAAEILILADLHSADQLKT QAVDFINYHASDVLETSGWKSMVVSHPHLVAEAYRSLA SAQCPFLGPPRKRLKQSGTTTPAPRPPTPAPTIASQPLSLR PEACRPAAGGAVHTRGLDFACDIYIWAPLAGTCGVLLLS LVIT | 86 |
| LG118* | MALPVTALLLPLALLLHAARPEVQLVESGGGLVQPGGSL KLSCAASGFTFNKYAMNWVRQAPGKGLEWVARIRSKY NNYATYYADSVKDRFTISRDDSKNTAYLQMNNLKTEDT AVYYCVRHGNFGNSYISYWAYWGQGTLVTVSSGGGGS GGGGSGGGGSGGGGSQTVVTQEPSLTVSPGGTVTLTCG SSTGAVTSGYYPNWVQQKPGQAPRGLIGGTKFLAPGTP ARFSGSLLGGKAALTLSGVQPEDEAEYYCALWYSNRWV FGGGTKLTVLGSGSGSGRLNFGDDIPSALRIAKKKRWNS IEERRIHQESELHSYLSRLIAAERERELEECQRNHEGDED DSHVRAQQACIEAKHDKYMADMDELFSQVDEKRKKRD IPDYLCGKISFELMREPCITPSGITYDRKDIEEHLQRVGHF DPVTRSPLTQEQLIPNLAMKEVIDAFISENGWVEDYGTT TPAPRPPTPAPTIASQPLSLRPEACRPAAGGAVHTRGLDF ACDIYIWAPLAGTCGVLLLSLVIT | 87 |
| EF1α promotor | gggcagagcgcacatcgcccacagtccccgagaagttggggggaggggtcggcaatt gaaccggtgcctagagaaggtggcgcggggtaaactgggaaagtgatgtcgtgtactg gctccgcctttttcccgagggtgggggagaaccgtatataagtgcagtagtcgccgtgaa cgttctttttcgcaacgggtttgccgccagaacacaggtaagtgccgtgtgtggttcccgc gggcctggcctctttacgggttatggcccttgcgtgccttgaattacttccacgcccctggc tgcagtacgtgattcttgatcccgagcttcgggttggaagtgggtgggagagttcgaggc cttgcgcttaaggagccccttcgcctcgtgcttgagttgaggcctggcctgggcgctggg | 88 |
| | gccgccgcgtgcgaatctggtggcaccttcgcgcctgtctcgctgctttcgataagtctct agccatttaaaatttttgatgacctgctgcgacgctttttttctggcaagatagtcttgtaaatg cgggccaagatctgcacactggtatttcggtttttggggccgcgggcggcgacggggc ccgtgcgtcccagcgcacatgttcggcgaggcggggcctgcgagcgcggccaccga gaatcggacgggggtagtctcaagctggccggcctgctctggtgcctggcctcgcgcc gccgtgtatcgccccgccctgggcggcaaggctggcccggtcggcaccagttgcgtga gcggaaagatggccgcttcccggccctgctgcagggagctcaaaatggaggacgcgg cgctcgggagagcgggcgggtgagtcacccacacaaaggaaaagggcctttccgtcc tcagccgtcgcttcatgtgactccacggagtaccgggcgccgtccaggcacctcgatta gttcttttggagtacgtcgtctttaggttggggggaggggttttatgcgatggagtttcccca cactgagtgggtggagactgaagttaggccagcttggcacttgatgtaattctccttggaa tttgccctttttgagtttggatcttggttcattctcaagcctcagacagtggttcaaagtttttttc ttccatttcag | |
| CD3ζ transmembrane domain variant(used in LG222,i.e. CD3ζ.ΔIC-1) | QSFGLLDPKLCYLLDGILFIYGVILTALFL | 89 |
| Intracellular domain (used in LG222, LG222.1, LG222.2) | RVKFSRSAD | 90 |
| Linker between scFv and E3 ligase or between CD8 TM and E3 ligase | GSGSGSG | 91 |

| | | |
|---|---|---|
| * P2A-GFP(i.e. P2A-eGFP) sequence was not included; ***Italics* indicate P2A sequence. | | |

### Example 1

### I. System control construct

LS008, SP- αCD19 scFv-CD8α hinge/TM-P2A-eGFP (Fig. 2), CD19 CAR, that is LS008 was adopted here as a negative control.

### II. Constructs used in Examples 2-3

### Example 2:

LG089, E3 ligase FBW7 fragment (2-293) linked to N-terminus of SP34 scFv (FBW7-SP34 scFv-P2A-eGFP);
LG091, E3 ligase VHL fragment (152-213) linked to C-terminus of SP34 scFv (SP34 scFv-VHL-P2A-eGFP);
LG092, E3 ligase SPOP fragment (167-374) linked to C-terminus of SP34 scFv (SP34 scFv-SPOP-P2A-eGFP);
LG085, E3 ligase CHIP fragment (128-303, CHIP.dTPR) linked to C-terminus of OKT3 scFv (OKT3 scFv-hCHIP.dTPR-P2A-eGFP);

### Example 3:

LG021, SP34 scFv-CD8α hinge/TM-P2A-eGFP;
LG022, membrane-anchored Signal Peptide (SP)-SP34 scFv-CD8α hinge/TM-P2A-eGFP;
LG023, cytoplasmic OKT3 scFv-CD8α hinge/TM-P2A-eGFP;
LG024, SP-OKT3 scFv-CD8α hinge/TM-P2A-eGFP;

### III. Constructs used in Examples 4-17

### Example 4:

LG112, SP-OKT3 scFv-CHIP.dTPR-CD8α hinge/TM-P2A-eGFP;
LG113, SP-OKT3 scFv-CD8α hinge/TM-CHIP.dTPR-P2A-eGFP;

### Example 5:

LG114, SP-OKT3 scFv-FBW7-CD8α hinge/TM-P2A-eGFP;
LG115, SP-OKT3 scFv-VHL-CD8α hinge/TM-P2A-eGFP;
LG116, SP-OKT3 scFv-SPOP-CD8α hinge/TM-P2A-eGFP;
LG117, SP-OKT3 scFv-SOCS2-CD8α hinge/TM-P2A-eGFP;

### Example 6:

LG118, SP-SP34 scFv-hCHIP.dTPR-CD8α hinge/TM-P2A-eGFP;
LG123, SP-UCHT1 scFv-hCHIP.dTPR-CD8α hinge/TM-P2A-eGFP;
LG124, SP-UCHT1.Y177T scFv-hCHIP.dTPR-CD8α hinge/TM-P2A-eGFP;
LG125, SP-L2K scFv-hCHIP.dTPR-CD8α hinge/TM-P2A-eGFP;
LG126, SP-F6A scFv-hCHIP.dTPR-CD8α hinge/TM-P2A-eGFP;

### Example 7:

LG119, SP-BMA031.wt scFv-hCHIP.dTPR-CD8α hinge/TM-P2A-eGFP;
LG120, SP-BMA031.H6L12 scFv-hCHIP.dTPR-CD8α hinge/TM-P2A-eGFP
LG133, SP-BMA031.wt scFv-CD8α hinge/TM-P2A-eGFP
LG134, SP-BMA031.H6L12 scFv-CD8α hinge/TM-P2A-eGFP

### Examples 9-10:

### CD5, CD7, PD-L1, CD47 downregulation constructs

LG137, SP-αCD5.14 scFv-hCHIP.dTPR-CD8α hinge/TM-P2A-eGFP;
LG138, SP-αCD7.TH69 scFv-hCHIP.dTPR-CD8α hinge/TM-P2A-eGFP;
MLB052, SP-αPD-L1 scFv-hCHIP.dTPR-CD8α hinge/TM-P2A-eGFP;
MLB053, SP-αCD47 scFv-hCHIP.dTPR-CD8α hinge/TM-P2A-eGFP;

Optionally, there also may be a Linker (e.g. SEQ ID No. 91) between scFv and E3 ligase.

Optionally, there also may be a Linker (e.g. SEQ ID No. 91) between CD8 TM and E3 ligase.

### Example 12:

LG222, SP-SP34-CD3ζ.ΔIC-1 -GRAIL.IC-P2A-eGFP
LG222.1, SP-SP34-CD8α hinge/TM -GRAIL.IC-P2A-eGFP
LG222.2, SP-SP34-CD4 hinge/TM -GRAIL.IC-P2A-Egfp

Optionally, there also may be an intracellular domain (e.g. SEQ ID No. 90) attached to the C-terminal of TM domain of LG222, LG222.1, LG222.2.

### Example 13:

MLB014, CD3ζ.ΔIC-IL2Rβjm-P2A-eGFP;
MLB046, CD3ε.ΔIC-IL2Rβjm-P2A-eGFP;

### Example 14:

LG171, CD3ζ.ΔIC-GRAIL.IC-P2A-eGFP
LG212, CD3ζ.ΔIC-P2A-eGFP
LG171.ZF, CD3ζ.ΔIC-GRAIL.ZF mutant-P2A-GFP
LG171.H2N2, CD3ζ.ΔIC-GRAIL.IC.H2N2 mutant-P2A-GFP
LG171p1, CAG-CD3ζ.ΔIC-GRAIL.IC-P2A-eGFP

### Example 15:

LG132, CD3ζ-CHIP. DTPR-P2A-GFP
LG213, CD3ζ.ΔIC-CHIP. DTPR-P2A-GFP
RNF133, RNF133-P2A-GFP
LG174, CD3ζ.ΔIC-RNF133.IC-P2A-GFP
RNF122, RNF122-P2A-GFP
LG180, CD3ζ.ΔIC-RNF122.rIC-P2A-GFP
RNF152, RNF152-P2A-GFP
LG183, CD3ζ.ΔIC-RNF152.rIC-P2A-GFP.

### Example 2

### Validation of cytoplasmic constructs (LG089, LG091, LG092, LG085) on TCR/CD3 complex in human primary T cells

Human isolated primary T cells were activated *in vitro* using StemCell Immunocult-XF/Activator (StemCell Technologies) for 3 days, in the presence of human IL-2, IL-7 and IL-15. Then the activated primary T cells were collected and electroporated with pBluescript II SK(+)-PB Donor plasmid expressing one of the cytoplasmic constructs (LG089, LG091, LG092, LG085 constructs) for TCR/CD3 degradation. Two days later, the primary T cells were harvested and stained for both CD3 (PE) and TCRα/β (APC) (Biolegend). Both pmaxGFP (plasmid only express GFP) and LS008 indicated a good transfection efficiency in primary T cells, and there was no downregulation of TCRα/β and CD3 in either of these two constructs **(****Fig. 3A****).** However, there was still no evident degradation of TCR/CD3 complex in GFP+ cells when SP34 scFv and OKT3 scFv were adopted to facilitate engaging the TCR/CD3 complex with E3 ligases, including FBW7 **(****Fig. 3B****),** VHL **(****Fig. 3C****),** SPOP **(****Fig. 3D****)** and hCHIP.dTPR **(****Fig. 3E****),** indicating a potential mistargeting effect in the current system.

### Example 3

### Validation of membrane-anchored constructs (LG021, LG022, LG023, LG024) on TCR/CD3 complex in human primary T cells and Jurkat cell line.

Human isolated primary T cells were activated *in vitro* using StemCell Immunocult-XF/Activator for 3 days, in the presence of human IL-2, IL-7 and IL-15. Then the activated primary T cells were collected and electroporated with plasmid expressing one of the cytoplasmic/membrane-anchored constructs (LG021, LG022, LG023, LG024) for TCR/CD3 degradation. Two days later, the primary T cells were harvested and stained for both CD3 (PE) and TCRα/β (APC). Results showed that the usage of SP34 scFv without E3 ligase/ubiquitin could not elicit any downregulation of TCR/CD3 regardless of whether it is expressed in the cytoplasm (LG021) **(****Fig. 4A****)** or on the cell membrane (LG022) **(****Fig. 4B****).** Even though cytoplasmic expression of OKT3 scFv(LG023) **(****Fig. 4C****)** showed a similar effect as SP34 scFv(LG021), it indeed elicited downregulation of TCR/CD3 complex in GFP+ cells when the expression was anchored on the cellular membrane (LG024) **(****Fig. 4D****).**

The Jurkat cells were electroporated with both PiggyBac transposase mRNA and transposon expressing the membrane-anchored OKT3 construct(LG024). The Jurkat Cells were maintained until day 57 and subjected to flow cytometry testing post staining with CD3 (PE) and TCRα/β (APC), showing a significant degradation of TCR/CD3 complex in cells with stable integration of transposon expressing the membrane-anchored OKT3 construct(LG024) (>85% in GFP positive, **Fig. 5****).**

### Example 4

### Validation of E3 ubiquitin ligase linkage to membrane-anchored OKT3 constructs (LG112, LG113) on TCR/CD3 complex in Jurkat cell line and human primary T cells

### 1. Validation of constructs (LG112, LG113) using Jurkat cells

Jurkat cells were electroporated with both PiggyBac transposase mRNA and transposonexpressing membrane-anchored OKT3 constructs linked to E3 ubiquitin ligase in different manners (LG112, LG113). The Jurkat Cells were harvested on day 3 and subjected to flow cytometry analysis post staining with CD3 (PE) and TCRα/β (APC). **Fig.6A** shows systemic controls pmaxGFP and LS008 have good transfection efficiency in Jurkat cells, and there was no downregulation of TCRα/β and CD3 in either of these two constructs. **Fig.6B** shows Membrane-anchored OKT3 scFv construct without E3 ubiquitin ligase (LG024) shows significant downregulation of TCR-CD3 complex in Jurkat cells. When hCHIP.dTPR was linked to OKT3 scFv in an outside membrane manner (proximal linkage) (LG112) **(****Fig.6C****),** instead of cytoplasmic manner (LG113) **(****Fig.6D****),** even stronger downregulation of TCR/CD3 complex was obtained.

### 2. Validation of constructs (LG112, LG113) using Human isolated primary T cells

Human isolated primary T cells were activated *in vitro* using StemCell Immunocult-XF/Activator for 3 days, in the presence of human IL-2, IL-7 and IL-15. Then the primary T cells were collected and electroporated with both PiggyBac transposase mRNA and transposon expressing different membrane-anchored OKT3 constructs. Three days later, the primary T cells were harvested and stained for both CD3 (PE) and TCRα/β (APC). **Fig.7A** shows systemic controls pmaxGFP and LS008 have good transfection efficiency in primary T cells, and there was no downregulation of TCRα/β and CD3 in either of these two constructs. Similar to what we found in the Jurkat cell line, the degradation could be more significant when hCHIP.dTPR was linked to OKT3 scFv in an outside membrane manner (proximal linkage) (LG112) **(****Fig.7C****),** instead of cytoplasmic manner (LG113) **(****Fig.7D****).** No significant changes on TCR/CD3 degradation were found when E3 ubiquitin ligase (CHIP.dTPR) was located in the cytoplasmic portion of the whole construct (LG113, **Fig. 7D****)** in comparison to Membrane-anchored OKT3 scFv construct without E3 ubiquitin ligase (LG024) **(****Fig. 7B****).**

### Example 5 Validation of TCR/CD3 complex downregulation effects by different E3 ligases linked to membrane-anchored OKT3 constructs in human primary T cells

A list of constructs with different E3 ligases, including FBW7, VHL, SPOP and SOCS2, linked to membrane-anchored OKT3 scFv were designed and synthesized **(****Fig. 8A****).** Human isolated primary T cells were activated *in vitro* using StemCell Immunocult-XF/Activator for 3 days, in the presence of human IL-2, IL-7 and IL-15. Then the primary T cells were collected and electroporated with plasmid expressing one of the membrane-anchored constructs for TCR/CD3 degradation. Three days later, the primary T cells were harvested and stained for both CD3 (PE) and TCRα/β (APC). Results showed that, when OKT3 scFv was adopted, E3 ligases, including FBW7 **(****Fig. 8B****),** VHL **(****Fig. 8C****)** and SOCS2 **(****Fig. 8E****),** could elicit mild downregulation of TCR/CD3. Intriguingly, SPOP may confer much stronger downregulation of TCR/CD3 in human primary T cells **(****Fig. 8D****).**

### Example 6 Validation of TCR/CD3 Degradation mediated by different CD3 targeting scFv

A list of TCR/CD3 degradation constructs with different CD3 targeting scFv, including SP34, UCHT1, UCHT1.Y177T, L2K and F6A, were designed and synthesized **(****Fig. 9A****).** Human isolated primary T cells were activated *in vitro* using StemCell Immunocult-XF/Activator for 3 days, in the presence of human IL-2, IL-7 and IL-15. Then the primary T cells were collected and electroporated with both PiggyBac transposase mRNA and transposon DNA constructs. Three days later, the primary T cells were harvested and stained for both CD3 (PE) and TCRα/β (APC). Results showed that, by directly linking to CHIP.dTPR in an outside membrane manner in the constructs, SP34 scfv (Fig. 9B) and F6A scfv (Fig. 9F) could elicit downregulation of TCR/CD3; while UCHT1 scfv (Fig. 9C), UCHT1.Y177T scfv (Fig. 9D), and L2K scfv (Fig. 9E) may confer much stronger downregulation of TCR/CD3 in human primary T cells **(****Fig. 9B-F).**

### Example 7 E3 ligase (CHIP.dTPR) is necessary for optimized TCR/CD3 downregulation in human primary T cells.

Alist of TCR degradation constructs with TCR targeting scFv, BMA031 or BMA031.H6L12, were designed and synthesized in the presence or absence of CHIP.dTPR **(****Fig. 10A****).** Human isolated primary T cells were activated *in vitro* using StemCell Immunocult-XF/Activator for 3 days, in the presence of human IL-2, IL-7 and IL-15. Then cells were collected and electroporated with both PiggyBac transposase mRNA and transposon DNA constructs. Three days later, the primary T cells were harvested and stained for both CD3 (PE) and TCRα/β (APC). Results showed that, in the presence of CHIP.dTPR, both TCR targeting scFv (BMA031 and BMA031.H6L12) led to strong transfection and clear downregulation of TCR/CD3 complex **(****Fig. 10B****-****Fig. 10C****).** However, in the absence of CHIP.dTPR, the primary T cells may not survive the transfection process, in which case only a minimal fraction of cells showed successful expression of the geneof-interest **(****Fig. 10D****-****Fig. 10E****).** Furthermore, LG024, another TCR/CD3 downregulation construct without E3 ligase, could not achieve good transfection (Fig. 4D) or TCR/CD3 downregulation (Fig. 7B) in human primary T cells either. Accordingly, it appears that E3 ligase is necessary for optimized TCR/CD3 downregulation in human primary T cells.

### Example 8 CD3 downregulation confers lower activation through CD3/CD28 stimulation in Jurkat cells.

Jurkat-NFAT cells were electroporated with both PiggyBac transposase mRNA and transposon expressing different membrane-anchored OKT3 constructs (LG024, LG112) or control LS008. Cells were maintained for 14 days and their activation status before and after CD3/CD28/CD2 Immunocult/Activator re-stimulation was checked. Expression of the constructs LG024, LG112 was inferred by joint GFP expression (vertical coordinates of the right panel in Fig. 11A-B) in the transduced Jurkat cells (i.e. transduced Jurkat cells); CD69(horizontal ordinates of the right panel in Fig. 11A-B) is an activation marker that is upregulated within hours of T cell activation, CD69 expression was evaluated using a PE-conjugated anti-CD69 primary antibody (BioLegend, San Diego, CA). Results showed that CD69 (a T cell activation marker) expression levels were low for all cells at the baseline (Fig. 11A). However, the CD69 expression levels in either LG024 or LG112 transfected GFP+ cells were significantly less compared to T cells from LS008 transfected cells post stimulation (Fig. 11B). This finding indicated that TCR/CD3 downregulation conferred lower responsiveness to activation through CD3/CD28/CD2 stimulation for T cells.

### Example 9 CD5 or CD7 downregulation constructs and validation.

A list of CD5 or CD7 degradation constructs were designed and synthesized **(****Fig. 12A****).** Human isolated primary T cells were activated *in vitro* using StemCell Immunocult-XF/Activator for 3 days, in the presence of human IL-2, IL-7 and IL-15. Then the primary T cells were collected and electroporated with both PiggyBac transposase mRNA and transposon DNA constructs. Four days later, cells were harvested and stained for both CD5 (PE) and CD7 (APC) expressions (Fig. 12B-Fig. 12C). Results showed that αCD5.14 scFv conferred strong downregulation of CD5 **(****Fig. 12B****).** For CD7, αCD7. TH69 scFv (CD7-targeting scFv) also showed significant downregulation effect in human primary T cells **(****Fig. 12C****).**

### Example 10 Downregulation of PD-L1 and CD47 by E3 CHIP construct

Transposon-mediated delivery of E3 ligase constructs was used to modify expression of surface molecules in Jurkat cells **(****Fig. 13B****).** The EF1α promoter was used to drive expression of an antigen-specific ScFv-E3 ligase chimeric fusion protein. A P2A sequence was used to drive simultaneous GFP expression for the identification and characterization of cells expressing the E3 Ligase system **(****Fig. 13A****).** Jurkat cells were electroporated with PiggyBac vectors expressing MLB052 or MLB053 and Piggy Bac transposase mRNA. Following stable integration of the transposon into the Jurkat cell genome, degradation of PD-L1 and CD47 was evaluated by flow cytometry at day 5 post-electroporation. Gating on GFP-positive cells, i.e., those expressing the E3 ligase construct, showed a marginal downregulation of PD-L1 surface expression (i.e. a small left shift of the black line marked area to the dashed line marked area in the upper panel of right column of Fig. 13B). Under standard cell culture conditions - 5% CO₂ in RPMI media supplemented with SmM HEPES and 10% FBS - Jurkat cells do not strongly express PD-L1. An identical gating strategy on Jurkat cells expressing MLB053 showed strong downregulation of CD47 expression in those cells expressing the E3 ligase construct (i.e. a significant left shift of the black line marked area to the dashed line marked area in the lower panel of right column of Fig. 13B). As would be expected, parental Jurkat cells show a bright staining for CD47(the dashed line marked area).

### Example 11 Paracrine downregulation of TCR expression by E3 CHIP linked CD3-targeting construct (LG112).

To confirm that the E3 ligase construct was able to downregulate surface molecules on adjacent cells in an antigen-specific manner, we designed a co-culture experiment between Jurkat cells transduced to express LG112 and parental stock cells. To distinguish between the two populations, the parental Jurkat cells were labeled with CellTrace Violet dye.

Fig 14A provides an overview of the gating strategy showing delineation between the CellTrace Violet-positive parental cells(i.e. Cell Trace ⁺ cells, without LG024-transfection) and CellTrace Violet-negative E3 ligase-transfected cells(i.e. Cell Trace ⁻ cells, LG024-transfected). In the upper panel of left column of Fig. 14A, the CellTrace Violet negative population (i.e. Cell Trace ⁻ cells, LG024-transfected) was further subdivided into a GFP-positive population expressing the E3 ligase construct (the right filled area in the middle column of Fig. 14A) and a GFP-negative population(the left filled area in the middle column of Fig. 14A), not expressing the E3 ligase construct. At a high ratio of E3 ligase-positive cells(i.e. GFP-positive population, 77.8%) to E3 ligase-negative cells(i.e. GFP-negative population, 22.2%); it was found that TCR expression, as determined by CD3 staining, was low across the entire cell population(i.e. the upper panel of right column of Fig. 14A).

Figure 14B shows, as the percentage of E3-ligase positive cells was reduced through serial dilutions with parental Jurkat cells, from approximately 78% positive to 11% positive, we observed an increase in TCR surface expression in the CellTrace Violet-positive parental cell population. Thus, in this system, TCR expression on adjacent cells is inversely related to the number of E3-ligase expressing cells within the population. These results show that our E3 ligase constructs promote antigen-specific downregulation of target molecules in non-E3 ligase-expressing cells in a paracrine fashion. Of note, the extent of TCR downregulation in E3 ligase-expressing cells is also dependent on the expression profile of neighboring cells, with more complete knockdown when the fraction of E3 ligase-expressing cells is higher. Diluting the number of E3 ligase positive cells also rescued the phenotype of CellTrace Violet-negative, GFP-negative cells within the transfected cell population, as seen by the increase of TCR positive cells in this population.

### Example 12 Hinge/transmembrane domain (TM) choice affected the downregulation of TCR/CD3 complex mediated through both SP34 and GRAIL.IC.

To enhance the specific engagement between CD3 and GRAIL.IC (E3 functional domain), CD3-targeting scFv, SP34, was adopted and linked to GRAIL.IC through different hinge/transmembrane domains as listed in Fig.15A. All three constructs, LG222, LG222.1 and LG222.2 were tested in both Jurkat and human primary T cells.

### 1. LG222, LG222.1 and LG222.2 were tested in both Jurkat cells.

At day 3 post-electroporation, Jurkat cells were harvested and stained for TCR expression using αCD3 and αTCR α/β antibodies. Cells expressing the construct were identified based on GFP co-expression on the fusion protein constructs. Results showed that both CD3ζ. ΔIC(LG222) (Fig.15B) and CD4 hinge/TM(LG222.2) (Fig.15D) worked better than CD8a hinge/TM(LG222.1) (Fig.15C), which is only less fraction of GFP⁺ cells showing downregulation of TCR/CD3 on cell surface(upper left area in the third and fourth panel from left to right of Fig.15C).

### 2. LG222, LG222.1 and LG222.2 were tested in human primary T cells.

Pre-activated primary T cells electroporated for expressing LG222, LG222.1 or LG222.2 construct were harvested at day 3 post-electroporation and TCR/CD3 expression was evaluated using both αCD3 and αTCRα/β antibodies (Fig.16A-C). Similarly as Jurkat cells, significant downregulation of TCR/CD3 expression was achieved by construct using CD3ζ. ΔIC domain (Fig.16A, LG222). Using CD8a hinge/TM domain only conferred TCR/CD3 downregulation in a small fraction of GFP⁺ cells (Fig.16B, LG222.1). However, multiple repeats of construct LG222.2 did not achieve good GFP expressions to draw a solid conclusion on TCR/CD3 downregulation (Fig.16C, LG222.2).

### Example 13 Expression of CD3-IL2Rβ juxtamembrane fusion proteins promotes TCR complex surface instability

MLB014/(CD3ζ.ΔIC-IL2Rβjm) construct and MLB046/(CD3ε.ΔIC-IL2Rβjm) construct are shown in Fig. 18A.

### 1. Jurkat cells transfected with MLB014 or MLB046

At day 5 post-electroporation with either MLB014/(CD3ζ.ΔIC-IL2Rβjm) construct or MLB046/(CD3ε.ΔIC-IL2Rβjm) construct, Jurkat cells were harvested and stained for TCR expression using an αCD3 antibody(i.e.anti- CD3 antibody). Cells expressing the construct were identified based on GFP co-expression on the fusion protein constructs. Gating on the GFP positive population indicated that there was a large negative shift in detectable TCR surface expression (upper left area in the second and third panel from left to right of Fig 18B). These data suggest that incorporation of MLB014/(CD3ζ.ΔIC-IL2Rβjm) construct or MLB046/(CD3ε.ΔIC-IL2Rβjm) into the TCR complex promotes increased internalization of the entire complex (Fig.18B).

### 2. Primary T cells transfected with MLB014 or MLB046

Primary T cells electroporated for expressing either MLB014/(CD3ζ.ΔIC-IL2Rβjm) construct or MLB046/(CD3ε.ΔIC-IL2Rβjm) were harvested at day 5 post-electroporation and TCR expression was evaluated using both αCD3 and αTCRα/β antibodies (Fig.18C). Here, downregulation of TCR expression in primary T cells was less pronounced than in Jurkat cells, but still observable as a negative correlation between GFP expression and TCR or CD3 expression(upper left in each panel of the second and third column from left to right of Fig 18C). These data suggest that the same underlying mechanisms are working in both primary T cells and Jurkat cells, but that the efficiency of TCR downregulation is somewhat reduced in primary T cells.

### Example 14 Expression of LG171/(CD3ζ. ΔIC/GRAIL.IC) fusion protein dramatically promotes TCR/CD3 complex expression downregulation in both Jurkat cells and human primary T cells

### 1. Jurkat cells transfected with LG171

LG171/(CD3ζ. ΔIC/GRAIL.IC) fusion protein construct is shown in Fig.19.

At day 3 post-electroporation with LG171(i.e.CD3ζ. ΔIC-GRAIL.IC) fusion protein construct, Jurkat cells were harvested and stained for TCR expression using an αCD3 antibody and αTCR α/β antibody. Cells expressing the construct were identified based on GFP co-expression on the fusion protein constructs. Gating on the GFP positive population indicated that there was a majority shift into undetectable TCR surface expression. These data suggest that incorporation of CD3ζ. ΔIC/GRAIL.IC construct into the TCR complex promotes increased instability of the entire complex (Fig.20).

### 2. Primary T cells transfected with LG171

Primary T cells electroporated for expressing LG171(i.e.CD3ζ. ΔIC-GRAIL.IC) construct were harvested at day 3 post-electroporation and TCR/CD3 expression was evaluated using both αCD3 and αTCRα/β antibodies (Fig.21). Similarly, downregulations of TCR/CD3 expression were significant in GFP+ fraction. These data suggest that the same underlying mechanisms of using CD3ζ. ΔIC-GRAIL.IC fusion protein are effective in both primary T cells and Jurkat cells.

### 3. LG212 (without E3 ligase)

Primary T cells were electroporated with PiggyBac vector LG212 and PiggyBac Transposase. At day 3 post-electroporation, TCR expression was evaluated by staining for CD3 and TCRα/β. We could not see any TCR/CD3 downregulation when only truncated CD3ζ (i.e.CD3ζ. ΔIC) was adopted in human primary T cells (Fig. 22).

### 4. Primary T cells transfected with construct LG171.ZF(contains domain switch) or LG171.H2N2(contains point mutation)

Two more constructs LG171.ZF, LG171.H2N2 were generated by switching zinc-finger domain (SEQ ID NO. 84) in GRAIL with CD8α hinge (SEQ ID NO. 85) or introducing point mutations into the zinc-finger domain (Fig.23A). Both FACS (Fig.23B) and western blot (Fig.23C) tests indicated that functional zinc-finger domain of GRAIL is necessary for a better TCR/CD3 downregulation in LG171 (Fig. 21).

### 5. LG171 chimeric proteins could not confer non-specific downregulation of CD5 or CD7

As with the Jurkat reporter cells, primary T cells were electroporated with PiggyBac vector LG171 and PiggyBac Transposase. At day 3 post-electroporation, both CD5 and CD7 expressions were evaluated by flow cytometry. The downregulation effect in primary T cells by LG171 was TCR/CD3 specific, as neither CD5 nor CD7 was affected at all (Fig. 24).

### 6. T cells transfected with LG171p1/(CAG/CD3ζ. ΔIC/GRAIL.IC)

CAG promoter is a good alternative conferring significant and sustainable downregulation of TCR/CD3 complex in both Jurkat cells and human primary T cells

Other than EF1a promoter tested in the other examples, CAG promoter was also investigated base on LG171 design. To achieve this purpose, new construct LG171p1 was designed (Fig. 25A) and tested in both Jurkat and human primary T cells.

### 6.1 Jurkat cells transfected with LG171p1

At day 3 post-electroporation with LG171p1/(CAG/CD3ζ. ΔIC/GRAIL.IC) fusion protein construct (Fig.25B), Jurkat cells were harvested and stained for TCR expression using αCD3 and αTCR α/β antibodies. Cells expressing the construct were identified based on GFP co-expression on the fusion protein constructs. Gating on the GFP positive population indicated that there was a majority shift into undetectable TCR surface expression (upper left area in the third and fourth panel from left to right of Fig 25B) in Jurkat. These data suggest that CAG promoter could be a good alternative for EF1a in Jurkat conferring significant gene expression level.

### 6.2 Primary T cells transfected with LG171p1

Pre-activated primary T cells electroporated for expressing LG171p1 construct were harvested at day 3 post-electroporation and TCR/CD3 expression was evaluated using both αCD3 and αTCRα/β antibodies (Fig.25C). Similarly, downregulations of TCR/CD3 expression were significant in GFP+ fraction (upper left in each panel of the third and fourth column from left to right of Fig 25C). These data suggest that the same underlying mechanisms of using LG171p1 fusion protein are working in both primary T cells and Jurkat cells. More significantly, long-term follow-up indicated the downregulation of TCR/CD3 could be maintained until day 13. (Fig. 25C)

### Example 15 TCR/CD3 Degradation constructs with different E3 ligases (CD3ζ. ΔIC with different E3 ligases other than GRAIL.IC).

1. We also tested CHIP for TCR downregulation in a similar design as LG171 (Fig.26A). Compared to no effects in LG132 with full length of CD3ζ, the downregulation in LG213 was mainly contributed by CD3ζ truncation(i.e. CD3ζ. ΔIC) (Fig.26B).
2. Other E3 ligases, including RNF133, RNF122 and RNF152, were also tested in a similar design as LG171 (Fig. 27A). Both FACS and western blot showed consistent results that TCR downregulated when E3 ligase functional domains were linked to CD3ζ truncation (Fig. 27 B-C).

### Example 16 Decreased activation in cells transfected with LG171 (CD69, ppERK, IFN-γ)

### 1. CD69, ppERK levels in Jurkat cells transfected with indicated constructs

To check the impacts on activation, Jurkat cells transfected with indicated constructs were briefly stimulated by aCD3/CD28(anti CD3/CD28 antibody) and then tested for CD69 upregulation and intracellular ERK phosphorylation (i.e. ppERK or ERK) levels. Both assays showed that LG171 significantly diminished the upregulation of CD69 and ERK phosphorylation (Fig.28A- Fig.28B). Even though CD3z truncation(i.e. CD3ζ.ΔIC) may delay ERK phosphorylation(left shift of the filled area to black line marked area) in a much shorter time stimulation (20 min, Fig.28B) than GFP, CD3ζ, GRAIL, it still could not decrease CD69 upregulation when cells were stimulated for 4 hrs (Fig 28A). However, both upregulations of CD69, ppERK were significantly delayed/inhibited in LG171, indicating synergistic effects between CD3ζ truncation(i.e. CD3ζ.ΔIC) and GRAIL.

### 2. IFN-γ level in primary T cell transfected with indicated constructs

We also tested the impacts on human primary T cell activation. Pre-activated primary T cells were transfected with indicated constructs. Several days later, T cells were re-stimulated with OKT3(anti CD3 antibody) for CD69 upregulation and IFN-y cytokine secretion. Similar to what we found in Jurkat, human primary T cells transfected with either LG171 or LG171p1 (CAG promoter) showed significant lower CD69 upregulation(Fig.29A) and IFN-y secretion(Fig.29B). When PHA-L(Fig.29B), a stronger TCR cross-linking chemical, was adopted, both LG171 and LG171p1 also confer lower IFN-y secretion compared to GFP control, indicating a safer strategy to avoid potential GvHD response during allogeneic T cell transfusion.

To further confirm this finding, MLR assay was employed(Fig.29C). In detail, mock T cells or T cells transfected with LG171p1 were cocultured with in-vitro derived allogeneic mature DC cells for 5 days. Then IFN-y levels within supernatant were tested by ELISA. Similarly, LG171p1 dramatically reduced IFN-y secretion when tested with mature DC from two different donors.

### Example 17 Expression and killing efficiency of LG171.CldnCar

To date, all CAR T cell therapies currently approved for clinical use rely on autologous T cell engineering and re-engraftment. To standardize therapies across donors and reduce the cost of CAR T cell therapies, there is a need to develop robust allogeneic T cell technologies.
1. CldnCar and LG171.CldnCar fusion protein construct are shown in Fig.30A. We combined the first nucleic acid encoding GRAIL-based ReceptorTAC (i.e.LG171 or CD3ζ-GRAIL.IC) into a single transposon vector with the second nucleic acid encoding a CAR (claudin18.2 CAR) to generate the LG171.CldnCar(i.e. LG171/claudin18.2 CAR joint expression construct).
2. TCR downregulation is detected by αCD3ε (anti-CD3 antibody, for CD3 expression) and CAR expression is detected by αF(ab')₂ staining (for scFv expression); the results are shown in Fig.30B.
   T cells expressing the ReceptorTAC/CAR construct were gated on GFP expression (the first peak from top to bottom in both left and right panels of Fig.30B). From the left panel of Fig.30B, it can be seen that, T cells transfected with LG171.CldnCar (the lowest peak) and LG171 (the second peak from top to bottom) lack of TCR expression compared to T cells transfected with CldnCar (the third peak from top to bottom). From the right panel of Fig.30B, it can be seen that, T cells transfected with LG171 lack of scFv expression (second peak from top to bottom), while T cells transfected with LG171.CldnCar have normal scFv expression (the lowest peak) compared to T cells transfected with CldnCar (the third peak from top to bottom).
3. To test CAR functionality in the TCR-deficient T cells, we assayed their cytotoxic potential against HEK-cldn18.2 cells, which are transduced to overexpress CLDN18.2, and HEK293T cells (i.e.parental HEK cells), which are claudin18.2-negative, the results are shown in Fig.30C.
   From the left panel of Fig.30C, it can be seen that, no cytotoxicity was detected against the claudin18.2-negative parental HEK cell line (i.e. HEK). From the right panel of Fig.30C, it can be seen that, T cells expressing the LG171/claudin18.2 CAR joint expression construct (i.e. LG171.CldnCar) showed antigen-specific cytotoxicity identical to cells expressing the claudin18.2 CAR(i.e. CldnCar) control plasmid (i.e. HEK-CLDN 18.2). Antigen- specific cell lysis increased with an increasing E:T ratio. T cells expressing LG171 alone showed minimal cytotoxicity in response to target cells expressing claudin18.2, indicating that this effect was indeed CAR-mediated.
4.Taken together, we have shown that E3 ligase fusion proteins can be used to downregulate TCR expression and prevent T cell activation, and that this effect is TCR-specific and does not affect CAR efficacy in a co-expression system. Thus, we believe that the ReceptorTAC platform represents a novel means of generating allogeneic CAR T cells through manipulation of TCR expression at the protein level.

## Claims

1. A fusion protein comprising:
degradation tag protein that mediates degradation of a membrane-bound protein (MBP), wherein the degradation tag protein comprises an ubiquitin ligase, or one or more subunits or structural domains of the cell surface receptors which is an Interleukin receptor that mediate degradation; and
a transmembrane-linking domain comprising:
1) a transmembrane domain and a linking protein;
wherein the transmembrane domain is selected from the transmembrane domain of one or more subunits or structural domains of CD8α, CD4, CD3, CD28, 4-1BB and IL2R; and the linking protein is selected from one or more of an antibody or antigen-binding fragment specific binding to the MBP to be degraded; and the transmembrane domain is linked directly or indirectly to the linking protein; or
2) a transmembrane domain of the MBP to be degraded;
wherein
(a) preferably, the MBP is a membrane-bound receptor; more preferably a mammalian origin membrane-bound receptor, most preferably a human membrane-bound receptor;
(b) preferably, the ubiquitin ligase comprises one or more subunits or structural domains of E3 ubiquitin ligase or a variant thereof;
(c) preferably, the antigen-binding fragment specific binding to MBP to be degraded in the above 1) comprises a scFv, nano antibody specifically binding to the MBP or a variant thereof;
(d) preferably, the fusion protein having the transmembrane-linking domain of above 1) further comprises a signal peptide;
(e) preferably, the transmembrane domain of the MBP to be degraded in the above 2) comprises a transmembrane domain of one or more of CD3ζ, CD3γ, CD3δ, CD3ε, CD3α, CD3β or a variant thereof; or
(f) preferably, the transmembrane-linking domain of above 2) comprises an extracellular domain and a transmembrane domain of one or more of CD3ζ, CD3γ, CD3δ, CD3ε, CD3α, CD3β.
(g) more preferably, the transmembrane-linking domain of above 2) comprises a signal peptide, extracellular domain and a transmembrane domain of one or more of CD3ζ, CD3γ, CD3δ, CD3ε, CD3α, CD3β.

2. The fusion protein of claim 1, wherein,
the membrane-bound receptor is selected from one or more subunits or structural domains of CD3, TCR, CD5, CD7, PD-L1, and CD47;
(a) the E3 ubiquitin ligase is selected from the following proteins or the truncated forms thereof: C-terminus of Hsc70-interacting protein (CHIP), CHIP.dTPR, F-box WD40-containing protein 7 (FBW7), FBW7.2-293, von Hippel-Lindau (VHL), VHL.152-213, Speckle-type BTB-POZ protein (SPOP), SPOP.167-374, SOCS2, SOCS2.143-198, Ubiquitin-protein ligase E3A (UBE3A), Mouse double minute 2 homolog (MDM2), Anaphase-promoting complex (APC), UBR5, LNX, Casitas B-lineage lymphoma-transforming sequence-like protein 1 (CBLL1), HECT domain and ankyrin repeat containing E3 ubiquitin protein ligase 1 (HACE1), HECT, C2 and WW domain containing E3 ubiquitin protein ligase 1 (HECW1), HECT, C2 and WW domain containing E3 ubiquitin protein ligase 2 (HECW2), HECT And RLD Domain Containing E3 Ubiquitin Protein Ligase 1 (HERC1), HERC2, HERC3, HERC4, HERC5, HERC6, HUWE1, ITCH, neural precursor cell expressed developmentally down-regulated protein 4 (NEDD4), Neural precursor cell expressed developmentally downregulated gene 4-like (NEDD4L), Peptidylprolyl isomerase (cyclophilin)-like 2 (PPIL2), PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UBOX5, UBR5, WWP1, WWP2, Parkin, MKRN1, GRAIL.IC, RNF133.IC, RNF122.rIC or RNF152.rIC; preferably, the E3 ubiquitin ligase comprises CHIP, CHIP.dTPR, FBW7.2-293, VHL.152-213, SPOP.167-374, SOCS2.143-198, GRAIL.IC, RNF133.IC, RNF122.rIC or RNF152.rIC; and more preferably, the CHIP, CHIP.dTPR, FBW7.2-293, VHL.152-213, SPOP.167-374, SOCS2.143-198, GRAIL.IC, RNF133.IC, RNF122.rIC or RNF152.rIC comprises an amino acid sequence at least about 95%, 96%, 97%, 98%, 99% or 100% identical to the amino acid sequence shown in any one of SEQ ID NOs. 21-26,62,79,81 or 83 respectively;
(b) the subunits or structural domains of Interleukin receptors comprises a lysosomal targeting motif of IL-2R; preferably, the lysosomal targeting motif of IL-2R comprises L2Rβ jm domain; more preferably, the L2Rβ jm domain(IL2Rβjm) comprises an amino acid sequence at least about 95%, 96%, 97%, 98%, 99% or 100% identical to the amino acid sequence shown in SEQ ID NO. 58;
(c) the transmembrane domain in the above 1) is selected from a transmembrane domain of CD3, CD8α, CD4; preferably, CD3ζ transmembrane domain variant, CD8α transmembrane domain, or CD4 transmembrane domain comprises an amino acid sequence at least about 95%, 96%, 97%, 98%, 99% or 100% identical to the amino acid sequence shown in SEQ ID NO. 89, 18 or 15 respectively;
(d) the signal peptide in the above 1) is selected from an amino acid sequence at least about 95%, 96%, 97%, 98%, 99% or 100% identical to the amino acid sequence shown in SEQ ID NO. 19 or 20 respectively;
(e) the antibody or antigen-binding fragment specific binding to the MBP in the above 1) is selected from an antibody or antigen-binding fragment of CD3, CD5, CD7, PD-L1 or CD47; preferably, antigen-binding fragment of CD3 comprises SP34 scFv, OKT3 scFv, UCHT1 scFv, UCHT1.Y177T scFv, L2K scFv, F6A scFv, BMA031 scFv, or BMA031.H6L12 scFv; antigen-binding fragment of CD5 comprises αCD5.14 scFv; antigen-binding fragment of CD7 comprises αCD7.TH69 scFv; antigen-binding fragment of PD-L1 comprises αPD-L1 scFv; antigen-binding fragment of CD47 comprises αCD47 scFv; more preferably, the SP34 scFv, OKT3 scFv, UCHT1 scFv, UCHT1.Y177T scFv, L2K scFv, F6A scFv, BMA031 scFv, BMA031.H6L12 scFv, αCD5.14 scFv, αCD7.TH69 scFv, αPD-L1 scFv or αCD47 scFv comprises an amino acid sequence at least about 95%, 96%, 97%, 98%, 99% or 100% identical to the amino acid sequence shown in any one of SEQ ID NOs. 2-13 respectively;
(f) the transmembrane-linking domain of above 2) comprises a transmembrane domain of CD3ζ or CD3ε, preferably, an extracellular domain and a transmembrane domain of CD3ζ or CD3ε, more preferably, a second signal peptide, extracellular domain and a transmembrane domain of CD3ζ or CD3ε; more preferably, the transmembrane domain(TM) of CD3ζ or CD3ε comprises an amino acid sequence at least about 95%, 96%, 97%, 98%, 99% or 100% identical to the amino acid sequence shown in SEQ ID NO. 67, 70 respectively; more preferably, the extracellular domain(EM) of CD3ζ or CD3ε comprises an amino acid sequence at least about 95%, 96%, 97%, 98%, 99% or 100% identical to the amino acid sequence shown in SEQ ID NO. 66, 69 respectively; more preferably, the second signal peptide of CD3ζ or CD3ε comprises an amino acid sequence at least about 95%, 96%, 97%, 98%, 99% or 100% identical to the amino acid sequence shown in SEQ ID NO. 65, 68 respectively; more preferably, the second signal peptide, extracellular domain and a transmembrane domain of CD3ζ (CD3ζ.ΔIC) and the second signal peptide, extracellular domain and a transmembrane domain of CD3ε (CD3ε.ΔIC) comprises an amino acid sequence at least about 95%, 96%, 97%, 98%, 99% or 100% identical to the amino acid sequence shown in SEQ ID NO. 61, 64 respectively.

3. The fusion protein according to any one of claims 1-2, wherein
the fusion protein further comprises one or more of a hinge or a P2A-GFP;
preferably, the hinge is selected from a CD8α hinge or CD4 hinge; more preferably, CD8α hinge or CD4 hinge comprises an amino acid sequence at least about 95%, 96%, 97%, 98%, 99% or 100% identical to the amino acid sequence shown in SEQ ID NO.:16 or 14, respectively; more preferably, the hinge is adjacent to the N-terminal of the first transmembrane domain,
preferably, P2A-GFP comprises an amino acid sequence at least about 95%, 96%, 97%, 98%, 99% or 100% identical to the amino acid sequence shown in SEQ ID NO.:57; more preferably, the P2A-GFP sequence is at the C-terminal of the fusion protein.

4. The fusion protein according to any one of claims 1-3, wherein the fusion protein comprises from N-terminal to C-terminal:
a) the linking protein, the intracellular degradation tag protein, the transmembrane domain;
preferably, the linking protein comprises the antibody or antigen-binding fragment specific binding to the MBP to be degraded; more preferably, the antibody or antigen-binding fragment specific binding to the MBP to be degraded comprises antibody or antigen-binding fragment of CD3, CD5, CD7, PD-L1 or CD47; more preferably, antigen-binding fragment of CD3 comprises SP34 scFv, OKT3 scFv, UCHT1 scFv, UCHT1.Y177T scFv, L2K scFv, F6A scFv, BMA031 scFv, or BMA031.H6L12 scFv; antigen-binding fragment of CD5 comprises αCD5.14 scFv; antigen-binding fragment of CD7 comprises αCD7.TH69 scFv; antigen-binding fragment of PD-L1 comprises αPD-L1 scFv; antigen-binding fragment of CD47 comprises αCD47 scFv;
preferably, the degradation tag protein comprises the E3 ubiquitin ligase; more preferably, the E3 ubiquitin ligase comprises CHIP.dTPR (CHIP: C-terminus of Hsc70-interacting protein), FBW7.2-293 (FBW7: F-box WD40-containing protein 7), VHL.152-213 (von Hippel-Lindau), SPOP.167-374 (Speckle-type BTB-POZ protein), SOCS2.143-198;
preferably, the transmembrane domain comprises CD8α transmembrane domain;
b) the linking protein, transmembrane domain, the intracellular degradation tag protein;
preferably, the linking protein comprises the antibody or antigen-binding fragment specific binding to the MBP to be degraded; more preferably, the antibody or antigen-binding fragment specific binding to the MBP to be degraded comprises antibody or antigen-binding fragment of CD3; more preferably, antigen-binding fragment of CD3 comprises SP34 scFv;
preferably, the intracellular degradation tag protein comprises the E3 ubiquitin ligase; more preferably, E3 ubiquitin ligase comprises GRAIL.IC;
preferably, the transmembrane domain is selected from one or more of CD3ζ transmembrane domain variant, CD8α transmembrane domain, and CD4 transmembrane domain;
c) the transmembrane-linking domain of the above 2), the cell surface receptors that mediate degradation;
preferably, the transmembrane-linking domain of the above 2) comprises CD3ζ.ΔIC or CD3ε.ΔIC;
preferably, the cell surface receptors that mediate degradation comprises IL2Rβjm; or
d) the transmembrane-linking domain of the above 2), the one or more of an E3 ubiquitin ligase;
preferably, the transmembrane-linking domain of the above 2) comprises CD3ζ.ΔIC;
preferably, the one or more of an E3 ubiquitin ligase or a variant thereof comprises GRAIL.IC, CHIP.dTPR, RNF133.IC, RNF122.rIC, RNF152.rIC.

5. The fusion protein of claim 4, wherein the fusion protein comprises from N-terminal to C-terminal:
a) :
SP-OKT3 scFv-CHIP.dTPR-CD8α hinge - CD8α TM-P2A-eGFP;
SP-OKT3 scFv-FBW7-CD8α hinge- CD8α TM-P2A-eGFP;
SP-OKT3 scFv-VHL-CD8α hinge- CD8α TM-P2A-eGFP;
SP-OKT3 scFv-SPOP-CD8α hinge- CD8α TM-P2A-eGFP;
SP-OKT3 scFv-SOCS2-CD8α hinge- CD8α TM-P2A-eGFP;
SP-SP34 scFv-hCHIP.dTPR-CD8α hinge- CD8α TM-P2A-eGFP;
SP-UCHT1 scFv-hCHIP.dTPR-CD8α hinge- CD8α TM-P2A-eGFP;
SP-UCHT1.Y177T scFv-hCHIP.dTPR-CD8α hinge-CD8αTM-P2A-eGFP;
SP-L2K scFv-hCHIP.dTPR-CD8α hinge- CD8α TM-P2A-eGFP;
SP-F6A scFv-hCHIP.dTPR-CD8α hinge- CD8α TM-P2A-eGFP;
SP-BMA031.wt scFv-hCHIP.dTPR-CD8α hinge- CD8α TM-P2A-eGFP;
SP-BMA031.H6L12 scFv-hCHIP.dTPR-CD8α hinge- CD8αTM-P2A-eGFP;
SP-αCD5.14 scFv-hCHIP.dTPR-CD8α hinge- CD8α TM-P2A-eGFP;
SP-αCD7.TH69 scFv-hCHIP.dTPR-CD8α hinge- CD8α TM-P2A-eGFP;
SP-αPD-L1 scFv-hCHIP.dTPR-CD8α hinge- CD8α TM-P2A-eGFP;
SP-αCD47 scFv-hCHIP.dTPR-CD8α hinge- CD8α TM-P2A-eGFP;
b) :
SP-SP34-CD3ζ.ΔIC-1 -GRAIL.IC-P2A-eGFP;
SP-SP34-CD8α hinge- CD8αTM -GRAIL.IC-P2A-eGFP;
SP-SP34-CD4 hinge- CD8αTM -GRAIL.IC-P2A-eGFP;
c):
CD3 ζ.ΔIC-IL2Rβjm-P2A-eGFP;
CD3ε.ΔIC-IL2Rβjm-P2A-eGFP;
d):
CD3ζ.ΔIC-GRAIL.IC-P2A-eGFP;
CAG-CD3ζ.ΔIC-GRAIL.IC-P2A-eGFP;
CD3ζ.ΔIC-CHIP. dTPR-P2A-GFP;
CD3ζ.ΔIC-RNF133.IC-P2A-GFP;
CD3ζ.ΔIC-RNF122.rIC-P2A-GFP;
CD3ζ.ΔIC-RNF152.rIC-P2A-GFP;
optionally, the fusion protein does not include P2A-eGFP and/or SP.

6. A nucleic acid comprising a polynucleotide encoding a fusion protein of any one of claims 1-5.

7. A vector comprising the nucleic acid of claim 6;
preferably, further comprising an expression promoter for the fusion protein comprising CAG or EF1a;
more preferably, the CAG or EF1a comprises a nucleic acid sequence at least about 95%, 96%, 97%, 98%, 99% or 100% identical to the nucleic acid sequence shown in SEQ ID NO.: 72 or 88, respectively;
more preferably, the expression promoter for the a) fusion protein is EF1a;
more preferably, the expression promoter for the b) fusion protein is EF1a;
more preferably, the expression promoter for the c) fusion protein is EF1a;
more preferably, the expression promoter for the CAG-CD3ζ.ΔIC-GRAIL.IC-P2A-eGFP in 5d) fusion protein is CAG, more preferably, the expression promoter for the other fusion proteins in 5d) fusion protein is EF1a.

8. A composition comprising a nucleic acid of claim 6 or a vector of claim 7.

9. A composition comprising a first nucleic acid and a second nucleic acid; or comprising a first vector having a first nucleic acid and a second vector having a second nucleic acid; or comprising a vector having a first nucleic acid and a second nucleic acid, wherein
(1) the first nucleic acid encodes a fusion protein of any one of claims 1-5, and
(2) the second nucleic acid encodes a chimeric antigen receptor (CAR) comprising:
(a) an extracellular ligand-binding domain comprising single chain variable fragment (scFv) specifically binding to a predetermined antigen;
(b) a transmembrane domain, preferably CD8α, CD28, 4-1BB or IL2R transmembrane domain, more preferably CD8α transmembrane domain, and
(c) a cytoplasmic segment comprising one or more signaling domains, preferably comprising a 4-1BB signaling domain and a CD3ζ signaling domain;
preferably, the predetermined antigen is a tumor-related antigen; more preferably, the tumor-related antigen is selected from the following group: CEA, Claudin 18.2, GPC3, Receptor tyrosine kinase-like Orphan Receptor 1 (ROR1), CD38, CD19, CD20, CD22, BCMA, CAIX, CD446, CD133, EGFR, EGFRvIII, EpCam, GD2, EphA2, Her1, Her2, ICAM-1, IL13Ra2, Mesothelin, MUC1, MUC16, NKG2D, PSCA, NY-ESO-1, MART-1, WT1, MAGE-A10, MAGE-A3, MAGE-A4, EBV, NKG2D, PD1, PD-L1, CD25, IL-2, or CD3; most preferably, the tumor-related antigen is CEA.

10. A cell comprising a nucleic acid of claim 6 or a vector of claim 7 or a composition of any one of claims 8-9; preferably, the cell is a mammalian cell, more preferably a human cell; more preferably, the cell is a T cell or a primary T cell, gamma delta T cell, NK cell, NKT cell, macrophage, B cell, or non-immune cell; most preferably the cell is an allogeneic cell.

11. A pharmaceutical composition comprising the fusion protein of any one of claims 1-5, a nucleic acid of claim 6 or a vector of claim 7 or a composition of any one of claims 8-9 or the cell of claim 10.

12. A nucleic acid of claim 6, a vector of claim 7, a composition of any one of claims 8-9, or a cell of claim 10 for use as a medicament.

13. A nucleic acid of claim 6, a vector of claim 7, or a composition of any one of claims 8-9 for *in vitro* use in reducing a target membrane-bound protein level in a cell; comprising introducing into the cell the nucleic acid of claim 6, the vector of claim 7, or the composition of any one of claims 8-9, preferably, the cell is a mammalian cell, more preferably a human cell; more preferably, the cell is a T cell or a primary T cell, gamma delta T cell, NK cell, NKT cell, macrophage, B cell, or non-immune cell; most preferably the cell is an allogeneic cell.

14. A composition of any one of claims 8-9, or a cell of claim 10 for use in the treatment of a disease, comprising administering to a subject in need thereof a therapeutically effective amount of allogeneic cells having a composition of any one of claims 8-9 or administering to a subject in need thereof a therapeutically effective amount of cells of claim 10; preferably, the cell is a T cell or a primary T cell, gamma delta T cell, NK cell, NKT cell, macrophage, B cell, or non-immune cell; preferably, the subject has reduced Graft-versus-Host Disease (GvHD).

## Patentansprüche

1. Ein Fusionsprotein, das umfasst:
ein Abbau-Tag-Protein, das den Abbau eines membrangebundenen Proteins (MBP) vermittelt, wobei das Abbau-Tag-Protein eine Ubiquitin-Ligase oder eine oder mehrere Untereinheiten oder Strukturdomänen der Zelloberflächenrezeptoren umfasst, bei denen es sich um Interleukin-Rezeptoren handelt, die den Abbau vermitteln; und
eine Transmembran-Verbindungsdomäne, umfassend:
1) eine Transmembrandomäne und ein Verbindungsprotein;
wobei die Transmembrandomäne ausgewählt ist aus der Transmembrandomäne einer oder mehrerer Untereinheiten oder Strukturdomänen von CD8α, CD4, CD3, CD28, 4-1BB und IL2R; und das Verbindungsprotein ausgewählt ist aus einem oder mehreren Antikörpern oder Antigen-bindenden Fragmenten, die spezifisch an das abzubauende MBP binden; und die Transmembrandomäne direkt oder indirekt an das Verbindungsprotein gebunden ist; oder
2) eine Transmembrandomäne des abzubauenden MBP;
wobei
(a) vorzugsweise das MBP ein membrangebundener Rezeptor ist, mehr bevorzugt ein membrangebundener Rezeptor, der aus einem Säugetier stammt, am meisten bevorzugt ein menschlicher membrangebundener Rezeptor;
(b) vorzugsweise die Ubiquitin-Ligase eine oder mehrere Untereinheiten oder Strukturdomänen der E3-Ubiquitin-Ligase oder einer Variante davon umfasst;
(c) vorzugsweise das Antigen-bindende Fragment, das spezifisch an das in obigem Punkt 1) abzubauende MBP bindet, ein scFv, einen Nano-Antikörper, der spezifisch an das MBP oder eine Variante davon bindet, umfasst;
(d) vorzugsweise das Fusionsprotein mit der Transmembran-Verbindungsdomäne aus obigem Punkt 1) ferner ein Signalpeptid umfasst;
(e) vorzugsweise die Transmembrandomäne des abzubauenden MBP in obigem Punkt 2) eine Transmembrandomäne von einem oder mehreren der folgenden umfasst: CD3ζ, CD3γ, CD3δ, CD3ε, CD3α, CD3β oder einer Variante davon; oder
(f) vorzugsweise die Transmembran-Verbindungsdomäne in obigem Punkt 2) eine extrazelluläre Domäne und eine Transmembrandomäne von einer oder mehreren der folgenden umfasst: CD3ζ, CD3γ, CD3δ, CD3ε, CD3α, CD3β.
(g) mehr bevorzugt die Transmembran-Verbindungsdomäne in obigem Punkt 2) ein Signalpeptid, eine extrazelluläre Domäne und eine Transmembrandomäne von einer oder mehreren der folgenden umfasst: CD3ζ, CD3γ, CD3δ, CD3ε, CD3α, CD3β.

2. Fusionsprotein nach Anspruch 1, wobei
der membrangebundene Rezeptor aus einer oder mehreren Untereinheiten oder Strukturdomänen von CD3, TCR, CD5, CD7, PD-L1 und CD47 ausgewählt ist;
(a) die E3-Ubiquitin-Ligase aus den folgenden Proteinen oder deren verkürzten Formen ausgewählt ist: C-Terminus des Hsc70-interagierenden Proteins (CHIP), CHIP.dTPR, F-Box-WD40-haltiges Protein 7 (FBW7), FBW7.2-293, von Hippel-Lindau (VHL), VHL.152-213, Speckle-Typ-BTB-POZ-Protein (SPOP), SPOP.167-374, SOCS2, SOCS2.143-198, Ubiquitin-Protein-Ligase E3A (UBE3A), Maus-Double-Minute-2-Homolog (MDM2), Anaphase-Promoting-Complex (APC), UBR5, LNX, Casitas-B-Linien Lymphom-transformierende Sequenz-ähnliches Protein 1 (CBLL1), HECT-Domäne und Ankyrin-Wiederholung enthaltende E3-Ubiquitin-Protein-Ligase 1 (HACE1), HECT-, C2- und WW-Domäne enthaltende E3-Ubiquitin-Protein-Ligase 1 (HECW1), HECT-, C2- und WW-Domäne enthaltende E3-Ubiquitin-Protein-Ligase 2 (HECW2), HECT- und RLD-Domäne enthaltende E3-Ubiquitin-Protein-Ligase 1 (HERC1), HERC2, HERC3, HERC4, HERC5, HERC6, HUWE1, ITCH, in neuralen Vorläuferzellen exprimiertes, entwicklungsbedingt herunterreguliertes Protein 4 (NEDD4), in neuralen Vorläuferzellen exprimiertes, entwicklungsbedingt herunterreguliertes Gen 4-Ähnliches (NEDD4L), Peptidylprolylisomerase (Cyclophilin)-Ähnliches 2 (PPIL2), PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UBOX5, UBR5, WWP1, WWP2, Parkin, MKRN1, GRAIL.IC, RNF133.IC, RNF122.rIC oder RNF152.rIC; vorzugsweise umfasst die E3-Ubiquitin-Ligase CHIP, CHIP.dTPR, FBW7.2-293, VHL.152-213, SPOP.167-374, SOCS2.143-198, GRAIL.IC, RNF133.IC, RNF122.rIC oder RNF152.rIC; und mehr bevorzugt umfasst die CHIP, CHIP.dTPR, FBW7.2-293, VHL.152-213, SPOP.167-374, SOCS2.143-198, GRAIL.IC, RNF133.IC, RNF122.rIC oder RNF152.rIC eine Aminosäuresequenz, die mindestens zu etwa 95 %, 96 %, 97 %, 98 %, 99 % oder 100 % mit der in einer der SEQ ID NOs. 21-26, 62, 79, 81 bzw. 83 gezeigten Aminosäuresequenz identisch ist;
(b) die Untereinheiten oder Strukturdomänen von Interleukinrezeptoren ein lysosomales Targeting-Motiv von IL-2R umfassen; vorzugsweise umfasst das lysosomale Targeting-Motiv von IL-2R die L2Rß-jm-Domäne; mehr bevorzugt umfasst die L2Rβ-jm-Domäne (IL2Rβjm) eine Aminosäuresequenz, die mindestens zu etwa 95 %, 96 %, 97 %, 98 %, 99 % oder 100 % mit der in SEQ ID NO. 58 gezeigten Aminosäuresequenz identisch ist;
(c) die Transmembrandomäne in obigem Punkt 1) ausgewählt ist aus einer Transmembrandomäne von CD3, CD8α, CD4; vorzugsweise umfasst die CD3ζ-Transmembrandomänenvariante, die CD8α-Transmembrandomäne oder die CD4-Transmembrandomäne eine Aminosäuresequenz, die mindestens zu etwa 95 %, 96 %, 97 %, 98 %, 99 % oder 100 % mit der in SEQ ID NO. 89, 18 bzw. 15 gezeigten Aminosäuresequenz identisch ist;
(d) das Signalpeptid in obigem Punkt 1) ausgewählt ist aus einer Aminosäuresequenz, die mindestens zu etwa 95 %, 96 %, 97 %, 98 %, 99 % oder 100 % identisch ist mit der in SEQ ID NO. 19 bzw. 20 gezeigten Aminosäuresequenz;
(e) der Antikörper oder das Antigen-bindende Fragment, die spezifisch an das MBP in obigem Punkt 1) binden, ausgewählt sind aus einem Antikörper oder einem Antigen-bindenden Fragment von CD3, CD5, CD7, PD-L1 oder CD47; vorzugsweise umfasst das Antigen-bindende Fragment von CD3 SP34 scFv, OKT3 scFv, UCHT1 scFv, UCHT1.Y177T scFv, L2K scFv, F6A scFv, BMA031 scFv oder BMA031.H6L12 scFv; das Antigen-bindende Fragment von CD5 umfasst aCD5.14 scFv; das Antigen-bindende Fragment von CD7 umfasst aCD7.TH69 scFv; das Antigen-bindende Fragment von PD-L1 umfasst αPD-L1 scFv; das Antigen-bindende Fragment von CD47 umfasst αCD47 scFv; mehr bevorzugt umfassen SP34 scFv, OKT3 scFv, UCHT1 scFv, UCHT1.Y177T scFv, L2K scFv, F6A scFv, BMA031 scFv, BMA031.H6L12 scFv, αCD5.14 scFv, αCD7.TH69 scFv, αPD-L1 scFv oder αCD47 scFv eine Aminosäuresequenz, die mindestens zu etwa 95 %, 96 %, 97 %, 98 %, 99 % oder 100 % mit der in einer der SEQ ID NOs. 2-13 gezeigten Aminosäuresequenz identisch ist;
(f) die Transmembran-Verbindungsdomäne aus obigem Punkt 2) eine Transmembrandomäne von CD3ζ oder CD3ε umfasst, vorzugsweise eine extrazelluläre Domäne und eine Transmembrandomäne von CD3ζ oder CD3ε, mehr bevorzugt ein zweites Signalpeptid, eine extrazelluläre Domäne und eine Transmembrandomäne von CD3ζ oder CD3ε; mehr bevorzugt umfasst die Transmembrandomäne (TM) von CD3ζ oder CD3ε eine Aminosäuresequenz, die mindestens zu etwa 95 %, 96 %, 97 %, 98 %, 99 % oder 100 % mit der in SEQ ID NO. 67 bzw. 70 gezeigten Aminosäuresequenz identisch ist; mehr bevorzugt umfasst die extrazelluläre Domäne (EM) von CD3ζ oder CD3ε eine Aminosäuresequenz, die mindestens zu etwa 95 %, 96 %, 97 %, 98 %, 99 % oder 100 % mit der in SEQ ID NO. 66 bzw. 69 gezeigten Aminosäuresequenz identisch ist; mehr bevorzugt umfasst das zweite Signalpeptid von CD3ζ oder CD3ε eine Aminosäuresequenz, die mindestens zu etwa 95 %, 96 %, 97 %, 98 %, 99 % oder 100 % mit der in SEQ ID NO. 65 bzw. 68 gezeigten Aminosäuresequenz identisch ist; mehr bevorzugt umfassen das zweite Signalpeptid, die extrazelluläre Domäne und eine Transmembrandomäne von CD3ζ (CD3ζ.ΔIC) sowie das zweite Signalpeptid, die extrazelluläre Domäne und eine Transmembrandomäne von CD3ε (CD3ε.ΔIC) eine Aminosäuresequenz, die mindestens zu etwa 95 %, 96 %, 97 %, 98 %, 99 % oder 100 % mit der in SEQ ID NO. 61 bzw. 64 gezeigten Aminosäuresequenz identisch ist.

3. Fusionsprotein nach einem der Ansprüche 1-2, wobei
das Fusionsprotein ferner eines oder mehrere von einem Hinge oder einem P2A-GFP umfasst;
vorzugsweise ist das Hinge ausgewählt aus einem CD8α-Hinge oder einem CD4-Hinge; mehr bevorzugt umfasst das CD8α-Hinge oder das CD4-Hinge eine Aminosäuresequenz, die mindestens zu etwa 95 %, 96 %, 97 %, 98 %, 99 % oder 100 % mit der in SEQ ID NO. 16 bzw. 14 gezeigten Aminosäuresequenz identisch ist; mehr bevorzugt liegt das Hinge benachbart zum N-Terminus der ersten Transmembrandomäne;
vorzugsweise umfasst P2A-GFP eine Aminosäuresequenz, die mindestens zu etwa 95 %, 96 %, 97 %, 98 %, 99 % oder 100 % mit der in SEQ ID NO. 57 gezeigten Aminosäuresequenz identisch ist; mehr bevorzugt befindet sich die P2A-GFP-Sequenz am C-Terminus des Fusionsproteins.

4. Fusionsprotein nach einem der Ansprüche 1-3, wobei das Fusionsprotein vom N-Terminus zum C-Terminus umfasst:
a) das Verbindungsprotein, das intrazelluläre Abbau-Tag-Protein, die Transmembrandomäne;
vorzugsweise umfasst das Verbindungsprotein den Antikörper oder das Antigen-bindende Fragment, die spezifisch an das abzubauende MBP binden; mehr bevorzugt umfassen der Antikörper oder das Antigen-bindende Fragment, die spezifisch an das abzubauende MBP binden, einen Antikörper oder ein Antigenbindendes Fragment von CD3, CD5, CD7, PD-L1 oder CD47; mehr bevorzugt umfasst das Antigen-bindende Fragment von CD3 SP34 scFv, OKT3 scFv, UCHT1 scFv, UCHT1.Y177T scFv, L2K scFv, F6A scFv, BMA031 scFv oder BMA031.H6L12 scFv; das Antigen-bindende Fragment von CD5 umfasst αCD5.14 scFv; das Antigen-bindende Fragment von CD7 umfasst αCD7.TH69 scFv; das Antigen-bindende Fragment von PD-L1 umfasst αPD-L1 scFv; das Antigen-bindende Fragment von CD47 umfasst αCD47 scFv;
vorzugsweise umfasst das Abbau-Tag-Protein die E3-Ubiquitin-Ligase; mehr bevorzugt umfasst die E3-Ubiquitin-Ligase CHIP.dTPR (CHIP: C-Terminus des Hsc70-interagierenden Proteins), FBW7.2-293 (FBW7: F-Box-WD40-haltiges Protein 7), VHL.152-213 (von Hippel-Lindau), SPOP.167-374 (Speckle-Typ-BTB-POZ-Protein), SOCS2.143-198;
vorzugsweise umfasst die Transmembrandomäne die CD8α-Transmembrandomäne;
b) das Verbindungsprotein, die Transmembrandomäne, das intrazelluläre Abbau-Tag-Protein;
vorzugsweise umfasst das Verbindungsprotein den Antikörper oder das Antigen-bindende Fragment, die spezifisch an das abzubauende MBP binden; mehr bevorzugt umfasst der Antikörper oder das Antigen-bindende Fragment, die spezifisch an das abzubauende MBP binden, den Antikörper oder das Antigen-bindende Fragment von CD3; mehr bevorzugt umfasst das Antigen-bindende Fragment von CD3 SP34 scFv;
vorzugsweise umfasst das intrazelluläre Abbau-Tag-Protein die E3-Ubiquitin-Ligase; mehr bevorzugt umfasst die E3-Ubiquitin-Ligase GRAIL.IC;
vorzugsweise ist die Transmembrandomäne ausgewählt aus einer oder mehreren der folgenden: einer CD3ζ-Transmembrandomänenvariante, der CD8α-Transmembrandomäne und der CD4-Transmembrandomäne;
c) die Transmembran-Verbindungsdomäne aus obigem Punkt 2), die Zelloberflächenrezeptoren, die den Abbau vermitteln;
vorzugsweise umfasst die Transmembran-Verbindungsdomäne aus obigem Punkt 2) CD3ζ.ΔIC oder CD3ε.ΔIC;
vorzugsweise umfassen die den Abbau vermittelnden Zelloberflächenrezeptoren IL2Rβjm; oder
d) die Transmembran-Verbindungsdomäne aus obigem Punkt 2), die eine oder mehrere E3-Ubiquitin-Ligasen;
vorzugsweise umfasst die Transmembran-Verbindungsdomäne in obigem Punkt 2) CD3ζ.ΔIC;
vorzugsweise umfasst die eine oder mehrere E3-Ubiquitin-Ligasen oder eine Variante davon GRAIL.IC, CHIP.dTPR, RNF133.IC, RNF122.rIC, RNF152.rIC.

5. Fusionsprotein nach Anspruch 4, wobei das Fusionsprotein vom N-Terminus zum C-Terminus umfasst:
a):
SP-OKT3 scFv-CHIP.dTPR-CD8α hinge - CD8α TM-P2A-eGFP;
SP-OKT3 scFv-FBW7-CD8α hinge- CD8α TM-P2A-eGFP;
SP-OKT3 scFv-VHL-CD8α hinge- CD8α TM-P2A-eGFP;
SP-OKT3 scFv-SPOP-CD8α hinge- CD8α TM-P2A-eGFP;
SP-OKT3 scFv-SOCS2-CD8α hinge- CD8α TM-P2A-eGFP;
SP-SP34 scFv-hCHIP.dTPR-CD8α hinge- CD8α TM-P2A-eGFP;
SP-UCHT1 scFv-hCHIP.dTPR-CD8α hinge- CD8α TM-P2A-eGFP;
SP-UCHT1.Y177T scFv-hCHIP.dTPR-CD8α hinge-CD8αTM-P2A-eGFP;
SP-L2K scFv-hCHIP.dTPR-CD8α hinge- CD8α TM-P2A-eGFP;
SP-F6A scFv-hCHIP.dTPR-CD8α hinge- CD8α TM-P2A-eGFP;
SP-BMA031.wt scFv-hCHIP.dTPR-CD8α hinge- CD8α TM-P2A-eGFP;
SP-BMA031.H6L12 scFv-hCHIP.dTPR-CD8α hinge- CD8αTM-P2A-eGFP;
SP-αCD5.14 scFv-hCHIP.dTPR-CD8α hinge- CD8α TM-P2A-eGFP;
SP-αCD7.TH69 scFv-hCHIP.dTPR-CD8α hinge- CD8α TM-P2A-eGFP;
SP-αPD-L1 scFv-hCHIP.dTPR-CD8α hinge- CD8α TM-P2A-eGFP;
SP-αCD47 scFv-hCHIP.dTPR-CD8α hinge- CD8α TM-P2A-eGFP;
b):
SP-SP34-CD3ζ.ΔIC-1 -GRAIL.IC-P2A-eGFP;
SP-SP34-CD8α hinge- CD8αTM -GRAIL.IC-P2A-eGFP;
SP-SP34-CD4 hinge- CD8αTM -GRAIL.IC-P2A-eGFP;
c):
CD3ζ.ΔIC-IL2Rβjm-P2A-eGFP;
CD3ε.ΔIC-IL2Rβjm-P2A-eGFP;
d):
CD3ζ.ΔIC-GRAIL.IC-P2A-eGFP;
CAG-CD3ζ.ΔIC-GRAIL.IC-P2A-eGFP;
CD3ζ.ΔIC-CHIP. dTPR-P2A-GFP;
CD3ζ.ΔIC-RNF133.IC-P2A-GFP;
CD3ζ.ΔIC-RNF122.rlC-P2A-GFP;
CD3ζ.ΔIC-RNF152.rlC-P2A-GFP;
optional schließt das Fusionsprotein kein P2A-eGFP und/oder SP ein.

6. Eine Nukleinsäure, die ein Polynukleotid, das ein Fusionsprotein gemäß einem der Ansprüche 1-5 codiert, umfasst.

7. Ein Vektor, der die Nukleinsäure gemäß Anspruch 6 umfasst;
vorzugsweise ferner umfassend einen Expressionspromotor für das Fusionsprotein, der CAG oder EF1a umfasst;
mehr bevorzugt umfasst das CAG oder EF1a eine Nukleinsäuresequenz, die mindestens zu etwa 95 %, 96 %, 97 %, 98 %, 99 % oder 100 % mit der in SEQ ID NO. 72 bzw. 88 gezeigten Nukleinsäuresequenz identisch ist;
mehr bevorzugt ist der Expressionspromotor für das Fusionsprotein a) EF1a;
mehr bevorzugt ist der Expressionspromotor für das Fusionsprotein b) EF1a;
mehr bevorzugt ist der Expressionspromotor für das Fusionsprotein c) EF1a;
mehr bevorzugt ist der Expressionspromotor für das CAG-CD3ζ.ΔIC-GRAIL.IC-P2A-eGFP-Fusionsprotein in 5d) CAG, mehr bevorzugt ist der Expressionspromotor für die anderen Fusionsproteine in 5d) EF1a.

8. Eine Zusammensetzung, die eine Nukleinsäure nach Anspruch 6 oder einen Vektor nach Anspruch 7 umfasst.

9. Eine Zusammensetzung, umfassend eine erste Nukleinsäure und eine zweite Nukleinsäure; oder umfassend einen ersten Vektor mit einer ersten Nukleinsäure und einen zweiten Vektor mit einer zweiten Nukleinsäure; oder umfassend einen Vektor mit einer ersten Nukleinsäure und einer zweiten Nukleinsäure, wobei
(1) die erste Nukleinsäure ein Fusionsprotein nach einem der Ansprüche 1-5 codiert, und
(2) die zweite Nukleinsäure einen chimären Antigenrezeptor (CAR) codiert, der umfasst:
(a) eine extrazelluläre Liganden-Bindungsdomäne, die ein Einzelketten-Variables Fragment (scFv) umfasst, das spezifisch an ein vorbestimmtes Antigen bindet;
(b) eine Transmembrandomäne, vorzugsweise eine CD8α-, CD28-, 4-1BB- oder IL2R-Transmembrandomäne, mehr bevorzugt eine CD8α-Transmembrandomäne, und
(c) ein zytoplasmatisches Segment, das eine oder mehrere Signaldomänen umfasst, vorzugsweise eine 4-1BB-Signaldomäne und eine CD3ζ-Signaldomäne;
vorzugsweise ist das vorbestimmte Antigen ein tumorassoziiertes Antigen; mehr bevorzugt ist das tumorassoziierte Antigen aus der folgenden Gruppe ausgewählt: CEA, Claudin 18.2, GPC3, Rezeptor-Tyrosinkinase-ähnlicher Orphan-Rezeptor 1 (ROR1), CD38, CD19, CD20, CD22, BCMA, CAIX, CD446, CD133, EGFR, EGFRvIII, EpCam, GD2, EphA2, Her1, Her2, ICAM-1, IL13Ra2, Mesothelin, MUC1, MUC16, NKG2D, PSCA, NY-ESO-1, MART-1, WT1, MAGE-A10, MAGE-A3, MAGE-A4, EBV, NKG2D, PD1, PD-L1, CD25, IL-2 oder CD3; am meisten bevorzugt ist das tumorassoziierte Antigen CEA.

10. Eine Zelle, die eine Nukleinsäure nach Anspruch 6 oder einen Vektor nach Anspruch 7 oder eine Zusammensetzung nach einem der Ansprüche 8-9 umfasst; vorzugsweise ist die Zelle eine Säugetierzelle, mehr bevorzugt eine menschliche Zelle; mehr bevorzugt ist die Zelle eine T-Zelle oder eine primäre T-Zelle, eine Gamma-Delta-T-Zelle, eine NK-Zelle, eine NKT-Zelle, ein Makrophage, eine B-Zelle oder eine Nicht-Immunzelle; am meisten bevorzugt ist die Zelle eine allogene Zelle.

11. Eine pharmazeutische Zusammensetzung, die das Fusionsprotein nach einem der Ansprüche 1-5, eine Nukleinsäure nach Anspruch 6 oder einen Vektor nach Anspruch 7 oder eine Zusammensetzung nach einem der Ansprüche 8-9 oder die Zelle nach Anspruch 10 umfasst.

12. Eine Nukleinsäure nach Anspruch 6, ein Vektor nach Anspruch 7, eine Zusammensetzung nach einem der Ansprüche 8-9 oder eine Zelle nach Anspruch 10 zur Verwendung als Arzneimittel.

13. Eine Nukleinsäure nach Anspruch 6, ein Vektor nach Anspruch 7 oder eine Zusammensetzung nach einem der Ansprüche 8-9 zur *in* vitro-Verwendung zur Verringerung des Spiegels eines membrangebundenen Ziel-Proteins in einer Zelle; umfassend das Einbringen der Nukleinsäure nach Anspruch 6, des Vektors nach Anspruch 7 oder der Zusammensetzung nach einem der Ansprüche 8-9 in die Zelle, wobei die Zelle vorzugsweise eine Säugetierzelle, mehr bevorzugt eine menschliche Zelle ist; mehr bevorzugt ist die Zelle eine T-Zelle oder eine primäre T-Zelle, eine Gamma-Delta-T-Zelle, eine NK-Zelle, eine NKT-Zelle, ein Makrophage, eine B-Zelle oder eine Nicht-Immunzelle; am meisten bevorzugt ist die Zelle eine allogene Zelle.

14. Eine Zusammensetzung nach einem der Ansprüche 8-9 oder eine Zelle nach Anspruch 10 zur Verwendung bei der Behandlung einer Krankheit, umfassend die Verabreichung einer therapeutisch wirksamen Menge allogener Zellen mit einer Zusammensetzung nach einem der Ansprüche 8-9 an einen Patienten, der diese benötigt, oder die Verabreichung einer therapeutisch wirksamen Menge von Zellen nach Anspruch 10 an einen Patienten, der diese benötigt; vorzugsweise ist die Zelle eine T-Zelle oder eine primäre T-Zelle, eine Gamma-Delta-T-Zelle, eine NK-Zelle, eine NKT-Zelle, ein Makrophage, eine B-Zelle oder eine Nicht-Immunzelle; vorzugsweise weist der Patient eine verminderte Graft-versus-Host-Reaktion (GvHD) auf.

## Revendications

1. Protéine de fusion comprenant :
une protéine de marquage de dégradation qui assure la médiation de la dégradation d'une protéine liée à la membrane (MBP), dans laquelle la protéine de marquage de dégradation comprend une ubiquitine ligase, ou une ou plusieurs sous-unités ou domaines structurels des récepteurs de la surface cellulaire qui est un récepteur d'interleukine qui assure la médiation de la dégradation ; et
un domaine de liaison transmembranaire comprenant :
1) un domaine transmembranaire et une protéine de liaison ;
dans laquelle le domaine transmembranaire est choisi parmi le domaine transmembranaire d'une ou plusieurs sous-unités ou domaines structurels de CD8α, CD4, CD3, CD28, 4-1BB et IL2R ; et la protéine de liaison est choisie parmi un ou plusieurs anticorps ou fragments de liaison à l'antigène se liant spécifiquement à la MBP à dégrader ; et le domaine transmembranaire est lié directement ou indirectement à la protéine de liaison ; ou
2) un domaine transmembranaire de la MBP à dégrader ; dans laquelle
(a) de préférence, la MBP est un récepteur lié à la membrane, plus préférentiellement un récepteur lié à la membrane d'origine mammifère, de préférence encore un récepteur lié à la membrane humaine ;
(b) de préférence, l'ubiquitine ligase comprend une ou plusieurs sous-unités ou domaines structurels de l'ubiquitine ligase E3 ou une variante de celle-ci ;
(c) de préférence, le fragment de liaison à l'antigène se liant spécifiquement à la MBP à dégrader au point 1) ci-dessus comprend un scFv, un nano-anticorps se liant spécifiquement à la MBP ou à une variante de celle-ci ;
(d) de préférence, la protéine de fusion ayant le domaine de liaison transmembranaire du point 1) ci-dessus comprend en outre un peptide signal ;
(e) de préférence, le domaine transmembranaire de la MBP à dégrader au point 2) ci-dessus comprend un domaine transmembranaire d'un ou de plusieurs parmi CD3ζ, CD3γ, CD3δ, CD3ε, CD3α, CD3β ou d'une variante de ceux-ci ; ou
(f) de préférence, le domaine de liaison transmembranaire du point 2) ci-dessus comprend un domaine extracellulaire et un domaine transmembranaire d'un ou de plusieurs parmi CD3ζ, CD3γ, CD3δ, CD3ε, CD3α, CD3β.
(g) plus préférentiellement, le domaine de liaison transmembranaire du point 2) ci-dessus comprend un peptide signal, un domaine extracellulaire et un domaine transmembranaire d'un ou de plusieurs parmi CD3ζ, CD3γ, CD3δ, CD3ε, CD3α, CD3β.

2. Protéine de fusion selon la revendication 1, dans laquelle,
le récepteur lié à la membrane est choisi parmi une ou plusieurs sous-unités ou domaines structurels de CD3, TCR, CD5, CD7, PD-L1 et CD47 ;
(a) l'ubiquitine ligase E3 est choisie parmi les protéines suivantes ou leurs formes tronquées : l'extrémité C-terminale de la protéine interagissant avec Hsc70 (CHIP), CHIP.dTPR, la protéine 7 contenant la boîte F et WD40 (FBW7), FBW7.2-293, von Hippel-Lindau (VHL), VHL.152-213, la protéine de type Speckle BTB-POZ (SPOP), SPOP.167-374, SOCS2, SOCS2.143-198, l'ubiquitine-protéine ligase E3A (UBE3A), l'homologue de la double minute 2 de souris (MDM2), le complexe promoteur de l'anaphase (APC), UBR5, LNX, la protéine 1 de type séquence de transformation du lymphome de la lignée B de Casitas (CBLL1), l'ubiquitine-protéine ligase E3 contenant un domaine HECT et une répétition ankyrine 1 (HACE1), l'ubiquitine-protéine ligase E3 contenant les domaines HECT, C2 et WW 1 (HECW1), l'ubiquitine protéine ligase 2 E3 contenant les domaines HECT, C2 et WW (HECW2), l'ubiquitine protéine ligase 1 E3 contenant les domaines HECT et RLD (HERC1), HERC2, HERC3, HERC4, HERC5, HERC6, HUWE1, ITCH, la protéine 4 exprimée par les cellules précurseurs neurales et régulée à la baisse au cours du développement (NEDD4), le gène 4 exprimé par les cellules précurseurs neurales et régulé à la baisse au cours du développement (NEDD4L), la protéine de type peptidylprolyl isomérase (cyclophiline) 2 (PPIL2), PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UB0X5, UBR5, WWP1, WWP2, Parkin, MKRN1, GRAIL.IC, RNF133. IC, RNF122.rlC ou RNF152.rlC ; de préférence, l'ubiquitine ligase E3 comprend CHIP, CHIP.dTPR, FBW7.2-293, VHL.152-213, SPOP.167-374, SOCS2.143-198, GRAIL.IC, RNF133. IC, RNF122.rlC ou RNF152.rlC ; et, plus préférentiellement, les protéines CHIP, CHIP.dTPR, FBW7.2-293, VHL.152-213, SPOP.167-374, SOCS2.143-198, GRAIL.IC, RNF133. IC, RNF122.rlC ou RNF152.rlC comprennent une séquence d'acides aminés identique à au moins environ 95 %, 96 %, 97 %, 98 %, 99 % ou 100 % à la séquence d'acides aminés indiquée dans l'une quelconque de SEQ ID NO. 21-26, 62, 79, 81 ou 83 respectivement ;
(b) les sous-unités ou domaines structurels des récepteurs d'interleukine comprennent un motif de ciblage lysosomal de l'IL-2R ; de préférence, le motif de ciblage lysosomal de l'IL-2R comprend le domaine L2Rβ jm ; plus préférentiellement, le domaine L2Rβ jm (IL2Rβjm) comprend une séquence d'acides aminés au moins identique à environ 95 %, 96 %, 97 %, 98 %, 99 % ou 100 % à la séquence d'acides aminés indiquée dans SEQ ID NO. 58 ;
(c) le domaine transmembranaire au point 1) ci-dessus est choisi parmi un domaine transmembranaire de CD3, CD8α, CD4 ; de préférence, la variante du domaine transmembranaire CD3ζ, le domaine transmembranaire CD8α ou le domaine transmembranaire CD4 comprend une séquence d'acides aminés identique à au moins 95 %, 96 %, 97 %, 98 %, 99 % ou 100 % à la séquence d'acides aminés indiquée dans SEQ ID NO. 89, 18 ou 15 respectivement ;
(d) le peptide signal au point 1) ci-dessus est choisi parmi une séquence d'acides aminés identique à au moins 95 %, 96 %, 97 %, 98 %, 99 % ou 100 % à la séquence d'acides aminés indiquée dans SEQ ID NO. 19 ou 20 respectivement ;
(e) l'anticorps ou le fragment de liaison à l'antigène se liant spécifiquement à la MBP au point 1) ci-dessus est choisi parmi un anticorps ou un fragment de liaison à l'antigène de CD3, CD5, CD7, PD-L1 ou CD47 ; de préférence, le fragment de liaison à l'antigène de CD3 comprend le scFv SP34, le scFv OKT3, le scFv UCHT1, le scFv UCHT1.Y177T, le scFv L2K, le scFv F6A, e scFv BMA031, ou le scFv BMA031.H6L12 ; le fragment de CD5 se liant à l'antigène comprend le scFv αCD5.14 ; le fragment de CD7 se liant à l'antigène comprend le scFv αCD7.TH69 ; le fragment de PD-L1 se liant à l'antigène comprend le scFv αPD-L1 ; le fragment CD47 se liant à l'antigène comprend le scFv αCD47 ; plus préférentiellement, le scFv SP34, le scFv OKT3, le scFv UCHT1, se scFv UCHT1.Y177T, le scFv L2K, le scFv F6A, le scFv BMA031, le scFv BMA031.H6L12, le scFv αCD5.14, le scFv αCD7.TH69, le scFv αPD-LI ou le scFv αCD47 comprend une séquence d'acides aminés au moins identique à 95 %, 96 %, 97 %, 98 %, 99 % ou 100 % à la séquence d'acides aminés indiquée dans l'une quelconque de SEQ ID NO. 2-13 respectivement ;
(f) le domaine de liaison transmembranaire du point 2) ci-dessus comprend un domaine transmembranaire de CD3ζ ou CD3ε, de préférence un domaine extracellulaire et un domaine transmembranaire de CD3ζ ou CD3ε, plus préférentiellement un second peptide signal, un domaine extracellulaire et un domaine transmembranaire de CD3ζ ou CD3ε ; plus préférentiellement, le domaine transmembranaire (TM) de CD3ζ ou CD3ε comprend une séquence d'acides aminés au moins identique à 95 %, 96 %, 97 %, 98 %, 99 % ou 100 % à la séquence d'acides aminés indiquée dans SEQ ID NO. 67, 70 respectivement ; plus préférentiellement, le domaine extracellulaire (EM) de CD3ζ ou CD3ε comprend une séquence d'acides aminés identique à au moins 95 %, 96 %, 97 %, 98 %, 99 % ou 100 % à la séquence d'acides aminés indiquée dans SEQ ID NO. 66, 69 respectivement; plus préférentiellement, le second peptide signal de CD3ζ ou CD3ε comprend une séquence d'acides aminés identique à au moins 95 %, 96 %, 97 %, 98 %, 99 % ou 100 % à la séquence d'acides aminés indiquée dans SEQ ID NO. 65, 68 respectivement ; plus préférentiellement, le second peptide signal, le domaine extracellulaire et un domaine transmembranaire de CD3ζ (CD3ζ.ΔIC) et le second peptide signal, le domaine extracellulaire et un domaine transmembranaire de CD3ε (CD3ε.ΔIC) comprennent une séquence d'acides aminés au moins identique à 95 %, 96 %, 97 %, 98 %, 99 % ou 100 % environ à la séquence d'acides aminés indiquée dans SEQ ID NO. 61 et 64 respectivement.

3. Protéine de fusion selon l'une quelconque des revendications 1-2, dans laquelle
la protéine de fusion comprend en outre un ou plusieurs éléments parmi une charnière ou une P2A-GFP ;
de préférence, la charnière est choisie parmi une charnière CD8α ou une charnière CD4 ; plus préférentiellement, la charnière CD8α ou la charnière CD4 comprend une séquence d'acides aminés au moins identique à 95 %, 96 %, 97 %, 98 %, 99 % ou 100 % à la séquence d'acides aminés indiquée dans SEQ ID NO. 16 ou 14, respectivement ; plus préférentiellement, la charnière est adjacente à l'extrémité N-terminale du premier domaine transmembranaire,
de préférence, la P2A-GFP comprend une séquence d'acides aminés identique à au moins 95 %, 96 %, 97 %, 98 %, 99 % ou 100 % à la séquence d'acides aminés indiquée dans SEQ ID NO. 57 ; plus préférentiellement, la séquence P2A-GFP se trouve à l'extrémité C-terminale de la protéine de fusion.

4. Protéine de fusion selon l'une quelconque des revendications 1 à 3, dans laquelle la protéine de fusion comprend une extrémité N-terminale à une extrémité C-terminale :
a) la protéine de liaison, la protéine de marquage de dégradation intracellulaire, le domaine transmembranaire ;
de préférence, la protéine de liaison comprend l'anticorps ou le fragment de liaison à l'antigène se liant spécifiquement à la MBP à dégrader ; plus préférentiellement, l'anticorps ou le fragment de liaison à l'antigène se liant spécifiquement à la MBP à dégrader comprend l'anticorps ou le fragment de liaison à l'antigène de CD3, CD5, CD7, PD-L1 ou CD47 ; plus préférentiellement, le fragment de liaison à l'antigène de CD3 comprend le scFv SP34, le scFv OKT3, le scFv UCHT1, le scFv UCHT1.Y177T, le scFv L2K, le F6A, le scFv BMA031, ou le scFv BMA031.H6L12 ; le fragment de liaison à l'antigène CD5 comprend le scFv αCD5.14 ; le fragment de liaison à l'antigène CD7 comprend le scFv αCD7.TH69 ; le fragment de liaison à l'antigène PD-L1 comprend le scFv αPD-LI ; le fragment de liaison à l'antigène CD47 comprend le scFv αCD47 ;
de préférence, la protéine de marquage de dégradation comprend l'ubiquitine ligase E3 ; plus préférentiellement, l'ubiquitine ligase E3 comprend CHIP.dTPR (CHIP : une extrémité C-terminale de la protéine interagissant avec Hsc70), FBW7.2-293 (FBW7 : protéine 7 contenant la boîte F et WD40), VHL.152-213 (von Hippel-Lindau), SPOP.167-374 (protéine de type Speckle BTB-POZ), SOCS2.143-198 ;
de préférence, le domaine transmembranaire comprend le domaine transmembranaire CD8α ;
b) la protéine de liaison, le domaine transmembranaire, la protéine de marquage de dégradation intracellulaire ;
de préférence, la protéine de liaison comprend l'anticorps ou le fragment de liaison à l'antigène se liant spécifiquement à la MBP à dégrader ; plus préférentiellement, l'anticorps ou le fragment de liaison à l'antigène se liant spécifiquement à la MBP à dégrader comprend l'anticorps ou le fragment de liaison à l'antigène de CD3 ; plus préférentiellement, le fragment de liaison à l'antigène de CD3 comprend le scFv SP34 ;
de préférence, la protéine de marquage de dégradation intracellulaire comprend l'ubiquitine ligase E3 ; plus préférentiellement, l'ubiquitine ligase E3 comprend GRAIL.IC ;
de préférence, le domaine transmembranaire est choisi parmi une ou plusieurs variantes du domaine transmembranaire CD3ζ, le domaine transmembranaire CD8α et le domaine transmembranaire CD4 ;
c) le domaine de liaison transmembranaire du point 2) ci-dessus, les récepteurs de la surface cellulaire qui assurent la médiation de la dégradation ;
de préférence, le domaine de liaison transmembranaire au point 2) ci-dessus comprend CD3ζ.ΔIC ou CD3ε.ΔIC ;
de préférence, les récepteurs de la surface cellulaire qui assurent la médiation de la dégradation comprennent l'IL2Rβjm ; ou
d) le domaine de liaison transmembranaire du point 2) ci-dessus, l'une ou plusieurs des ubiquitines ligases E3 ;
de préférence, le domaine de liaison transmembranaire au point 2) ci-dessus comprend CD3ζ.ΔIC ;
de préférence, la ou les E3 ubiquitines ligases ou une variante de celle-ci comprennent GRAIL.IC, CHIP.dTPR, RNF133.IC, RNF122.rIC, RNF152.rlC.

5. Protéine de fusion selon la revendication 4, dans laquelle la protéine de fusion comprend des extrémités de N-terminale à C-terminale :
a) :
SP-OKT3 scFv-CHIP.dTPR-charnière CD8α- CD8α TM-P2A-eGFP ;
SP-OKT3 scFv-FBW7-charnière CD8α- CD8α TM-P2A-eGFP ;
SP-OKT3 scFv-VHL-charnière CD8α- CD8α TM-P2A-eGFP ;
SP-OKT3 scFv-SPOP-charnière CD8α- CD8α TM-P2A-eGFP ;
SP-OKT3 scFv-SOCS2-charnière CD8α-CD8α TM-P2A-eGFP ;
SP-SP34 scFv-hCHIP.dTPR-charnière CD8α- CD8α TM-P2A-eGFP ;
SP-UCHT1 scFv-hCHIP.dTPR-charnière CD8α- CD8α TM-P2A-eGFP ;
SP-UCHT1.Y177T scFv-hCHIP.dTPR-charnière CD8α-CD8αTM-P2A-eGFP ;
SP-L2K scFv-hCHIP.dTPR-charnière CD8α- CD8α TM-P2A-eGFP ;
SP-F6A scFv-hCHIP.dTPR-charnière CD8α- CD8α TM-P2A-eGFP ;
SP-BMA031.wt scFv-hCHIP.dTPR-charnière CD8α- CD8α TM-P2A-eGFP ;
SP-BMA031.H6L12 scFv-hCHIP.dTPR-charnière CD8α- CD8αTM-P2A-eGFP;
SP-αCD5.14 scFv-hCHIP.dTPR-charnière CD8α- CD8α TM-P2A-eGFP ;
SP-αCD7.TH69 scFv-hCHIP.dTPR-charnière CD8α- CD8α TM-P2A-eGFP ;
SP-αPD-LI scFv-hCHIP.dTPR-charnière CD8α- CD8α TM-P2A-eGFP ;
SP-αCD47 scFv-hCHIP.dTPR-charnière CD8α- CD8α TM-P2A-eGFP ;
b) :
SP-SP34-CD3ζ.ΔIC-1 -GRAIL.IC-P2A-eGFP ;
SP-SP34-charnière CD8α- CD8αTM -GRAIL.IC-P2A-eGFP ;
SP-SP34-charnière CD4- CD8αTM -GRAIL.IC-P2A-eGFP ;
c) :
CD3ζ.ΔIC-IL2Rβjm-P2A-eGFP ;
CD3ε.ΔIC-IL2Rβjm-P2A-eGFP ;
d) :
CD3ζ.ΔIC-GRAIL.IC-P2A-eGFP ;
CAG-CD3ζ.ΔIC-GRAIL IC-P2A-eGFP ;
CD3ζ.ΔIC-CHIP dTPR-P2A-GFP ;
CD3ζ.ΔIC-RNF133.IC-P2A-GFP ;
CD3ζ.ΔIC-RNF122.rIC-P2A-GFP ;
CD3ζ.ΔIC-RNF152.rIC-P2A-GFP ;
éventuellement, la protéine de fusion ne comprend pas P2A-eGFP et/ou SP.

6. Acide nucléique comprenant un polynucléotide codant pour une protéine de fusion selon l'une quelconque des revendications 1 à 5.

7. Vecteur comprenant l'acide nucléique selon la revendication 6 ;
de préférence, comprenant en outre un promoteur d'expression pour la protéine de fusion comprenant CAG ou EF1a ;
plus préférentiellement, le CAG ou l'EF1a comprend une séquence d'acide nucléique identique à au moins 95 %, 96 %, 97 %, 98 %, 99 % ou 100 % à la séquence d'acide nucléique indiquée dans SEQ ID NO. 72 ou 88, respectivement ;
plus préférentiellement, le promoteur d'expression de la protéine de fusion a) est EF1a ;
plus préférentiellement, le promoteur d'expression de la protéine de fusion b) est EF1a ;
plus préférentiellement, le promoteur d'expression de la protéine de fusion c) est EF1a ;
plus préférentiellement, le promoteur d'expression de la protéine de fusion CAG-CD3ζ.ΔIC-GRAIL.IC-P2A-eGFP au point 5d) est CAG, plus préférentiellement, le promoteur d'expression des autres protéines de fusion au point 5d) est EF1a.

8. Composition comprenant un acide nucléique selon la revendication 6 ou un vecteur selon la revendication 7.

9. Composition comprenant un premier acide nucléique et un second acide nucléique ; ou comprenant un premier vecteur ayant un premier acide nucléique et un second vecteur ayant un second acide nucléique ; ou comprenant un vecteur ayant un premier acide nucléique et un second acide nucléique, dans laquelle
(1) le premier acide nucléique code pour une protéine de fusion selon l'une quelconque des revendications 1 à 5, et
(2) le second acide nucléique code pour un récepteur d'antigène chimérique (CAR) comprenant :
(a) un domaine extracellulaire de liaison au ligand comprenant un fragment variable à chaîne unique (scFv) se liant spécifiquement à un antigène prédéterminé ;
(b) un domaine transmembranaire, de préférence un domaine transmembranaire CD8α, CD28, 4-1BB ou IL2R, plus préférentiellement un domaine transmembranaire CD8α, et
(c) un segment cytoplasmique comprenant un ou plusieurs domaines de signalisation, de préférence comprenant un domaine de signalisation 4-1BB et un domaine de signalisation CD3ζ ;
de préférence, l'antigène prédéterminé est un antigène lié à la tumeur ; plus préférentiellement, l'antigène lié à la tumeur est choisi dans le groupe suivant : CEA, Claudin 18.2, GPC3, récepteur orphelin de type tyrosine kinase 1 (ROR1), CD38, CD19, CD20, CD22, BCMA, CAIX, CD446, CD133, EGFR, EGFRvIII, EpCam, GD2, EphA2, Her1, Her2, ICAM-1, IL13Ra2, Mésothéline, MUC1, MUC16, NKG2D, PSCA, NY-ESO-1, MART-1, WT1, MAGE-A10, MAGE-A3, MAGE- A4, EBV, NKG2D, PD1, PD-L1, CD25, IL-2 ou CD3 ; plus préférentiellement, l'antigène lié à la tumeur est le CEA.

10. Cellule comprenant un acide nucléique selon la revendication 6 ou un vecteur selon la revendication 7 ou une composition selon l'une quelconque des revendications 8 à 9 ; plus préférentiellement, la cellule est une cellule de mammifère, plus préférentiellement une cellule humaine ; plus préféreniellement, la cellule est une cellule T ou une cellule T primaire, une cellule T gamma delta, une cellule NK, une cellule NKT, un macrophage, une cellule B ou une cellule non immunitaire ; plus préférentiellement, la cellule est une cellule allogène.

11. Composition pharmaceutique comprenant la protéine de fusion selon l'une quelconque des revendications 1 à 5, un acide nucléique selon la revendication 6 ou un vecteur selon la revendication 7 ou une composition selon l'une quelconque des revendications 8 à 9 ou la cellule selon la revendication 10.

12. Acide nucléique selon la revendication 6, vecteur selon la revendication 7, composition selon l'une quelconque des revendications 8 à 9, ou cellule selon la revendication 10 pour utilisation comme médicament.

13. Acide nucléique selon la revendication 6, vecteur selon la revendication 7, ou composition selon l'une quelconque des revendications 8 à 9 pour utilisation *in vitro* dans la réduction d'un niveau cible de protéine liée à la membrane dans une cellule ; comprenant l'introduction dans la cellule de l'acide nucléique selon la revendication 6, du vecteur selon la revendication 7, ou de la composition selon l'une quelconque des revendications 8 à 9, de préférence, la cellule est une cellule de mammifère, plus préférentiellement une cellule humaine ; plus préférentiellement, la cellule est une cellule T ou une cellule T primaire, une cellule T gamma delta, une cellule NK, une cellule NKT, un macrophage, une cellule B ou une cellule non immunitaire ; plus préférentiellement, la cellule est une cellule allogénique.

14. Composition selon l'une quelconque des revendications 8 à 9, ou cellule selon la revendication 10 pour utilisation dans le traitement d'une maladie, comprenant l'administration à un sujet qui en a besoin d'une quantité thérapeutiquement efficace de cellules allogéniques ayant une composition selon l'une quelconque des revendications 8 à 9 ou l'administration à un sujet qui en a besoin d'une quantité thérapeutiquement efficace de cellules selon la revendication 10 ; de préférence, la cellule est une cellule T ou une cellule T primaire, une cellule T gamma delta, une cellule NK, une cellule NKT, un macrophage, une cellule B ou une cellule non immunitaire ; de préférence, le sujet a réduit la maladie du greffon contre l'hôte (GvHD).
